(19) Europäisches Patentamt
European Patent Office
Office européen des brevets

(11) EP 1 482 020 B1

(12) EUROPÄISCHE PATENTSCHRIFT

(45) Veröffentlichungstag und Bekanntmachung des Hinweises auf die Patenterteilung:
**06.09.2006 Patentblatt 2006/36**

(51) Int Cl.:
***C09K 19/34*** *(2006.01)* ***C07D 309/20*** *(2006.01)*
***C07D 309/32*** *(2006.01)* ***C07C 43/174*** *(2006.01)*
***C07C 69/52*** *(2006.01)* ***C07C 33/00*** *(2006.01)*

(21) Anmeldenummer: **04010545.4**

(22) Anmeldetag: **04.05.2004**

(54) **Pyranderivative mit exocyclischer Doppelbindung**

Pyran derivatives with an exocyclic double bond

Dérivés de pyrane avec une double liaison exocyclique

(84) Benannte Vertragsstaaten:
**AT BE BG CH CY CZ DE DK EE ES FI FR GB GR HU IE IT LI LU MC NL PL PT RO SE SI SK TR**

(30) Priorität: **27.05.2003 DE 10324348**

(43) Veröffentlichungstag der Anmeldung:
**01.12.2004 Patentblatt 2004/49**

(73) Patentinhaber: **Merck Patent GmbH**
**64293 Darmstadt (DE)**

(72) Erfinder:
- **Poetsch, Eike**
  **64367 Mühltal (DE)**
- **Meyer, Volker**
  **64846 Gross-Zimmen (DE)**
- **Binder, Werner**
  **64807 Dieburg (DE)**
- **Heckmeier, Michael**
  **69502 Hemsbach (DE)**
- **Lüssem, Georg**
  **85238 Petershausen (DE)**
- **Gürtler, Stephan**
  **64347 Griesheim (DE)**

(56) Entgegenhaltungen:
**EP-A- 0 409 066** **DE-A- 3 306 960**
**DE-A- 19 640 618** **US-A- 5 480 580**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 482 020 B1

## Beschreibung

Die Erfindung betrifft Pyranderivate mit exocyclischer Doppelbindung, Verfahren und Zwischenverbindungen zur ihrer Herstellung und Derivatisierung sowie ihre Verwendung in flüssigkristallinen Mischungen.

Pyranderivate spielen eine bedeutende Rolle in Chemie und Pharmazie, unter anderem als Inhaltsstoffe von natürlichen und synthetischen Aromastoffen, in Arzneimitteln und in flüssigkristallinen Materialien. Allerdings ist der präparative Zugang zu vielen Pyranen, insbesondere solchen mit 2,5-Disubstitution gegenwärtig limitiert und beschränkt sich oft auf die Derivatisierung von Kohlenhydraten, die Pyranoseringeinheiten aufweisen. Viele theoretisch denkbare Pyranderivate sind bislang synthetisch nicht zugänglich.

In der DE 3306960 A1 werden Herstellungsverfahren für Pyranderivate offenbart, u. a. durch Reduktion, Cyclisierung oder Decarboxylierung zu Tetrahydropyranen.

Der vorliegenden Erfindung liegt daher als eine Aufgabe zugrunde, neue Pyranderivate bereitzustellen, die über technisch nützliche Eigenschaften verfügen beziehungsweise als Ausgangsverbindungen zur effizienten Synthese weiterer Pyranderivate dienen können.

Gelöst wird diese Aufgabe durch Verbindungen der allgemeinen Formel I

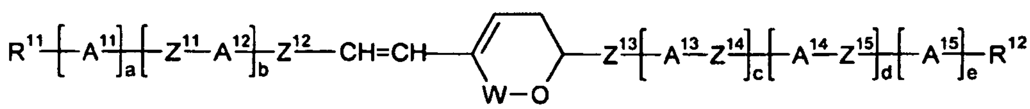

I

wobei

a, b, c, d und e — jeweils unabhängig voneinander 0 oder 1 sind;

W — $-CH_2-$ oder -C (=O)- bedeutet;

$R^{11}$ — H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C≡C-, -CH=CH-, -O-, -S-, -C(O)-O- und/oder -O-C(O)- so ersetzt sein können, dass Heteroatome (O, S) nicht direkt miteinander verknüpft sind;

$R^{12}$ — H, Halogen, -CN, -NCS, Aralkyl, -O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C≡C-, -CH=CH-, -O-, -S-, -C (O)-O- und/ oder -O- C (O)- so ersetzt sein können, dass Heteroatome (O, S) nicht direkt miteinander verknüpft sind;

$Z^{11}$ — eine Einfachbindung, $-CH_2-$, $-CH_2CH_2-$, $-CF_2CH_2-$, $-CH_2CF_2-$, $-CF_2CF_2-$, -CH=CH- oder -C=C- bedeutet;

$Z^{12}$ — eine Einfachbindung, $-CH_2-$, $-CH_2CH_2-$, $-CF_2CH_2-$, $-CH_2CF_2-$ oder $-CF_2CF_2-$ bedeutet;

$Z^{13}$, $Z^{14}$ und $Z^{15}$ — jeweils unabhängig voneinander eine Einfachbindung, $-CH_2CH_2-$, $-CF_2CH_2-$, $-CH_2CF_2-$, $-CF_2CF_2-$, -CH=CH-, -C=C-, $-CH_2O-$, $-CF_2O-$, -C(O)- oder -C (O)-O- bedeuten;

$A^{11}$ und $A^{12}$ — unabhängig voneinander für

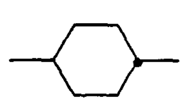

oder

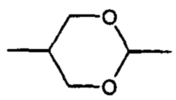

stehen;

A$^{13}$ und A$^{14}$ unabhängig voneinander für

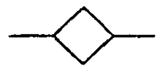,

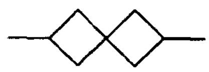, 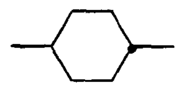, 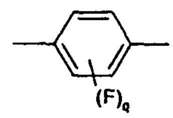,

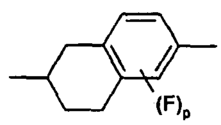 oder 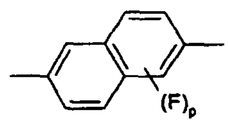

stehen;

A$^{15}$ für

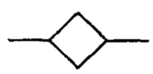, 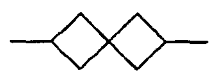, 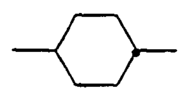,

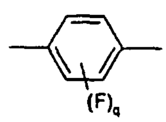, 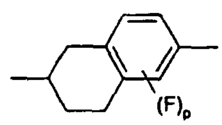 oder 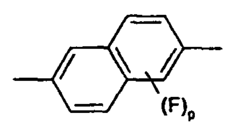

steht oder

A$^{15}$-R$^{12}$ zusammen für

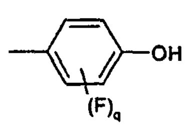, 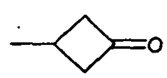,

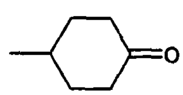 oder 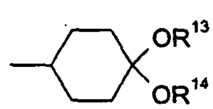

steht;

oder

$Z^{13}$-[-$A^{13}$-$Z^{14}$-]$_c$-[-$A^{14}$-$Z^{15}$]$_d$-[-$A^{15}$-]$_e$-$R^{12}$ für

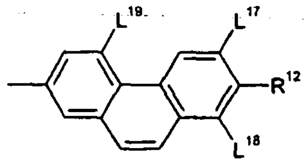

oder

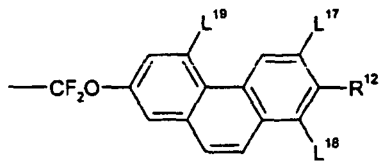

steht, wobei $R^{12}$ wie oben definiert ist und $L^{17}$, $L^{18}$ und $L^{19}$ unabhängig voneinander H oder F bedeuten;

q 0, 1, 2, 3 oder 4 ist;

p 0, 1, 2 oder 3 ist;

$R^{13}$ und $R^{14}$ unabhängig voneinander einen Alkanylrest mit 1 bis 7 Kohlenstoffatomen bedeutet oder zusammen für eine Alkylenbrücke mit 2 bis 7 Kohlenstoffatomen stehen;

mit der Maßgabe,
dass für den Fall der direkten Verknüpfung von $Z^{13}$ und $R^{12}$ zu -$Z^{13}$-$R^{12}$ $R^{12}$ H, Aralkyl, Alkanyl oder Alkenyl bedeutet, wenn $Z^{13}$ -C(=O)-O- oder -C(=O)-bedeutet, und $Z^{13}$ nicht -$CH_2O$- oder -$CF_2O$- bedeutet;
dass für den Fall der direkten Verknüpfung von $Z^{14}$ und $R^{12}$ zu -$Z^{14}$-$R^{12}$ $R^{12}$ H, Aralkyl, Alkanyl oder Alkenyl bedeutet, wenn $Z^{14}$ -C(=O)-O- oder -C(=O)-bedeutet, und $Z^{14}$ nicht -$CH_2O$- oder -$CF_2O$- bedeutet;
dass für den Fall der direkten Verknüpfung von $Z^{15}$ und $R^{12}$ zu -$Z^{15}$-$R^{12}$ $R^{12}$ H, Aralkyl, Alkanyl oder Alkenyl bedeutet, wenn $Z^{15}$ -C(=O)-O- oder -C(=O)-bedeutet, und $Z^{15}$ nicht -$CH_2O$- oder -$CF_2O$- bedeutet.

Die erfindungsgemäßen Verbindungen der Formel I finden auf Grund ihrer Eigenschaften Verwendung in flüssigkristallinen Medien beziehungsweise dienen als Ausgangsverbindungen zur effizienten Synthese weiterer Pyranverbindungen, insbesondere solchen mit mesogenen Eigenschaften. Sie sind vorzugsweise mesogen und insbesondere flüssigkristallin.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen; dieser Rest ist unsubstituiert oder einfach oder mehrfach gleich oder verschieden mit Fluor, Chlor, Brom, Iod und/oder Cyano substituiert.

Sofern es sich bei diesem Alkylrest um einen gesättigten Rest handelt, wird er auch als "Alkanyl" bezeichnet ($C_aH_{2a+1}$-, wobei a eine ganze Zahl von 1 bis 15 ist und ein oder mehrere Wasserstoffatome durch Halogen, insbesondere Fluor, und/oder Cyano ersetzt sein können). Ferner umfaßt der Ausdruck "Alkyl" auch unsubstituierte oder entsprechend mit F, Cl, Br, I und/oder -CN ein- oder mehrfach gleich oder verschieden substituierte Kohlenwasserstoffreste, in denen eine oder mehrere $CH_2$-Gruppen derart durch -O- ("Alkoxy", "Oxaalkyl"), -S- ("Thioalkyl"), -CH=CH- ("Alkenyl"), -C≡C- ("Alkinyl"), -CO-O- oder -O-CO- ersetzt sein können, dass Heteroatome (O, S) nicht direkt miteinander verknüpft sind. Vorzugsweise ist Alkyl ein geradkettiger oder verzweigter, unsubstituierter oder substituierter Alkanyl-, Alkenyl- oder Alkoxyrest mit 1, 2, 3, 4, 5, 6, 7 oder 8 Kohlenstoffatomen. Sofern Alkyl einen Alkanylrest bedeutet, ist dieser bevorzugt Methyl, Ethyl, n-Propyl, i-Propyl, n-Butyl, i-Butyl, t-Butyl, n-Pentyl, Neopentyl, n-Hexyl, n-Heptyl, n-Octyl; $CF_3$, $CHF_2$, $CH_2F$; $CF_2CF_3$. Besonders bevorzugt ist der Alkanylrest geradkettig und unsubstituiert oder mit F substituiert.

Da in einem Alkylrest erfindungsgemäß eine oder mehrere $CH_2$-Gruppen durch -O- ersetzt sein können, umfaßt der Ausdruck "Alkyl" auch "Alkoxy"-beziehungsweise "Oxaalkyl"-Reste. Unter Alkoxy ist ein O-Alkyl-Rest zu verstehen, in dem das Sauerstoffatom direkt mit der durch den Alkoxyrest substituierten Gruppe oder dem substituierten Ring verbunden ist und Alkyl wie oben definiert ist; vorzugsweise ist Alkyl dann Alkanyl oder Alkenyl. Bevorzugte Alkoxyreste

sind Methoxy, Ethoxy, Propoxy, Butoxy, Pentoxy, Hexoxy, Heptoxy und Octoxy, wobei jeder dieser Reste auch substituiert sein kann, und zwar vorzugsweise mit einem oder mehreren Fluoratomen. Besonders bevorzugt ist Alkoxy $-OCH_3$, $-OC_2H_5$, $-O-n-C_3H_7$, $-O-n-C_4H_9$, $-O-t-C_4H_9$, $-OCF_3$, $-OCHF_2$, -OCHF or $-OCHFCHF_2$. Im Zusammenhang mit der vorliegenden Erfindung bedeutet der Ausdruck "Oxaalkyl" Alkylreste, in denen wenigstens eine nicht-terminale $CH_2$-Gruppe durch -O- derart ersetzt ist, dass keine benachbarten Heteroatome (O, S) vorliegen. Vorzugsweise umfaßt Oxaalkyl geradkettige Reste der Formel $-C_aH_{2a+1}-O-(CH_2)_b-$, wobei a und b jeweils unabhängig voneinander 1, 2, 3, 4, 5, 6, 7, 8, 9 oder 10 bedeuten; besonders bevorzugt ist a eine ganze Zahl von 1 bis 6 und b 1 oder 2.

Sofern in einem wie oben definerten Alkylrest eine oder mehrere $CH_2$-Gruppen durch Schwefel ersetzt sind, liegt ein "Thioalkyl"-Rest vor. Vorzugsweise umfaßt "Thioalkyl" einen geradkettigen Rest der Formel $C_aH_{2a+1}-S-(CH_2)_b-$, wobei a 1, 2, 3, 4, 5, 6, 7, 8, 9 oder 10 ist und b 0, 1, 2, 3, 4, 5, 6, 7, 8, 9 oder 10 ; besonders bevorzugt ist a eine ganze Zahl von 1 bis 6 und b 0, 1 oder 2. Der Thioalkylrest kann ebenfalls mit F, Cl, Br, I und/oder -CN substituiert sein und ist vorzugsweise unsubstituiert.

Im Zusammenhang der vorliegenden Erfindung bedeutet der Ausdruck "Alkenyl" einen wie oben definierten Alkylrest, in dem eine oder mehrere -CH=CH-Gruppen vorhanden sind. Sofern zwei -CH=CH-Gruppen in dem Rest vorhanden sind, kann dieser auch als "Alkadienyl" bezeichnet werden. Ein Alkenylrest kann 2 bis 15 (d.h. 2, 3, 4, 5, 6, 7, 8, 9, 10, 11, 12, 13, 14 oder 15) Kohlenstoffatome enthalten und ist verzweigtkettig oder vorzugsweise geradkettig. Der Rest ist unsubstituiert oder ein- oder mehrfach gleich oder verschieden mit F, Cl, Br, I und/oder CN substituiert. Ferner können eine oder mehrere $CH_2$-Gruppen jeweils unabhängig voneinander durch -O-, -S-, -C≡C-, -CO-O-, -OC-O- so ersetzt sein, dass Heteroatome (O, S) nicht direkt miteinander verbunden sind. Falls die CH=CH-Gruppe an beiden Kohlenstoffatomen einen anderen Rest als Wasserstoff trägt, etwa wenn sie eine nicht-terminale Gruppe ist, kann die CH=CH-Gruppe in zwei Konfigurationen vorliegen, nämlich als E-Isomer und als Z-Isomer. Im allgemeinen ist das E-Isomer (trans) bevorzugt. Vorzugsweise enthält der Alkenylrest 2, 3, 4, 5, 6 oder 7 Kohlenstoffatome und bedeutet Vinyl, 1 E-Propenyl, 1 E-Butenyl, 1 E-Pentenyl, 1 E-Hexenyl, 1 E-Heptenyl, 2-Propenyl, 2E-Butenyl, 2E-Pentenyl, 2E-Hexenyl, 2E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Besonders bevorzugte Alkenylreste sind Vinyl, 1E-Propenyl und 3E-Butenyl.

Falls in einem Alkylrest eine oder mehrere $CH_2$-Gruppen durch -C≡Cersetzt sind, liegt ein Alkinylrest vor. Auch die Ersetzung von einer oder mehreren $CH_2$-Gruppen durch -CO-O- oder -O-CO- ist möglich. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxycarbonyl)-propyl oder 4-(Methoxycarbonyl)-butyl.

Im Zusammenhang der vorliegenden Erfindung steht der Ausdruck "Aralkyl" für einen Aryl-Alkyl-Rest, d.h. für einen Rest, in dem ein Aryl-Substituent über eine Alkyl-Brücke mit einem Atom, einer Kette, einem anderen Rest oder einer funktionellen Gruppe verknüpft ist. Der Ausdruck "-O-Aralkyl" steht für einen Aryl-Alkoxy-Rest, d.h. für einen Rest, in dem ein Aryl-Alkyl-Rest über ein Sauerstoffatom mit einem Atom, einer Kette, einem anderen Rest oder einer funktionellen Gruppe verknüpft ist. Unter einem Aryl-Substituenten versteht man dabei einen aromatischen Kohlenwasserstoff mit 6 bis 18 Kohlenstoffatomen, der gegebenenfalls mit Halogen, $-NO_2$, Alkanyl- und/oder Alkoxy-Resten substituiert ist, insbesondere einen Phenyl- und einen Naphthylrest. Bei der Alkyl-Brücke handelt es sich vorzugsweise um einen gesättigten Kohlenwasserstoffrest, insbesondere um Methylen ($-CH_2-$) und Ethylen ($-CH_2CH_2-$). Bevorzugte Beispiele eines Aralkylrests sind Benzyl und Phenethyl. Bevorzugte Beispiele eines -O-Aralkylrests sind O-Benzyl ($-O-CH_2$Phenyl), O-Phenethyl ($-O-CH_2CH_2$-Phenyl) und O-(p-Nitrobenzyl).

Erfindungsgemäß bedeutet "Alkylenbrücke" eine aliphatische Kohlenwasserstoffkette, die unverzweigt oder verzweigt ist und die Formel $-C_nH_{2n}$ aufweist, beispielsweise $-CH_2CH_2-$, $-CH_2CH_2CH_2-$ oder $-CH_2C(CH_3)_2CH_2-$.

Der Ausdruck "Halogen" steht für Fluor, Chlor, Brom oder lod, während unter einem "halogenierten" Rest beziehungsweise einer "halogenierten" Verbindung ein Rest beziehungsweise eine Verbindung zu verstehen ist, der/die ein- oder mehrfach mit F, Cl, Br und/oder **I** substituiert ist.

Sofern Reste oder Substituenten der erfindungsgemäßen Pyranderivate bzw. die erfindungsgemäßen Pyranderivate selbst als optisch aktive oder stereoisomere Reste, Substituenten beziehungsweise Verbindungen vorliegen können, weil sie beispielsweise ein asymmetrisches Zentrum aufweisen, so sind diese von der vorliegenden Erfindung mit umfaßt. Dabei ist es selbstverständlich, daß die erfindungsgemäßen Pyranderivate der allgemeinen Formeln I und IV in isomerenreiner Form, zum Beispiel als reine Enantiomeren, Diastereomeren, E- beziehungsweise Z-Isomeren, trans- bzw. cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Eine bevorzugte Klasse erfindungsgemäßer Verbindungen der Formel I wird von Pyranderivaten gebildet, bei denen W in Formel I für eine Carbonylgruppe, d.h. für -C(=O)- steht. Es handelt sich dann um Lactone der allgemeinen Formel I-A:

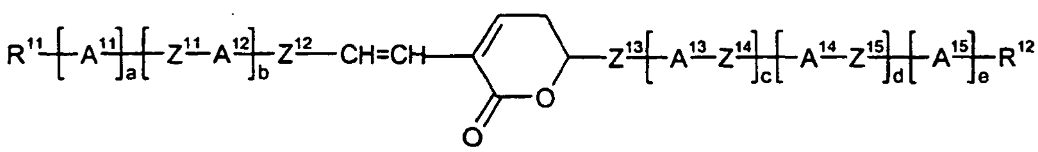

I-A

wobei a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ die gleiche Bedeutung wie für Formel I oben besitzen.

Eine andere bevorzugte Klasse erfindungsgemäßer Verbindungen der Formel I wird von Pyranderivaten gebildet, bei denen W in Formel I für eine Methylengruppe, d.h. für $-CH_2-$ steht. Es handelt sich dann um Dihydropyrane der allgemeinen Formel I-B:

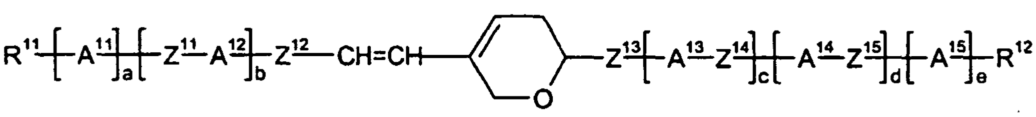

I-B

wobei a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ die gleiche Bedeutung wie für Formel I oben besitzen.

Im Zusammenhang der vorliegenden Erfindung werden die erfindungsgemäßen Verbindungen der Formel I auch als "Pyranderivate" bezeichnet; der Ausdruck "Pyranderivate" umfasst sowohl Pyrane der Formel I-B als auch Lactone der Formel I-A.

Bevorzugt sind ferner Verbindungen der Formel I, bei denen $Z^{12}$ eine Einfachbindung bedeutet. Weiterhin ist bevorzugt, dass gilt: $a+b+c+d+e \leq 3$, d.h., dass die erfindungsgemäßen Verbindungen der Formel I nicht mehr als insgesamt vier Ringsysteme enthalten. Vorzugsweise ist $R^{11}$ H, ein geradkettiger Alkenyl- oder insbesondere Alkanylrest mit 1 bis 7 Kohlenstoffatomen, und ist $R^{12}$ vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. Außerdem ist es, wenn $Z^{13}$ für $CF_2O$ steht, bevorzugt, dass die Difluoroxymethylenbrücke mit einen aromatischen Ring $A^{13}$ (c = 1), $A^{14}$ (c = 0, d = 1) beziehungsweise $A^{15}$ (c = d = 0, e = 1), d. h. einem 1,4-Phenylen-, 2,6-Naphthylenrest oder Phenanthrenylrest, verbunden ist.

Eine weitere bevorzugte Gruppe erfindungsgemäßer Verbindungen umfasst solche Verbindungen, in denen in Formel a und b zugleich null sind, $R^{11}$ für H steht und $Z^{12}$ eine Einfachbindung bedeutet, das heißt Pyrane der Formel I-C

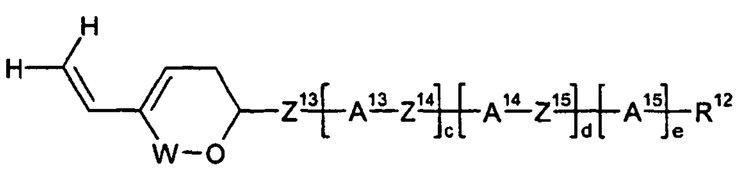

I-C

wobei c, d, e, W, $R^{12}$, $Z^{13}$, $Z^{14}$, $Z^{15}$, $A^{13}$, $A^{14}$ und $A^{15}$ wie für Formel I definiert sind. Diese Verbindungen eignen sich insbesondere als Ausgangsverbindungen zur Herstellung weiterer Substanzen, die als einen Molekülbestandteil einen 2,5-disubstitutierten Pyranring aufweisen; beispielhaft seien Verbindungen genannt, die durch Reaktion der exocyclischen C=C-Doppelbindung in 5-Position des Pyranring einer Verbindung der Formel I-C mit geeigneten Reaktionspartnern erhalten werden können (siehe unten).

Bei passender Auswahl der jeweiligen Bedeutung von c, d, e, W, $R^{12}$, $Z^{13}$, $Z^{14}$, $Z^{15}$, $A^{13}$ , $A^{14}$ und $A^{15}$ weisen Verbindungen der Formel I-C auch mesogene Eigenschaften auf, so dass sie zum Beispiel Verwendung finden in flüssigkristallinen Medien zum Einsatz in beispielsweise elektrooptischen Anzeigevorrichtungen.

Bevorzugte Untergruppen von Verbindungen der Formel I-C sind Verbindungen der Formel I-CA und insbesondere der Formel I-CB:

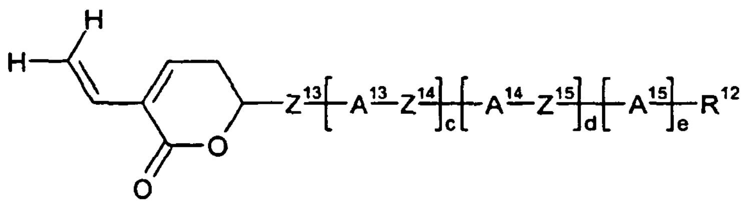

I-CA

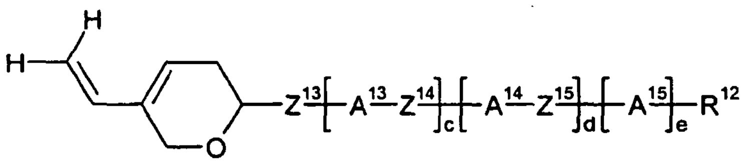

I-CB

wobei c, d, e, $R^{12}$, $Z^{13}$, $Z^{14}$, $Z^{15}$, $A^{13}$, $A^{14}$ und $A^{15}$ die gleiche Bedeutung wie für Formel I besitzen.

Unter den Verbindungen der Formel I-CB sind besonders bevorzugt Verbindungen der Formeln I-CBI ($Z^{13}$ = Einfachbindung), I-CBII, I-CBIII, I-CBIV und I-CBV:

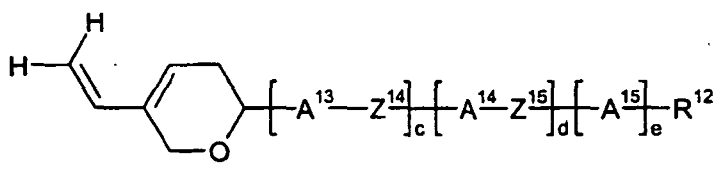

I-CBI

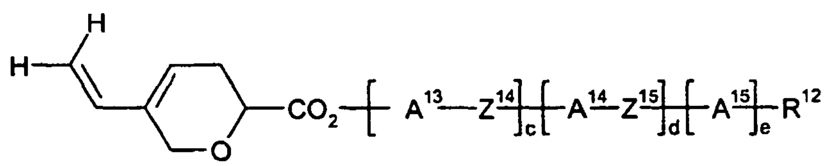

I-CBII

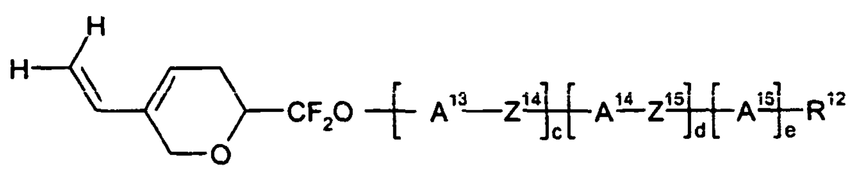

I-CBIII

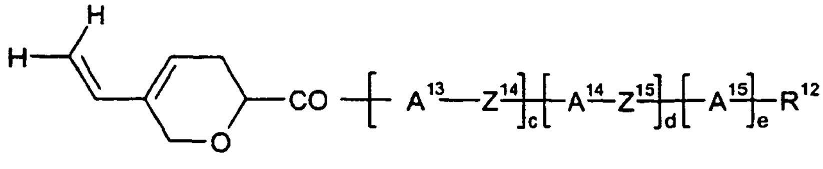

I-CBIV

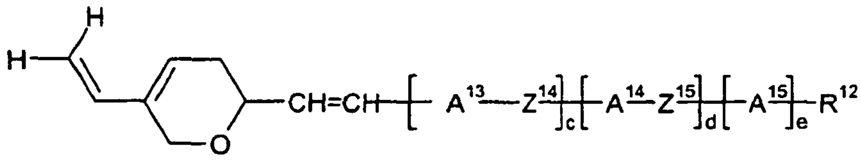

I-CBV

worin c, d, e, $R^{12}$, $Z^{14}$, $Z^{15}$, $A^{13}$, $A^{14}$ und $A^{15}$ die gleiche Bedeutung wie für Formel I besitzen.

Ganz besonders bevorzugte Verbindungen der allgemeinen Formel I-CBI sind solche der Formeln I-CBIa, I-CBIb, I-CBIc, I-CBId, I-CBIe, I-CBIf, I-CBIg, I-CBIh, I-CBIi, I-CBIj, I-CBIk, I-CBIm und I-CBIn:

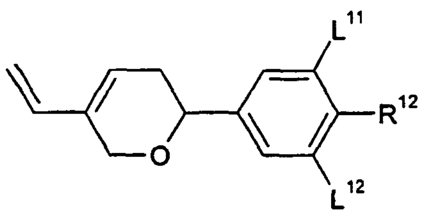

I-CBIa

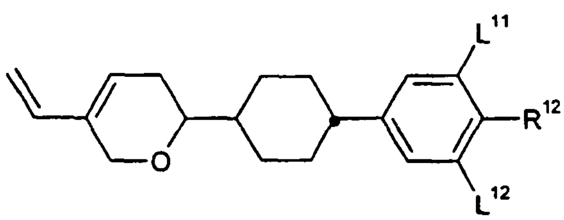

I-CBIb

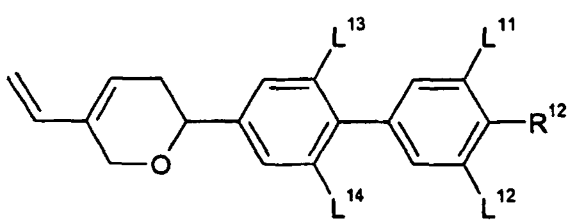

I-CBIc

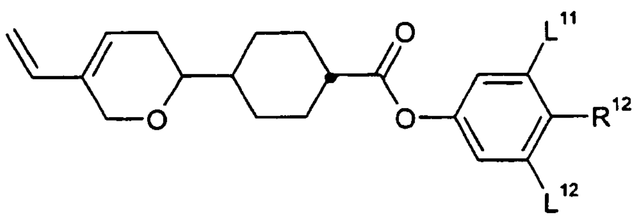

I-CBId

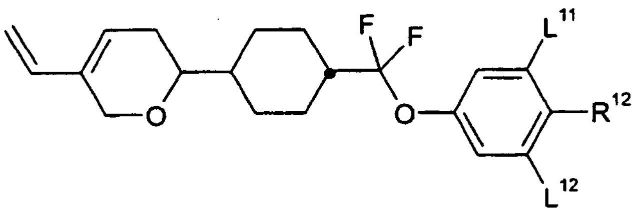

I-CBIe

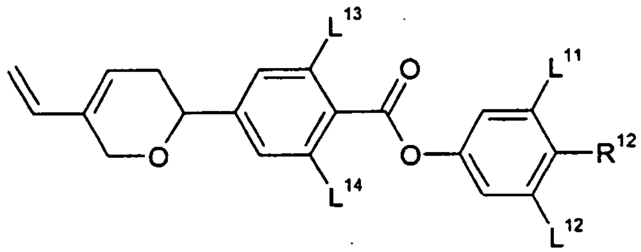

I-CBIf

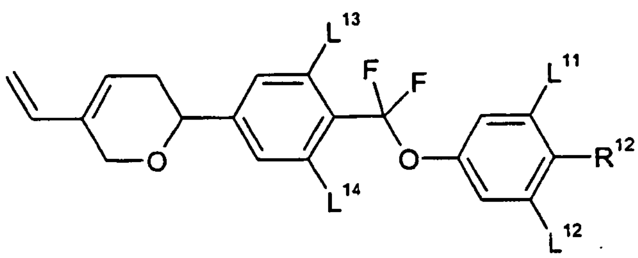

I-CBIg

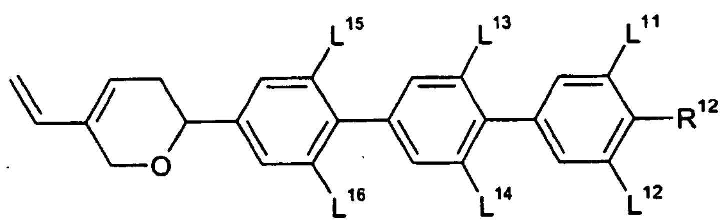

I-CBIh

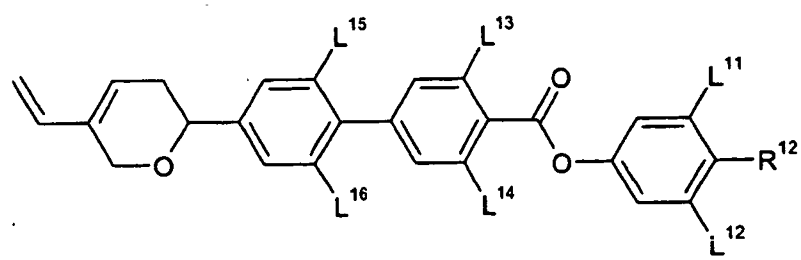

I-CBIi

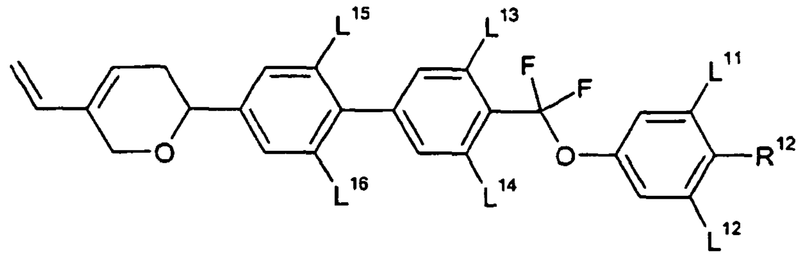

I-CBIj

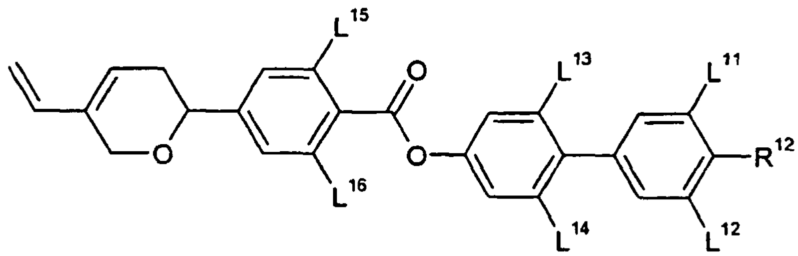

I-CBIk

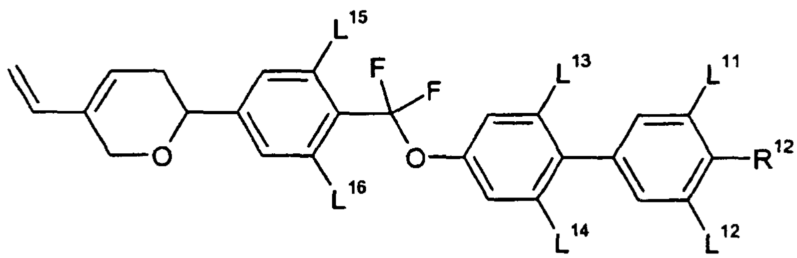

I-CBIm

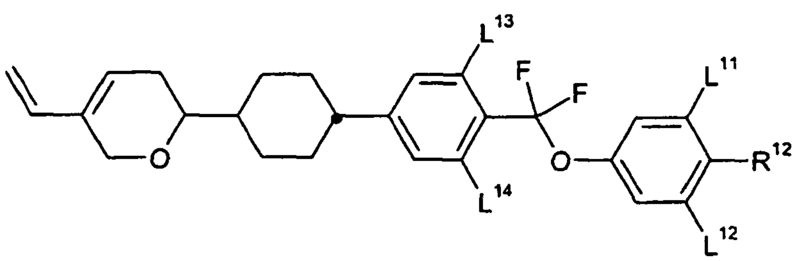

I-CBIn

wobei $R^{12}$ die für Formel I angegebene Bedeutung besitzt, vorzugsweise -CN, Halogen, insbesondere Fluor, oder gegebenenfalls mit Halogen, insbesondere Fluor, einfach oder mehrfach substituiertes geradkettiges Alkanyl oder Alkoxy mit 1 bis 5 Kohlenstoffatomen ist und $L^{11}$, $L^{12}$, $L^{13}$, $L^{14}$, $L^{15}$ und $L^{16}$ jeweils unabhängig voneinander H oder F bedeuten.

Beispielhafte Verbindungen der Formeln I-CBIa, I-CBIb, I-CBId und I-CBIe sind solche, die die in Tabelle 1 unten für die Reste $R^{12}$, $L^{11}$ und $L^{12}$ angegebenen Bedeutungen aufweisen.

Beispielhafte Verbindungen der Formeln I-CBIc, I-CBIf, I-CBIg und I-CBIn sind solche, die die in Tabelle 2 unten für die Reste $R^{12}$, $L^{11}$, $L^{12}$, $L^{13}$ und $L^{14}$ angegebenen Bedeutungen aufweisen.

Beispielhafte Verbindungen der Formeln I-CBIh, I-CBIi, I-CBIj, I-CBIk und I-CBIm sind solche, die die in Tabelle 3 unten für die Reste $R^{12}$, $L^{11}$, $L^{12}$, $L^{13}$, $L^{14}$, $L^{15}$ und $L^{16}$ angegebenen Bedeutungen aufweisen.

Ganz besonders bevorzugte Verbindungen der allgemeinen Formel I-CBII sind solche der Formel I-CBIIa:

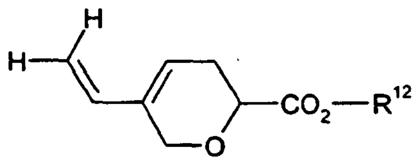

I-CBIIa

worin $R^{12}$ H, Alkanyl, Alkenyl oder Aralkyl, bevorzugt H, Alkanyl oder Aralkyl, insbesondere H oder Alkanyl ist. Beispielhafte Verbindungen der Formel I-CBIIa sind solche, die die in Tabelle 4 unten für $R^{12}$ wiedergegebenen Bedeutungen aufweisen.

Weitere ganz besonders bevorzugte Verbindungen der Formel I-CBII sind solche der Formeln I-CBIIb, I-CBIIc, I-CBIId, I-CBIIe und I-CBIIf, die unter anderem durch Veresterung beziehungsweise Umesterung von Verbindungen der Formel I-CBIIa oder aktivierten Derivaten, zum Beispiel Carbonsäurehalogeniden, mit den entsprechenden Alkoholen zugänglich sind:

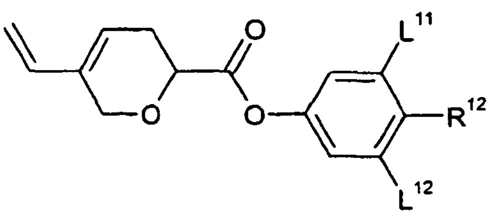

I-CBIIb

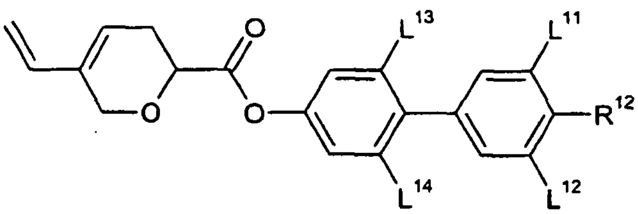

I-CBIIc

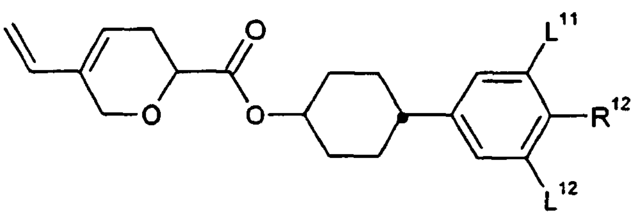

I-CBIId

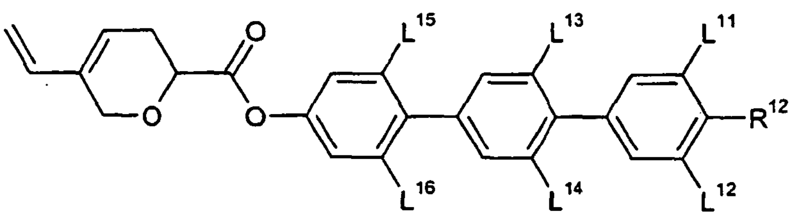

I-CBIIe

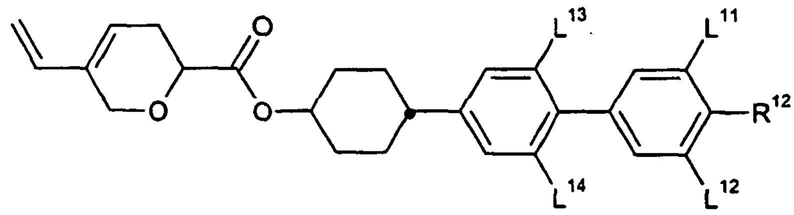

I-CBIIf

wobei $R^{12}$ die für Formel I angegebene Bedeutung besitzt und vorzugsweise für Halogen, insbesondere Fluor, oder gegebenenfalls mit Halogen, insbesondere Fluor, einfach oder mehrfach substituiertes geradkettiges Alkanyl oder Alkoxy mit 1 bis 5 Kohlenstoffatomen steht, während $L^{11}$, $L^{12}$, $L^{13}$, $L^{14}$, $L^{15}$ und $L^{16}$ jeweils unabhängig voneinander H oder F bedeuten.

Beispielhafte Verbindungen der Formeln I-CBIIb und I-CBIId sind solche, die die in Tabelle 1 unten für die Reste $R^{12}$, $L^{11}$ und $L^{12}$ angegebenen Bedeutungen aufweisen.

Beispielhafte Verbindungen der Formeln I-CBIIc und I-CBIIf sind solche, die die in Tabelle 2 unten für die Reste $R^{12}$, $L^{11}$, $L^{12}$, $L^{13}$ und $L^{14}$ angegebenen Bedeutungen aufweisen.

Beispielhafte Verbindungen der Formeln I-CBIIe sind solche, die die in Tabelle 3 unten für die Reste $R^{12}$, $L^{11}$, $L^{12}$, $L^{13}$, $L^{14}$, $L^{15}$ und $L^{16}$ angegebenen Bedeutungen aufweisen.

Ganz besonders bevorzugte Verbindungen der Formel I-CBIII sind solche der Formeln I-CBIIIa, I-CBIIIb, I-CBIIIc, I-CBIIId, I-CBIIIe und I-CBIIIf:

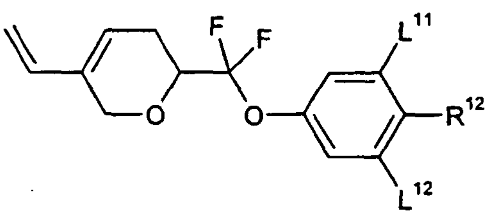

I-CBIIIa

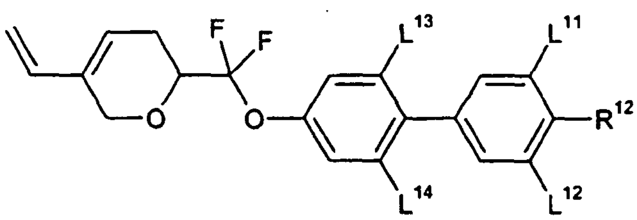

I-CBIIIb

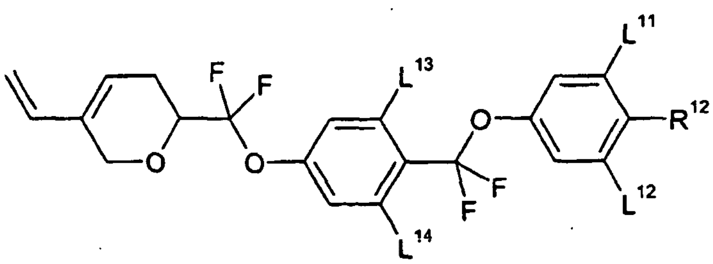

I-CBIIIc

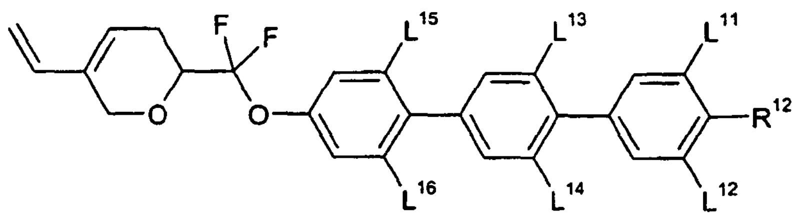

I-CBIIId

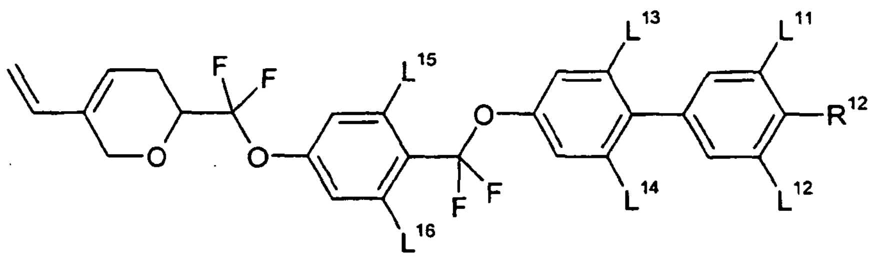

I-CBIIIe

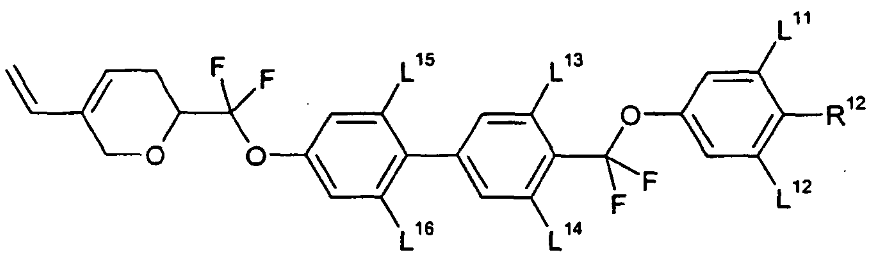

I-CBIIIf

wobei $R^{12}$ die für Formel I angegebene Bedeutung besitzt und vorzugsweise für Halogen, insbesondere Fluor, oder gegebenenfalls mit Halogen, insbesondere Fluor, einfach oder mehrfach substituiertes geradkettiges Alkanyl oder Alkoxy mit 1 bis 5 Kohlenstoffatomen steht, während $L^{11}$, $L^{12}$, $L^{13}$, $L^{14}$, $L^{15}$ und $L^{16}$ jeweils unabhängig voneinander H oder F bedeuten.

Beispielhafte Verbindungen der Formel I-CBIIIa sind solche, die die in Tabelle 1 unten für die Reste $R^{12}$, $L^{11}$ und $L^{12}$ angegebenen Bedeutungen aufweisen.

**Tabelle 1**

| I-CBIa/I-CBIb/I-CBId/I-CBIe/I-CBIIb/I-CBIId/I-CBIIIa-Nr. | $L^{11}$ | $L^{12}$ | $R^{12}$ |
|---|---|---|---|
| -1 | H | H | F |
| -2 | H | H | $CF_3$ |
| -3 | H | H | $OCF_3$ |
| -4 | H | H | $OCHF_2$ |
| -5 | H | H | CN |
| -6 | F | H | F |
| -7 | F | H | $CF_3$ |
| -8 | F | H | $OCF_3$ |
| -9 | F | H | $OCHF_2$ |
| -10 | F | H | CN |
| -11 | F | F | F |
| -12 | F | F | $CF_3$ |
| -13 | F | F | $OCF_3$ |
| -14 | F | F | $OCHF_2$ |
| -15 | F | F | CN |

Beispielhafte Verbindungen der Formeln I-CBIIIb und I-CBIIIc sind solche, die die in Tabelle 2 unten für die Reste $R^{12}$, $L^{11}$, $L^{12}$, $L^{13}$ und $L^{14}$ angegebenen Bedeutungen aufweisen.

**Tabelle 2**

| I-CBIc/I-CBIf/I-CBIg/I-CBIn/I-CBIIc/I-CBIIf/ I-CBIIIb/I-CBIIIc-Nr. | $L^{13}$ | $L^{14}$ | $L^{11}$ | $L^{12}$ | $R^{12}$ |
|---|---|---|---|---|---|
| -1 | H | H | H | H | F |
| -2 | H | H | H | H | $CF_3$ |
| -3 | H | H | H | H | $OCF_3$ |
| -4 | H | H | H | H | $OCHF_2$ |
| -5 | H | H | H | H | CN |
| -6 | H | H | H | F | F |
| -7 | H | H | H | F | $CF_3$ |
| -8 | H | H | H | F | $OCF_3$ |
| -9 | H | H | H | F | $OCHF_2$ |
| -10 | H | H | H | F | CN |
| -11 | H | H | F | F | F |
| -12 | H | H | F | F | $CF_3$ |
| -13 | H | H | F | F | $OCF_3$ |
| -14 | H | H | F | F | $OCHF_2$ |
| -15 | H | H | F | F | CN |
| -16 | H | F | F | F | F |
| -17 | H | F | F | F | $CF_3$ |
| -18 | H | F | F | F | $OCF_3$ |
| -19 | H | F | F | F | $OCHF_2$ |
| -20 | H | F | F | F | CN |
| -21 | F | F | F | F | F |
| -22 | F | F | F | F | $CF_3$ |
| -23 | F | F | F | F | $OCF_3$ |
| -24 | F | F | F | F | $OCHF_2$ |
| -25 | F | F | F | F | CN |
| -26 | F | H | F | H | F |
| -27 | F | H | F | H | $CF_3$ |
| -28 | F | H | F | H | $OCF_3$ |
| -29 | F | H | F | H | $OCHF_2$ |
| -30 | F | H | F | H | CN |

Beispielhafte Verbindungen der Formeln I-CBIlld, I-CBIlle und I-CBIllf sind solche, die die in Tabelle 3 unten für die Reste $R^{12}$, $L^{11}$, $L^{12}$, $L^{13}$, $L^{14}$, $L^{15}$ und $L^{16}$ angegebenen Bedeutungen aufweisen.

**Tabelle 3**

| I-CBIh/I-CBIl/I-CBIj/I-CBik/I-CBIm/ I-CBIle/I-CBIIId/I-CBIIIe/ICBIIIf-Nr. | $L^{15}$ | $L^{16}$ | $L^{13}$ | $L^{14}$ | $L^{11}$ | $L^{12}$ | $R^{12}$ |
|---|---|---|---|---|---|---|---|
| -1 | H | H | H | H | H | H | F |
| -2 | H | H | H | H | H | H | $CF_3$ |
| -3 | H | H | H | H | H | H | $OCF_3$ |
| -4 | H | H | H | H | H | H | $OCHF_2$ |
| -5 | H | H | H | H | H | H | CN |
| -6 | H | H | H | H | F | H | F |
| -7 | H | H | H | H | F | H | $CF_3$ |
| -8 | H | H | H | H | F | H | $OCF_3$ |
| -9 | H | H | H | H | F | H | $OCHF_2$ |
| -10 | H | H | H | H | F | H | CN |
| -11 | H | H | H | H | F | F | F |

(fortgesetzt)

| I-CBIh/I-CBII/I-CBIj/I-CBik/I-CBIm/ I-CBIIe/I-CBIIId/I-CBIIIe/ICBIIIf-Nr. | $L^{15}$ | $L^{16}$ | $L^{13}$ | $L^{14}$ | $L^{11}$ | $L^{12}$ | $R^{12}$ |
|---|---|---|---|---|---|---|---|
| -12 | H | H | H | H | F | F | $CF_3$ |
| -13 | H | H | H | H | F | F | $OCF_3$ |
| -14 | H | H | H | H | F | F | $OCHF_2$ |
| -15 | H | H | H | H | F | F | CN |
| -16 | H | H | F | H | F | F | F |
| -17 | H | H | F | H | F | F | $CF_3$ |
| -18 | H | H | F | H | F | F | $OCF_3$ |
| -19 | H | H | F | H | F | F | $OCHF_2$ |
| -20 | H | H | F | H | F | F | CN |
| -21 | H | H | F | F | F | F | F |
| -22 | H | H | F | F | F | F | $CF_3$ |
| -23 | H | H | F | F | F | F | $OCF_3$ |
| -24 | H | H | F | F | F | F | $OCHF_2$ |
| -25 | H | H | F | F | F | F | CN |
| -26 | F | H | F | F | F | F | F |
| -27 | F | H | F | F | F | F | $CF_3$ |
| -28 | F | H | F | F | F | F | $OCF_3$ |
| -29 | F | H | F | F | F | F | $OCHF_2$ |
| -30 | F | H | F | F | F | F | CN |
| -31 | H | H | F | H | F | H | F |
| -32 | H | H | F | H | F | H | $CF_3$ |
| -33 | H | H | F | H | F | H | $OCF_3$ |
| -34 | H | H | F | H | F | H | $OCHF_2$ |
| -35 | H | H | F | H | F | H | CN |

**Tabelle 4**

| I-CBIIa-Nr. | $R^{12}$ | I-CBIIa-Nr. | $R^{12}$ |
|---|---|---|---|
| -1 | H | -6 | $n\text{-}C_4H_9$ |
| -2 | $CH_3$ | -7 | $t\text{-}C_4H_9$ |
| -3 | $C_2H_5$ | -8 | $CH_2$-Phenyl |
| -4 | $n\text{-}C_3H_7$ | -9 | $CH_2CH_2$-Phenyl |
| -5 | $i\text{-}C_3H_7$ | -10 | $CH_2\text{-}CH{=}CH_2$ |

Ganz besonders bevorzugte Verbindungen der Formel I-CBIV sind solche der Formel I-CBIVa:

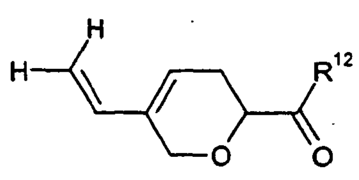

I-CBIVa

worin $R^{12}$ H, Alkanyl, Alkenyl oder Aralkyl ist. Besonders bevorzugt ist dabei die Verbindung I-CBIVa-1, in der $R^{12}$ H bedeutet.

Eine weitere Gruppe bevorzugter Verbindungen der vorliegenden Erfindung sind solche der Formel I, bei denen $Z^{12}$ eine Einfachbindung ist und - im Unterschied zu den Verbindungen der Formel I-C - die exocyclische C=C-Doppelbindung in 5-Position des zentralen Pyranrings über die Einfachbindung $Z^{12}$ mit einem Rest verbunden ist, der von H

verschieden ist (Formel I-D). Das bedeutet, dass die mit dem zentralen Pyranring unmittelbar verknüpfte exocyclische CH=CH-Gruppe direkt entweder mit dem Cyclohexyl- oder Dioxanring der Gruppe $-Z^{11}-A^{12}-$ verbunden ist (wenn b = 1) (Formel I-DA) oder direkt mit dem Cyclohexyl- oder Dioxanring $A^{11}$ verbunden ist (wenn b = 0 und a = 1) (Formel I-DB) oder direkt mit dem Rest $R^{11}$, der nicht H ist, verbunden ist (wenn a = b = 0) (Formel I-DC):

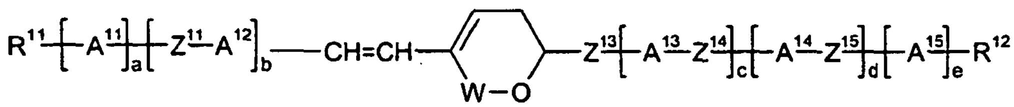

I-D

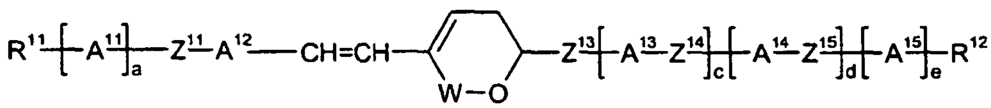

I-DA

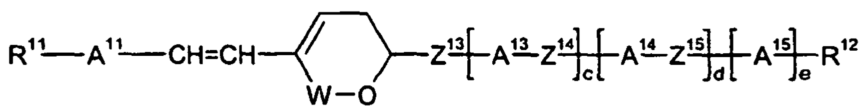

I-DB

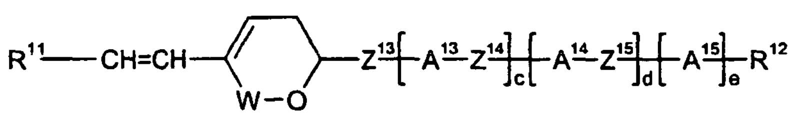

I-DC

wobei a, b, c, d, e, W, $R^{11}$, $R^{12}$, $Z^{11}$, $Z^{13}$, $Z^{14}$, $Z^{15}$, $A^{11}$, $A^{12}$, $A^{13}$, $A^{14}$ und $A^{15}$ ansonsten die gleiche Bedeutung wie in Formel I oben besitzen.

Bevorzugte Untergruppen der Formel I-DA werden dabei von Verbindungen der Formel I-DAA mit W = -C(=O)- und insbesondere von Verbindungen der Formel I-DAB mit W = $-CH_2-$ gebildet:

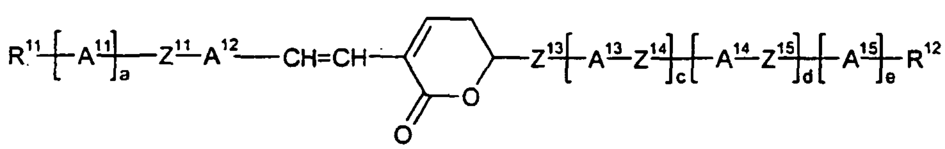

I-DAA

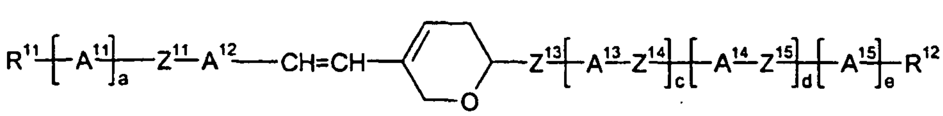

I-DAB

wobei a, c, d, e, $R^{11}$, $R^{12}$, $Z^{11}$, $Z^{13}$, $Z^{14}$, $Z^{15}$, $A^{11}$, $A^{12}$, $A^{13}$, $A^{14}$ und $A^{15}$ die gleiche Bedeutung wie für Formel I-D oben besitzen.

Unter den Verbindungen der Formel I-DAB sind besonders bevorzugt Verbindungen der Formeln I-DABI, I-DABII und I-DABIII:

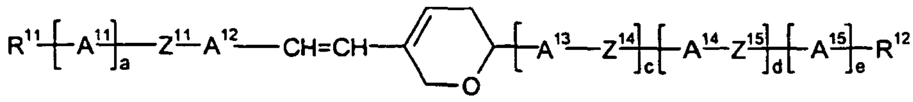

I-DABI

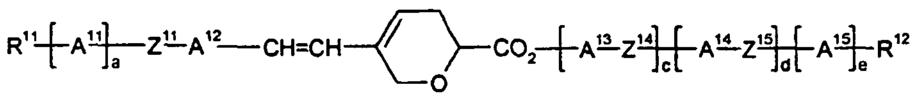

I-DABII

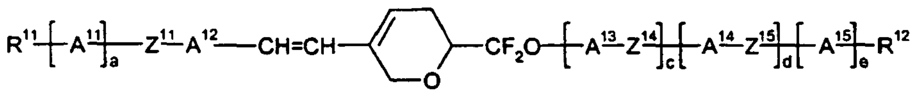

I-DABIII

worin a, c, d, e, $R^{11}$, $R^{12}$, $Z^{11}$, $Z^{14}$, $Z^{15}$ , $A^{11}$, $A^{12}$, $A^{13}$ , $A^{14}$ und $A^{15}$ die gleiche Bedeutung wie für Formel I-D besitzen.

Ganz besonders bevorzugte Verbindungen der allgemeinen Formel I-DABI, I-DABII und I-DABIII sind solche der Formeln I-DABIa, I-DABIIa und I-DABIIIa:

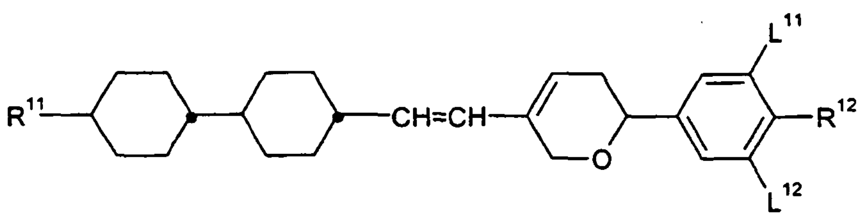

I-DABIa

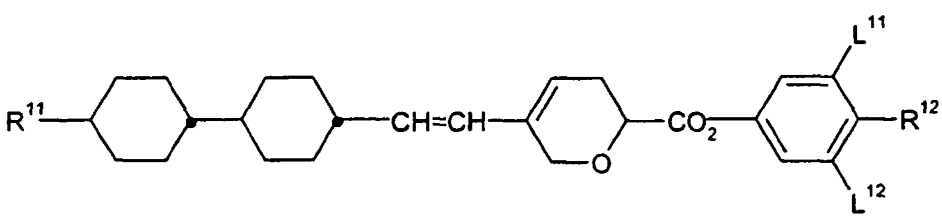

I-DABIIa

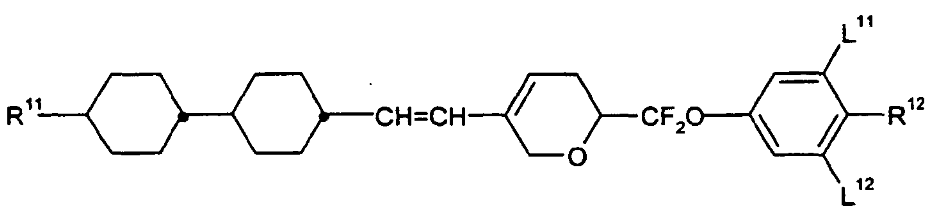

I-DABIIIa

wobei $R^{11}$ und $R^{12}$ die für Formel I-D angegebene Bedeutung besitzen und $L^{11}$ und $L^{12}$ jeweils unabhängig voneinander H oder F bedeuten.

Beispielhafte Verbindungen der Formeln I-DABIa, I-DABIIa und I-DABIIIa sind solche, die die in Tabelle 5 unten für die Reste $R^{11}$, $R^{12}$, $L^{11}$ und $L^{12}$ angegebenen Bedeutungen aufweisen.

Bevorzugte Untergruppen der Formel I-DB werden von Verbindungen der Formel I-DBA mit W = -C(=O)- und insbesondere von Verbindungen der Formel I-DBB mit W = $-CH_2-$ gebildet:

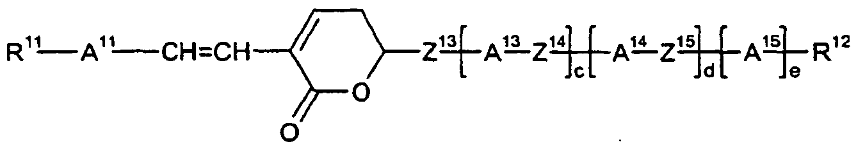

I-DBA

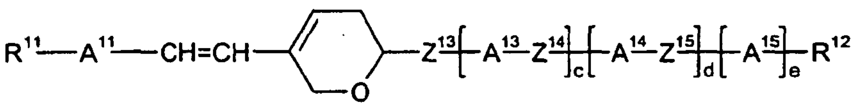

I-DBB

wobei c, d, e, $R^{11}$, $R^{12}$, $Z^{13}$, $Z^{14}$, $Z^{15}$, $A^{11}$, $A^{13}$, $A^{14}$ und $A^{15}$ die gleiche Bedeutung wie für Formel I-D oben besitzen.

Unter den Verbindungen der Formel I-DBB sind besonders bevorzugt Verbindungen der Formeln I-DBBI, I-DBBII und I-DBBIII:

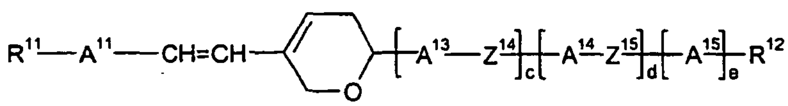

I-DBBI

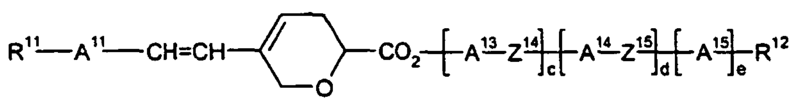

I-DBBII

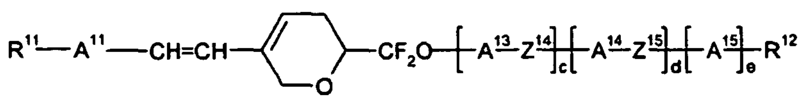

I-DBBIII

worin c, d, e, $R^{11}$, $R^{12}$, $Z^{14}$, $Z^{15}$, $A^{11}$, $A^{13}$, $A^{14}$ und $A^{15}$ die gleiche Bedeutung wie für Formel I-D besitzen. $R^{11}$ ist vorzugsweise ein geradkettiger Alkenyl- oder insbesondere Alkanylrest mit 1 bis 7 Kohlenstoffatomen.

Ganz besonders bevorzugte Verbindungen der allgemeinen Formeln I-DBBI, I-DBBII und I-DBBIII sind solche der Formeln I-DBBIa, I-DBBIb, I-DBBIc, I-DBBId, I-DBBIe, I-DBBIf, I-DBBIg, I-DBBIIa, I-DBBIIb, I-DBBIIIa und I-DBBIIIb:

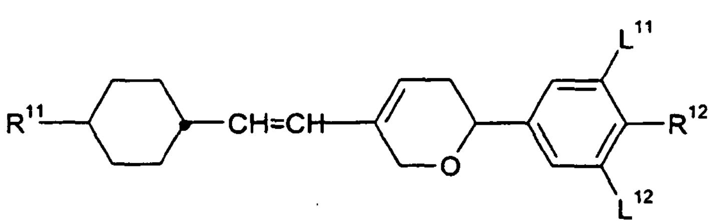

I-DBBIa

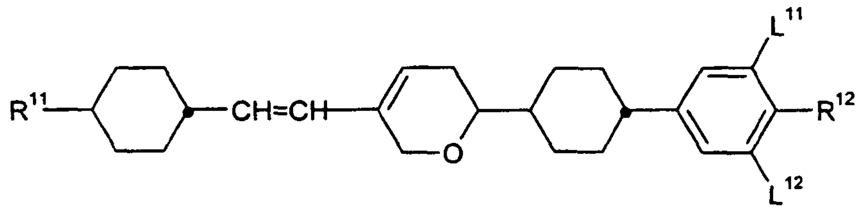

I-DBBIb

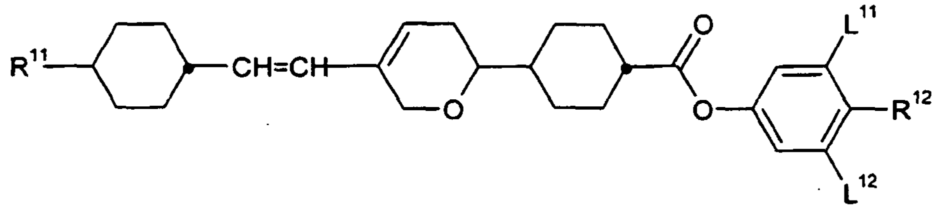

I-DBBIc

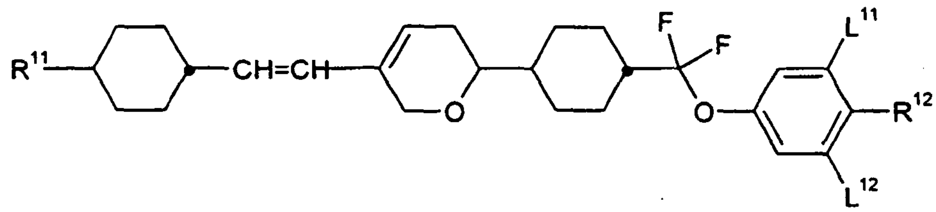

I-DBBId

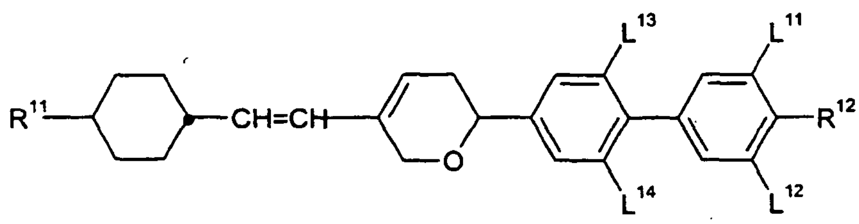

I-DBBIe

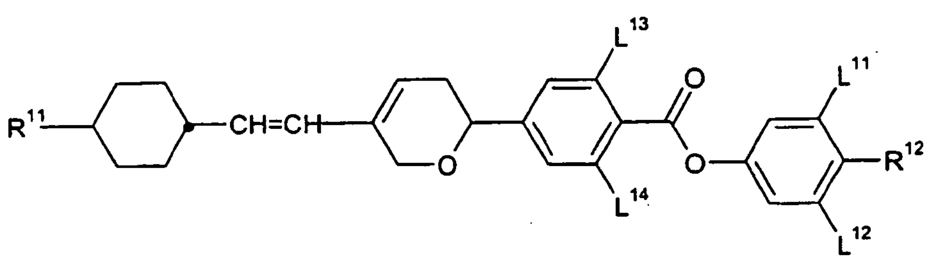

I-DBBIf

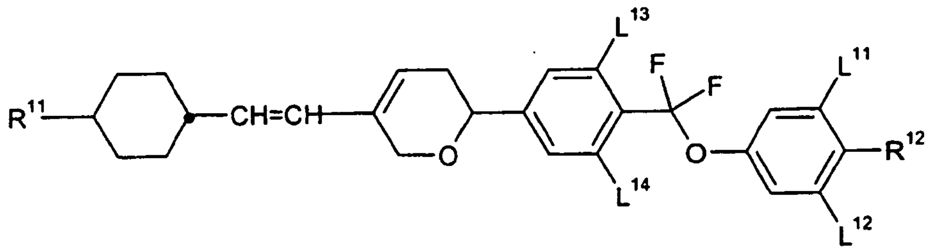

I-DBBIg

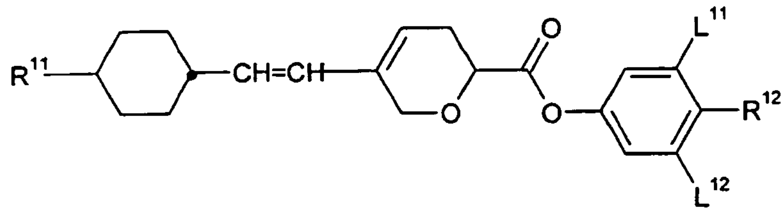

I-DBBIIa

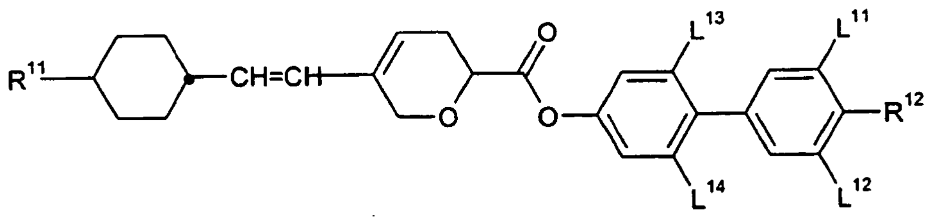

I-DBBIIb

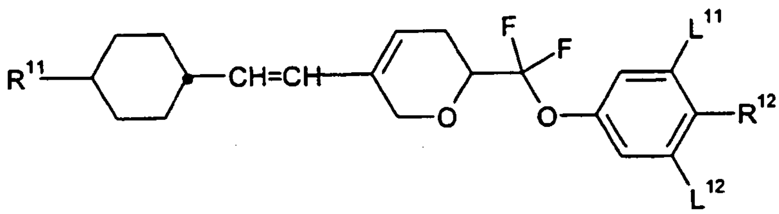

I-DBBIIIa

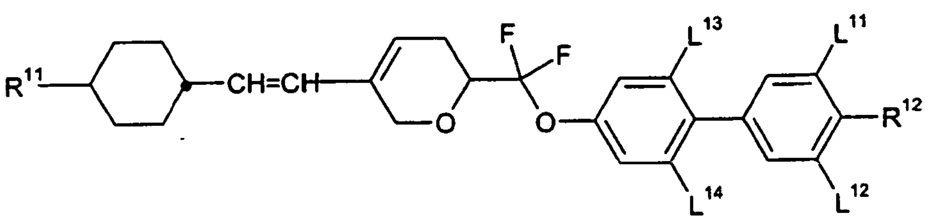

I-DBBIIIb

wobei $R^{11}$ und $R^{12}$ die für Formel I-D angegebene Bedeutung besitzen und $L^{11}$, $L^{12}$, $L^{13}$ und $L^{14}$ jeweils unabhängig voneinander H oder F bedeuten. $R^{11}$ ist vorzugsweise ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen.

Beispielhafte Verbindungen der Formeln I-DBBIa, I-DBBIb, I-DBBIc, I-DBBId, I-DBBIIa und I-DBBIIIa sind solche, die die in Tabelle 5 unten für die Reste $R^{11}$, $R^{12}$, $L^{11}$ und $L^{12}$ angegebenen Bedeutungen aufweisen.

Beispielhafte Verbindungen der Formeln I-DBBIe, I-DBBIf, I-DBBIg, I-DBBIIb, und I-DBBIIIb sind solche, die die in Tabelle 6 unten für die Reste $R^{11}$, $R^{12}$ , $L^{11}$, $L^{12}$, $L^{13}$ und $L^{14}$ angegebenen Bedeutungen aufweisen.

Bevorzugte Untergruppen der Formel I-DC werden von Verbindungen der Formel I-DCA mit W = -C(=O)- und insbesondere von Verbindungen der Formel I-DCB mit W = $-CH_2-$ gebildet:

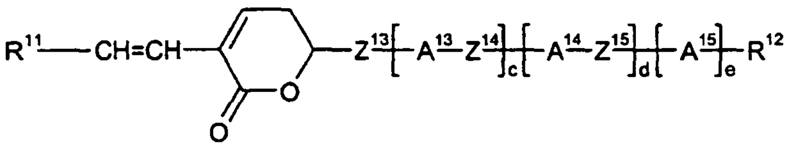

I-DCA

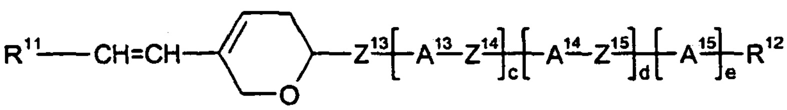

I-DCB

wobei c, d, e, $R^{11}$, $R^{12}$, $Z^{13}$, $Z^{14}$, $Z^{15}$, $A^{13}$, $A^{14}$ und $A^{15}$ die gleiche Bedeutung wie für Formel I-D oben besitzen.

Unter den Verbindungen der Formel I-DCB sind besonders bevorzugt Verbindungen der Formeln I-DCBI, I-DCBII und I-DCBIII:

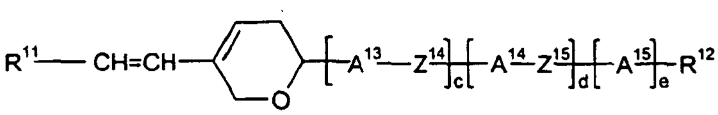

I-DCBI

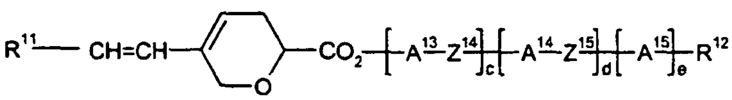

I-DCBII

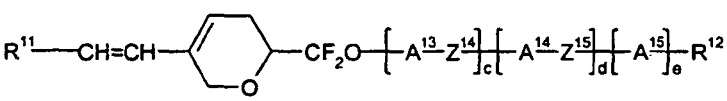

I-DCBIII

worin c, d, e, $R^{11}$, $R^{12}$, $Z^{14}$, $Z^{15}$, $A^{13}$, $A^{14}$ und $A^{15}$ die gleiche Bedeutung wie für Formel I-D besitzen.

Ganz besonders bevorzugte Verbindungen der allgemeinen Formel I-DCBI, I-DCBII und I-DCBIII sind solche der Formeln I-DCBIa, I-DCBIb, I-DCBIc, I-DCBId, I-DCBIe, I-DCBIf, I-DCBIg, I-DCBIIa, I-DCBIIb, I-DCBIIc, I-DCBIIIa und I-DCBIIIb:

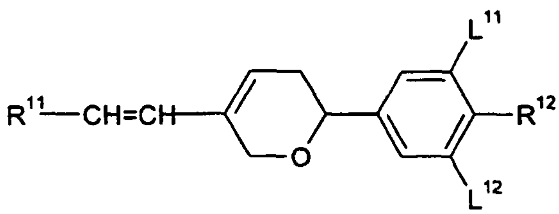

I-DCBIa

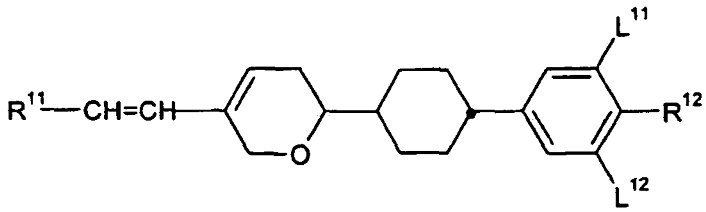

I-DCBIb

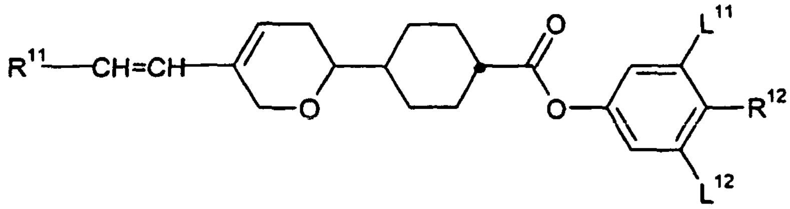

I-DCBIc

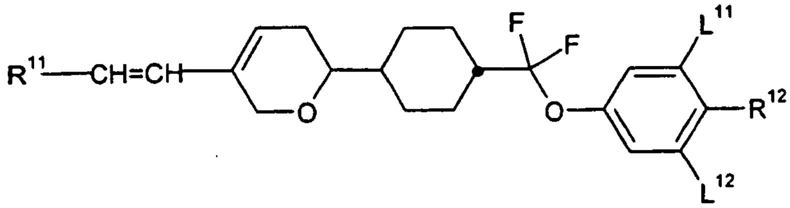

I-DCBId

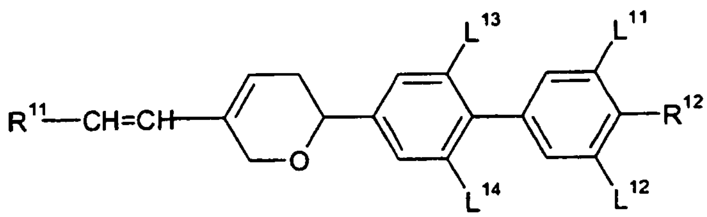

I-DCBIe

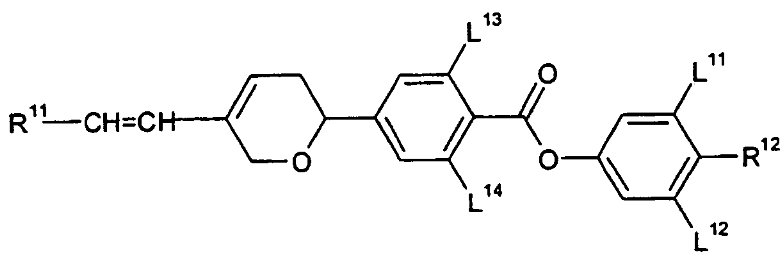

I-DCBIf

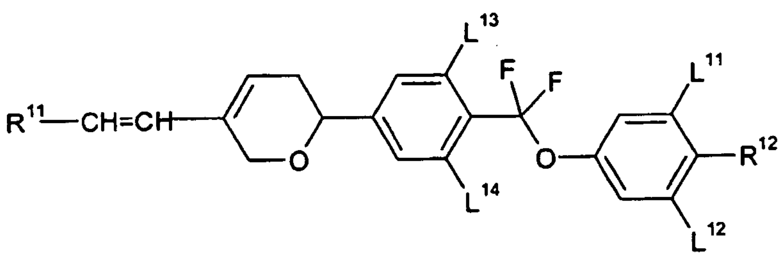

I-DCBIg

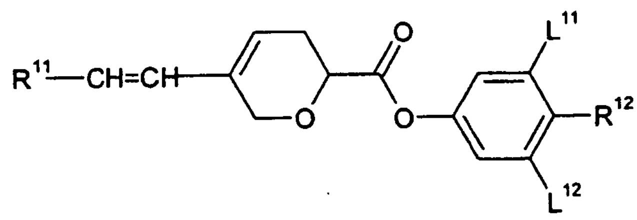

I-DCBIIa

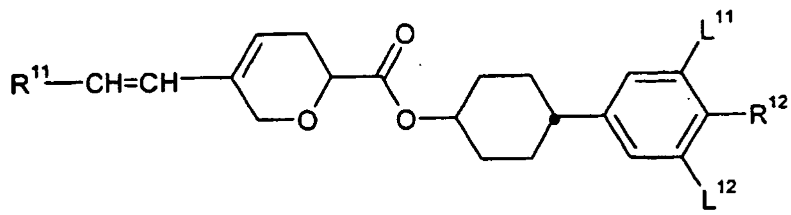

I-DCBIIb

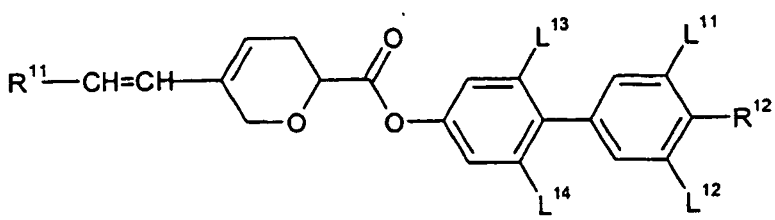

I-DCBIIc

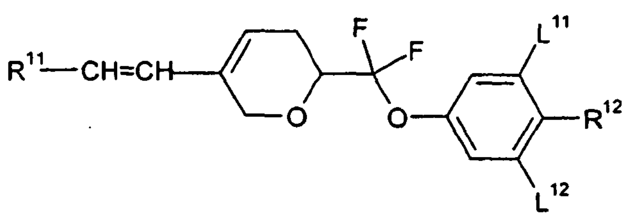

I-DCBIIIa

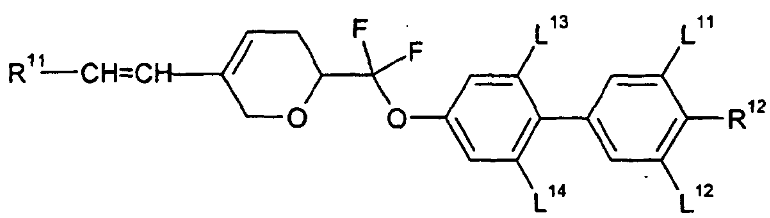

I-DCBIIIb

wobei $R^{11}$ und $R^{12}$ die für Formel I angegebene Bedeutung besitzen und $L^{11}$, $L^{12}$, $L^{13}$ und $L^{14}$ jeweils unabhängig voneinander H oder F bedeuten. $R^{11}$ ist vorzugsweise ein geradkettiger Alkenyl- oder insbesondere Alkanylrest mit 1 bis 7 Kohlenstoffatomen, und $R^{12}$ ist vorzugsweise -CN, Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen.

Beispielhafte Verbindungen der Formeln I-DCBIa, I-DCBIb, I-DCBIc, I-DCBId, I-DCBIIa, I-DCBIIb und I-DCBIIIa sind solche, die die in Tabelle 5 unten für die Reste $R^{11}$, $R^{12}$, $L^{11}$ und $L^{12}$ angegebenen Bedeutungen aufweisen:

**Tabelle 5**

| I-DABIa/I-DABIIa/I-DABIIIa/I-DBBIa/I-DBBIb/I-DBBIc/ I-DBBId/I-DBBIIa/I-DBBIIIa/I-DCBIa/I-DCBIb/I-DCBIc/ I-DCBId/I-DCBIIa/I-DCBIIb/I-DCBIIIa-Nr. | $L^{11}$ | $L^{12}$ | $R^{12}$ | $R^{11}$ |
|---|---|---|---|---|
| -1 | H | H | F | $CH_3$ |
| -2 | H | H | $CF_3$ | $CH_3$ |
| -3 | H | H | $OCF_3$ | $CH_3$ |
| -4 | H | H | $OCHF_2$ | $CH_3$ |
| -5 | H | H | CN | $CH_3$ |
| -6 | F | H | F | $CH_3$ |
| -7 | F | H | $CF_3$ | $CH_3$ |
| -8 | F | H | $OCF_3$ | $CH_3$ |
| -9 | F | H | CN | $CH_3$ |
| -10 | F | H | $OCHF_2$ | $CH_3$ |
| -11 | F | F | F | $CH_3$ |
| -12 | F | F | $CF_3$ | $CH_3$ |
| -13 | F | F | $OCF_3$ | $CH_3$ |
| -14 | F | F | $OCHF_2$ | $CH_3$ |
| -15 | F | F | CN | $CH_3$ |
| -16 | H | H | F | $C_2H_5$ |
| -17 | H | H | $CF_3$ | $C_2H_5$ |
| -18 | H | H | $OCF_3$ | $C_2H_5$ |
| -19 | H | H | $OCHF_2$ | $C_2H_5$ |
| -20 | H | H | CN | $C_2H_5$ |
| -21 | F | H | F | $C_2H_5$ |
| -22 | F | H | $CF_3$ | $C_2H_5$ |
| -23 | F | H | $OCF_3$ | $C_2H_5$ |
| -24 | F | H | $OCHF_2$ | $C_2H_5$ |
| -25 | F | H | CN | $C_2H_5$ |
| -26 | F | F | F | $C_2H_5$ |
| -27 | F | F | $CF_3$ | $C_2H_5$ |
| -28 | F | F | $OCF_3$ | $C_2H_5$ |
| -29 | F | F | $OCHF_2$ | $C_2H_5$ |
| -30 | F | F | CN | $C_2H_5$ |
| -31 | H | H | F | $n-C_3H_7$ |
| -32 | H | H | $CF_3$ | $n-C_3H_7$ |
| -33 | H | H | $OCF_3$ | $n-C_3H_7$ |
| -34 | H | H | $OCHF_2$ | $n-C_3H_7$ |
| -35 | H | H | CN | $n-C_3H_7$ |
| -36 | F | H | F | $n-C_3H_7$ |
| -37 | F | H | $CF_3$ | $n-C_3H_7$ |
| -38 | F | H | $OCF_3$ | $n-C_3H_7$ |
| -39 | F | H | $OCHF_2$ | $n-C_3H_7$ |
| -40 | F | H | CN | $n-C_3H_7$ |
| -41 | F | F | F | $n-C_3H_7$ |
| -42 | F | F | $CF_3$ | $n-C_3H_7$ |
| -43 | F | F | $OCF_3$ | $n-C_3H_7$ |
| -44 | F | F | $OCHF_2$ | $n-C_3H_7$ |
| -45 | F | F | CN | $n-C_3H_7$ |
| -46 | H | H | F | $n-C_4H_9$ |
| -47 | H | H | $CF_3$ | $n-C_4H_9$ |
| -48 | H | H | $OCF_3$ | $n-C_4H_9$ |

(fortgesetzt)

| I-DABIa/I-DABIIa/I-DABIIIa/I-DBBIa/I-DBBIb/I-DBBIc/ I-DBBId/I-DBBIIa/I-DBBIIIa/I-DCBIa/I-DCBIb/I-DCBIc/ I-DCBId/I-DCBIIa/I-DCBIIb/I-DCBIIIa-Nr. | $L^{11}$ | $L^{12}$ | $R^{12}$ | $R^{11}$ |
|---|---|---|---|---|
| -49 | H | H | $OCHF_2$ | $n-C_4H_9$ |
| -50 | H | H | CN | $n-C_4H_9$ |
| -51 | F | H | F | $n-C_4H_9$ |
| -52 | F | H | $CF_3$ | $n-C_4H_9$ |
| -53 | F | H | $OCF_3$ | $n-C_4H_9$ |
| -54 | F | H | $OCHF_2$ | $n-C_4H_9$ |
| -55 | F | H | CN | $n-C_4H_9$ |
| -56 | F | F | F | $n-C_4H_9$ |
| -57 | F | F | $CF_3$ | $n-C_4H_9$ |
| -58 | F | F | $OCF_3$ | $n-C_4H_9$ |
| -59 | F | F | $OCHF_2$ | $n-C_4H_9$ |
| -60 | F | F | CN | $n-C_4H_9$ |
| -61 | H | H | F | $n-C_5H_{11}$ |
| -62 | H | H | $CF_3$ | $n-C_5H_{11}$ |
| -63 | H | H | $OCF_3$ | $n-C_5H_{11}$ |
| -64 | H | H | $OCHF_2$ | $n-C_5H_{11}$ |
| -65 | H | H | CN | $n-C_5H_{11}$ |
| -66 | F | H | F | $n-C_5H_{11}$ |
| -67 | F | H | $CF_3$ | $n-C_5H_{11}$ |
| -68 | F | H | $OCF_3$ | $n-C_5H_{11}$ |
| -69 | F | H | $OCHF_2$ | $n-C_5H_{11}$ |
| -70 | F | H | CN | $n-C_5H_{11}$ |
| -71 | F | F | F | $n-C_5H_{11}$ |
| -72 | F | F | $CF_3$ | $n-C_5H_{11}$ |
| -73 | F | F | $OCF_3$ | $n-C_5H_{11}$ |
| -74 | F | F | $OCHF_2$ | $n-C_5H_{11}$ |
| -75 | F | F | CN | $n-C_5H_{11}$ |

Beispielhafte Verbindungen der Formeln I-DCBIe, I-DCBIf, I-DCBIg, I-DCBIIc, und I-DCBIIIb sind solche, die die in Tabelle 6 unten für die Reste $R^{11}$, $R^{12}$, $L^{11}$, $L^{12}$, $L^{13}$ und $L^{14}$ angegebenen Bedeutungen aufweisen:

**Tabelle 6**

| I-DBBIe/I-DBBIf/I-DBBIg/I-DBBIIb/I-DBBIIIb/ I-DCBUIe/I-DCBIf/I-DCBIg/I-DCBIIc/I-DCBIIIb-Nr. | $L^{11}$ | $L^{12}$ | $L^{13}$ | $L^{14}$ | $R^{12}$ | $R^{11}$ |
|---|---|---|---|---|---|---|
| -1 | H | H | H | H | F | $CH_3$ |
| -2 | H | H | H | H | $CF_3$ | $CH_3$ |
| -3 | H | H | H | H | $OCF_3$ | $CH_3$ |
| -4 | H | H | H | H | $OCHF_2$ | $CH_3$ |
| -5 | H | H | H | H | CN | $CH_3$ |
| -6 | F | H | H | H | F | $CH_3$ |
| -7 | F | H | H | H | $CF_3$ | $CH_3$ |
| -8 | F | H | H | H | $OCF_3$ | $CH_3$ |
| -9 | F | H | H | H | $OCHF_2$ | $CH_3$ |
| -10 | F | H | H | H | CN | $CH_3$ |
| -11 | F | F | H | H | F | $CH_3$ |
| -12 | F | F | H | H | $CF_3$ | $CH_3$ |
| -13 | F | F | H | H | $OCF_3$ | $CH_3$ |
| -14 | F | F | H | H | $OCHF_2$ | $CH_3$ |

(fortgesetzt)

| **I-DBBIe/I-DBBIf/I-DBBIg/I-DBBIIb/I-DBBIIIb/ I-DCBUIe/I-DCBIf/I-DCBIg/I-DCBIIc/I-DCBIIIb-Nr.** | **$L^{11}$** | **$L^{12}$** | **$L^{13}$** | **$L^{14}$** | **$R^{12}$** | **$R^{11}$** |
|---|---|---|---|---|---|---|
| -15 | F | F | H | H | CN | $CH_3$ |
| -16 | H | H | H | H | F | $C_2H_5$ |
| -17 | H | H | H | H | $CF_3$ | $C_2H_5$ |
| -18 | H | H | H | H | $OCF_3$ | $C_2H_5$ |
| -19 | H | H | H | H | $OCHF_2$ | $C_2H_5$ |
| -20 | H | H | H | H | CN | $C_2H_5$ |
| -21 | F | H | H | H | F | $C_2H_5$ |
| -22 | F | H | H | H | $CF_3$ | $C_2H_5$ |
| -23 | F | H | H | H | $OCF_3$ | $C_2H_5$ |
| -24 | F | H | H | H | $OCHF_2$ | $C_2H_5$ |
| -25 | F | H | H | H | CN | $C_2H_5$ |
| -26 | F | F | H | H | F | $C_2H_5$ |
| -27 | F | F | H | H | $CF_3$ | $C_2H_5$ |
| -28 | F | F | H | H | $OCF_3$ | $C_2H_5$ |
| -29 | F | F | H | H | $OCHF_2$ | $C_2H_5$ |
| -30 | F | F | H | H | CN | $C_2H_5$ |
| -31 | H | H | H | H | F | $n-C_3H_7$ |
| -32 | H | H | H | H | $CF_3$ | $n-C_3H_7$ |
| -33 | H | H | H | H | $OCF_3$ | $n-C_3H_7$ |
| -34 | H | H | H | H | $OCHF_2$ | $n-C_3H_7$ |
| -35 | H | H | H | H | CN | $n-C_3H_7$ |
| -36 | F | H | H | H | F | $n-C_3H_7$ |
| -37 | F | H | H | H | $CF_3$ | $n-C_3H_7$ |
| -38 | F | H | H | H | $OCF_3$ | $n-C_3H_7$ |
| -39 | F | H | H | H | $OCHF_2$ | $n-C_3H_7$ |
| -40 | F | H | H | H | CN | $n-C_3H_7$ |
| -41 | F | F | H | H | F | $n-C_3H_7$ |
| -42 | F | F | H | H | $CF_3$ | $n-C_3H_7$ |
| -43 | F | F | H | H | $OCF_3$ | $n-C_3H_7$ |
| -44 | F | F | H | H | $OCHF_2$ | $n-C_3H_7$ |
| -45 | F | F | H | H | CN | $n-C_3H_7$ |
| -46 | H | H | H | H | F | $n-C_4H_9$ |
| -47 | H | H | H | H | $CF_3$ | $n-C_4H_9$ |
| -48 | H | H | H | H | $OCF_3$ | $n-C_4H_9$ |
| -49 | H | H | H | H | $OCHF_2$ | $n-C_4H_9$ |
| -50 | H | H | H | H | CN | $n-C_4H_9$ |
| -51 | F | H | H | H | F | $n-C_4H_9$ |
| -52 | F | H | H | H | $CF_3$ | $n-C_4H_9$ |
| -53 | F | H | H | H | $OCF_3$ | $n-C_4H_9$ |
| -54 | F | H | H | H | $OCHF_2$ | $n-C_4H_9$ |
| -55 | F | H | H | H | CN | $n-C_4H_9$ |
| -56 | F | F | H | H | F | $n-C_4H_9$ |
| -57 | F | F | H | H | $CF_3$ | $n-C_4H_9$ |
| -58 | F | F | H | H | $OCF_3$ | $n-C_4H_9$ |
| -59 | F | F | H | H | $OCHF_2$ | $n-C_4H_9$ |
| -60 | F | F | H | H | CN | $n-C_4H_9$ |
| -61 | H | H | H | H | F | $n-C_5H_{11}$ |
| -62 | H | H | H | H | $CF_3$ | $n-C_5H_{11}$ |
| -63 | H | H | H | H | $OCF_3$ | $n-C_5H_{11}$ |

(fortgesetzt)

| I-DBBIe/I-DBBIf/I-DBBIg/I-DBBIIb/I-DBBIIIb/ I-DCBUIe/I-DCBIf/I-DCBIg/I-DCBIIc/I-DCBIIIb-Nr. | $L^{11}$ | $L^{12}$ | $L^{13}$ | $L^{14}$ | $R^{12}$ | $R^{11}$ |
|---|---|---|---|---|---|---|
| -64 | H | H | H | H | $OCHF_2$ | n-$C_5H_{11}$ |
| -65 | H | H | H | H | CN | n-$C_5H_{11}$ |
| -66 | F | H | H | H | F | n-$C_5H_{11}$ |
| -67 | F | H | H | H | $CF_3$ | n-$C_5H_{11}$ |
| -68 | F | H | H | H | $OCF_3$ | n-$C_5H_{11}$ |
| -69 | F | H | H | H | $OCHF_2$ | n-$C_5H_{11}$ |
| -70 | F | H | H | H | CN | n-$C_5H_{11}$ |
| -71 | F | F | H | H | F | n-$C_5H_{11}$ |
| -72 | F | F | H | H | $CF_3$ | n-$C_5H_{11}$ |
| -73 | F | F | H | H | $OCF_3$ | n-$C_5H_{11}$ |
| -74 | F | F | H | H | $OCHF_2$ | n-$C_5H_{11}$ |
| -75 | F | F | H | H | CN | n-$C_5H_{11}$ |
| -76 | F | F | F | H | F | $CH_3$ |
| -77 | F | F | F | H | $CF_3$ | $CH_3$ |
| -78 | F | F | F | H | $OCF_3$ | $CH_3$ |
| -79 | F | F | F | H | $OCHF_2$ | $CH_3$ |
| -80 | F | F | F | H | CN | $CH_3$ |
| -81 | F | F | F | H | F | n-$C_3H_7$ |
| -82 | F | F | F | H | $CF_3$ | n-$C_3H_7$ |
| -83 | F | F | F | H | $OCF_3$ | n-$C_3H_7$ |
| -84 | F | F | F | H | $OCHF_2$ | n-$C_3H_7$ |
| -85 | F | F | F | H | CN | n-$C_3H_7$ |
| -86 | F | F | F | H | F | n-$C_5H_{11}$ |
| -87 | F | F | F | H | $CF_3$ | n-$C_5H_{11}$ |
| -88 | F | F | F | H | $OCF_3$ | n-$C_5H_{11}$ |
| -89 | F | F | F | H | $OCHF_2$ | n-$C_5H_{11}$ |
| -90 | F | F | F | H | CN | n-$C_5H_{11}$ |

Eine weitere bevorzugte Ausführungsform der Erfindung umfaßt Verbindungen der Formel I, in denen kein $A^{11}$-Ring enthalten ist (a = 0), $A^{12}$ für einen 1,4-Cyclohexylenring steht und $Z^{11}$ eine Einfachbindung darstellt (b = 1) (Formel I-E):

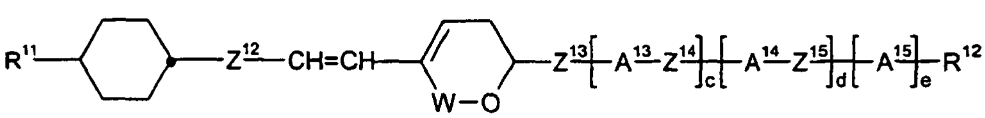

I-E

wobei c, d, e, W, $R^{11}$, $R^{12}$, $Z^{12}$, $Z^{13}$, $Z^{14}$, $Z^{15}$, $A^{13}$, $A^{14}$ und $A^{15}$ die gleiche Bedeutung wie für Formel I oben besitzen.

Bevorzugte Untergruppen von Verbindungen der Formel I-E sind Verbindungen der Formel I-EA und insbesondere der Formel I-EB:

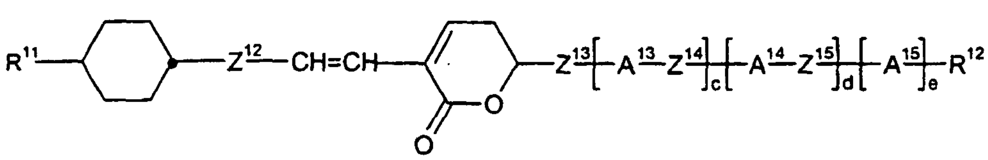

I-EA

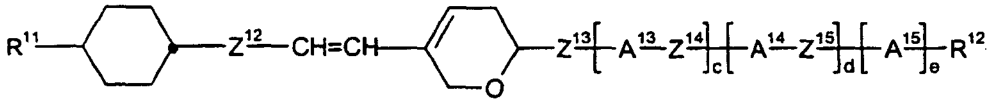

I-EB

wobei c, d, e, $R^{11}$, $R^{12}$, $Z^{12}$, $Z^{13}$, $Z^{14}$, $Z^{15}$, $A^{13}$, $A^{14}$ und $A^{15}$ die gleiche Bedeutung wie für Formel I besitzen. $R^{11}$ ist vorzugsweise ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen.

Besonders bevorzugte Verbindungen der Formel I-EB sind solche, in denen $Z^{12}$ eine Einfachbindung bedeutet (Formel I-EBI) oder eine $CH_2CH_2$-Gruppe darstellt (Formel I-EBII):

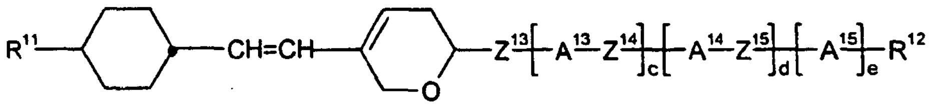

I-EBI

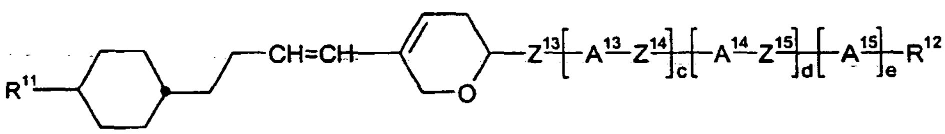

I-EBII

wobei c, d, e, $R^{11}$, $R^{12}$, $Z^{13}$, $Z^{14}$, $Z^{15}$, $A^{13}$, $A^{14}$ und $A^{15}$ die gleiche Bedeutung wie in Formel I besitzen. Beispielhafte Verbindungen der Formel I-EBI sind unter anderem solche der oben genannten Verbindungen der Formeln I-DBBIa, I-DBBIb, I-DBBIc, I-DBBId, I-DBBIe, I-DBBIf, I-DBBIg, I-DBBIIa, I-DBBIIb, I-DBBIIIa und I-DBBIIIb.

Weiter ist es bevorzugt, dass $Z^{13}$ in den erfindungsgemäßen Verbindungen der Formel I eine Einfachbindung (Formel I-F), eine Carboxyl-Gruppe -C(O)-O- (Formel I-G) oder eine Difluoroxymethylen-Gruppe $-CF_2O-$(Formel I-H) bedeutet:

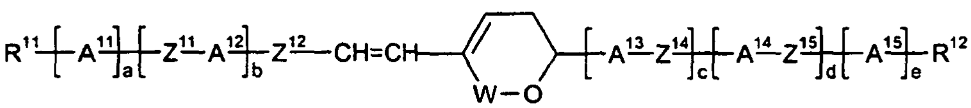

I-F

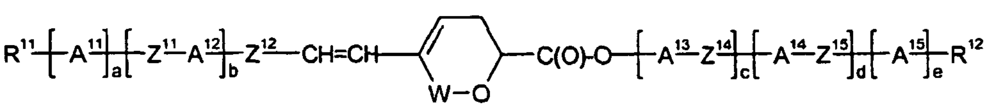

I-G

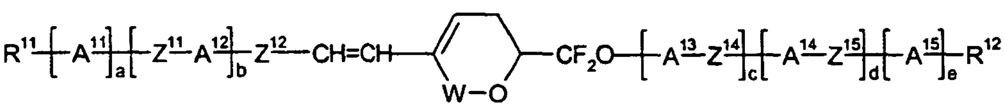

I-H

wobei a, b, c, d, e, W, $R^{11}$, $R^{12}$, $Z^{11}$, $Z^{12}$, $Z^{14}$, $Z^{15}$, $A^{11}$, $A^{12}$, $A^{13}$, $A^{14}$ und $A^{15}$ die gleiche Bedeutung wie in Formel I

oben besitzen. Bevorzugte Untergruppen werden von Verbindungen der Formeln I-FA, I-FB, I-GA, I-GB, I-HA und I-HB gebildet:

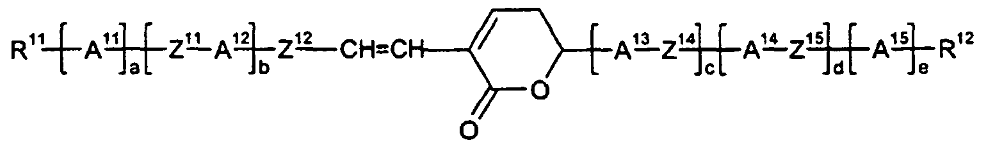

I-FA

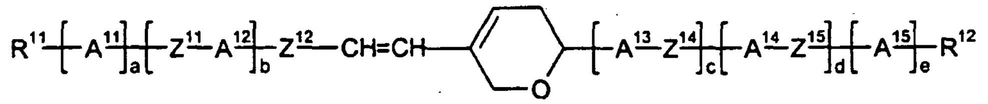

I-FB

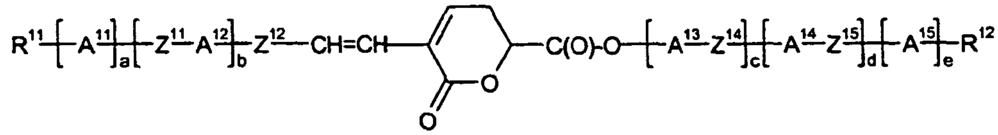

I-GA

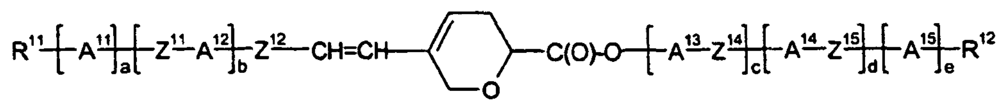

I-GB

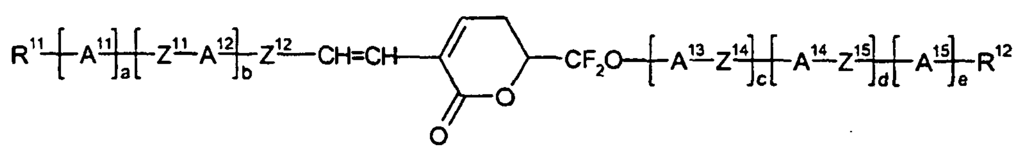

I-HA

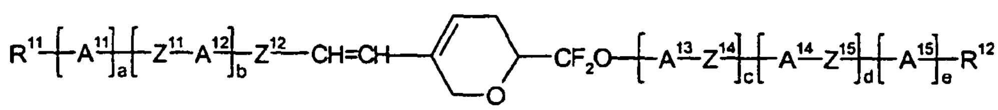

I-HB

wobei a, b, c, d, e, $R^{11}$, $R^{12}$, $Z^{11}$, $Z^{12}$, $Z^{14}$, $Z^{15}$, $A^{11}$, $A^{12}$, $A^{13}$, $A^{14}$ und $A^{15}$ die gleiche Bedeutung wie in Formel I oben besitzen. Bei entsprechender Auswahl der Bedeutung von a, b, c, d, e, $R^{11}$, $R^{12}$, $Z^{11}$, $Z^{12}$, $Z^{14}$, $Z^{15}$, $A^{11}$, $A^{12}$, $A^{13}$, $A^{14}$ und $A^{15}$ stellen

die Verbindungen der Formeln I-CBI, I-DABI, I-DBBI, I-DCBI und Verbindungen der Formel I-EB mit $Z^{13}$ = Einfachbindung einige der bevorzugten Verbindungen der Formel I-FB dar;

die Verbindungen der Formeln I-CBII, I-DABII, I-DBBII, I-DCBII und Verbindungen der Formel I-EB mit $Z^{13}$ = -CO-O- einige der bevorzugten Verbindungen der Formel I-GB dar;

die Verbindungen der Formeln 1-CBIII, I-DABIII, I-DBBIII, I-DCBIII und Verbindungen der Formel I-EB mit $Z^{13}$ = $CF_2O$ einige der bevorzugten Verbindungen der Formel I-HB dar.

Zu den bevorzugten Verbindungen der Erfindung zählen ferner Pyranderivate der Formel I, bei denen keine der Gruppen $A^{13}$-$Z^{14}$, $A^{14}$-$Z^{15}$

und $A^{15}$ vorhanden ist (d.h. c, d und e zugleich null sind), $Z^{13}$ ein Carboxylrest (C(O)-O) ist und $R^{12}$ entweder H oder

Aralkyl oder Alkanyl
oder Alkenyl bedeutet. Diese Carbonsäuren beziehungsweise Carbonsäureester werden durch Formel I-J veranschaulicht und bilden zugleich eine bevorzugte Gruppe von Verbindungen der Formel I-G:

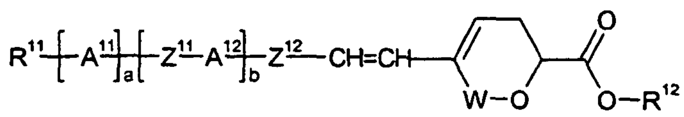

I-J

wobei a, b, W, $R^{11}$, $Z^{11}$, $Z^{12}$, $A^{11}$ und $A^{12}$ die gleiche Bedeutung wie in Formel I oben besitzen. Bevorzugte Untergruppen werden von Verbindungen der Formeln I-JA und I-JB gebildet:

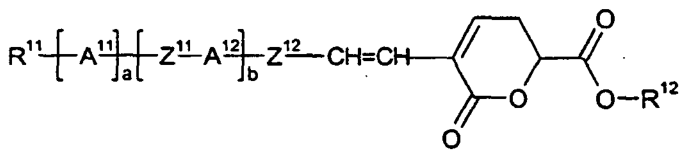

I-JA

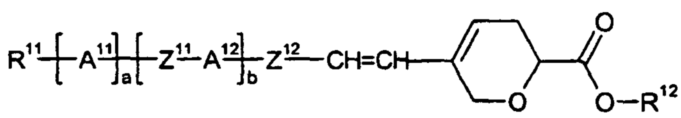

I-JB

wobei a, b, $R^{11}$, $R^{12}$, $Z^{11}$, $Z^{12}$, $A^{11}$ und $A^{12}$ die gleiche Bedeutung wie in Formel I oben besitzen.

Besonders bevorzugte Verbindungen der Formel I-JB sind solche, in denen a und b beide zugleich null sind, $Z^{12}$ für eine Einfachbindung steht und $R^{11}$ H bedeutet, d.h. Verbindungen der obigen Formel I-CBIIa, sowie Verbindungen der Formeln I-JBI, I-JBII und I-JBIII:

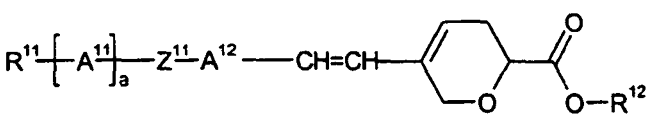

I-JBI

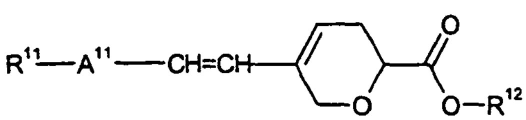

I-JBII

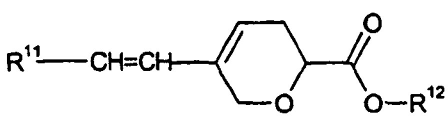

I-JBIII

wobei a, $R^{11}$, $R^{12}$, $Z^{11}$, $Z^{12}$ $A^{11}$ und $A^{12}$ die gleiche Bedeutung wie in Formel I oben besitzen, $R^{11}$ in Formel I-JBIII aber nicht H ist. Neben den Verbindungen der Formel I-CBIIa und I-JBIII sind jene der Formeln I-JBIa und I-JBIIa ganz besonders bevorzugt:

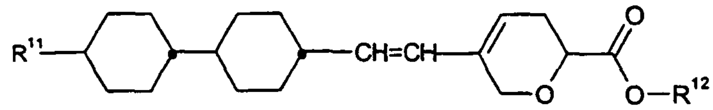

I-JBIa

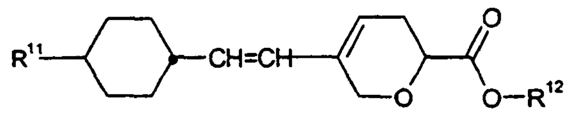

I-JBII

wobei $R^{11}$ und $R^{12}$ die gleiche Bedeutung wie in Formel I oben besitzen. Beispielhafte Verbindungen der Formel I-JBIa, I-JBIIa und I-JBIII sind solche, die die in Tabelle 7 unten für die Reste $R^{11}$ und $R^{12}$ angegebenen Bedeutungen aufweisen.

**Tabelle 7**

| I-JBIa/I-JBIIa/I-JBIII-Nr. | $R^{12}$ | $R^{11}$ |
|---|---|---|
| -1 | H | $CH_3$ |
| -2 | H | $C_2H_5$ |
| -3 | H | n-$C_3H_7$ |
| -4 | $CH_3$ | $CH_3$ |
| -5 | $CH_3$ | $C_2H_5$ |
| -6 | $CH_3$ | n-$C_3H_7$ |
| -7 | $C_2H_5$ | $CH_3$ |
| -8 | $C_2H_5$ | $C_2H_5$ |
| -9 | $C_2H_5$ | n-$C_3H_7$ |
| -10 | n-$C_3H_7$ | $CH_3$ |
| -11 | n-$C_3H_7$ | $C_2H_5$ |
| -12 | n-$C_3H_7$ | n-$C_3H_7$ |
| -13 | i-$C_3H_7$ | $CH_3$ |
| -14 | i-$C_3H_7$ | $C_2H_5$ |
| -15 | i-$C_3H_7$ | n-$C_3H_7$ |
| -16 | n-$C_4H_9$ | $CH_3$ |
| -17 | n-$C_4H_9$ | $C_2H_5$ |
| -18 | n-$C_4H_9$ | n-$C_3H_7$ |
| -19 | t-$C_4H_9$ | $CH_3$ |
| -20 | t-$C_4H_9$ | $C_2H_5$ |
| -21 | t-$C_4H_9$ | n-$C_3H_7$ |
| -22 | $CH_2$-Phenyl | $CH_3$ |
| -23 | $CH_2$-Phenyl | $C_2H_5$ |
| -24 | $CH_2$-Phenyl | n-$C_3H_7$ |
| -25 | $CH_2CH_2$-Phenyl | $CH_3$ |
| -26 | $CH_2CH_2$-Phenyl | $C_2H_5$ |
| -27 | $CH_2CH_2$-Phenyl | n-$C_3H_7$ |
| -28 | $CH_2$-CH=$CH_2$ | $CH_3$ |
| -29 | $CH_2$-CH=$CH_2$ | $C_2H_5$ |
| -30 | $CH_2$-CH=$CH_2$ | n-$C_3H_7$ |

Eine weitere bevorzugte Ausführungsform der Erfindung betrifft Verbindungen der Formel I, die eine Gruppe $A^{15}$ aufweisen (e = 1), die für einen gegebenenfalls in 3- und/oder 5-Position Fluor-substituierten 1,4-Phenylenring steht (Formel I-K):

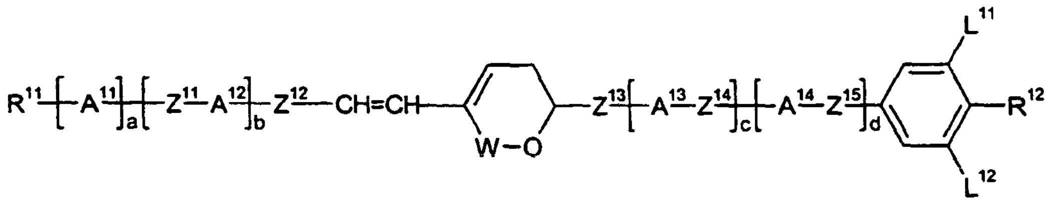

I-K

wobei a, b, c, d, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$, $L^{11}$ und $L^{12}$ die gleiche Bedeutung wie für Formel I oben besitzen.

Bevorzugte Untergruppen von Verbindungen der Formel I-K werden von Verbindungen der Formel I-KA und insbesondere der Formel I-KB gebildet:

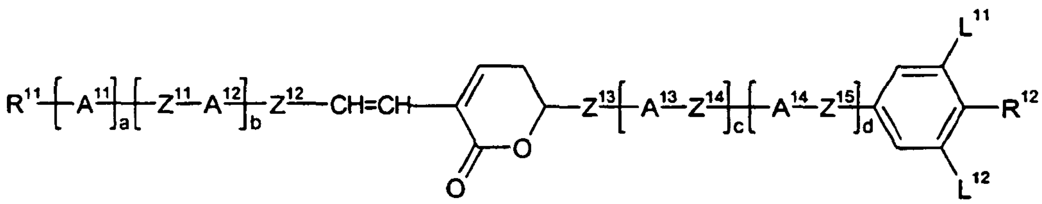

I-KA

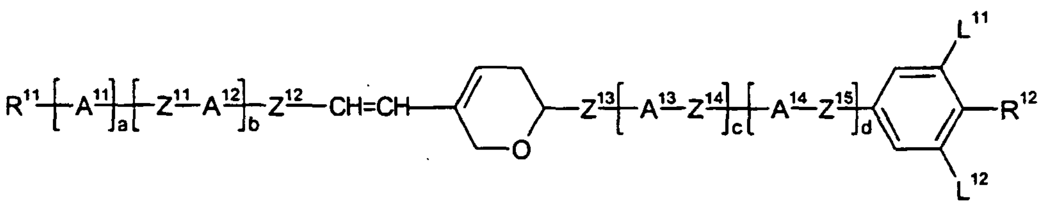

I-KB

wobei a, b, c, d, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$, $L^{11}$ und $L^{12}$ die gleiche Bedeutung wie für Formel I besitzen.

Besonders bevorzugte Verbindungen der Formel I-KB sind solche, in denen c und d beide zugleich null sind und $Z^{13}$ für eine Einfachbindung, -CO-O- oder $-CF_2O-$ steht (Formeln I-KBA, I-KBB beziehungsweise I-KBC):

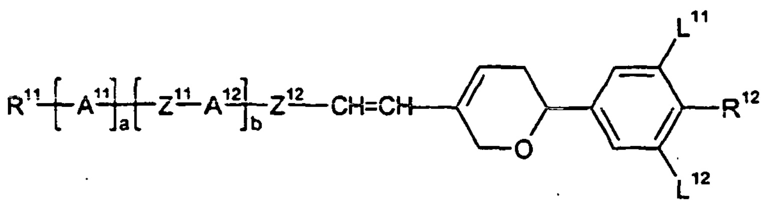

I-KBA

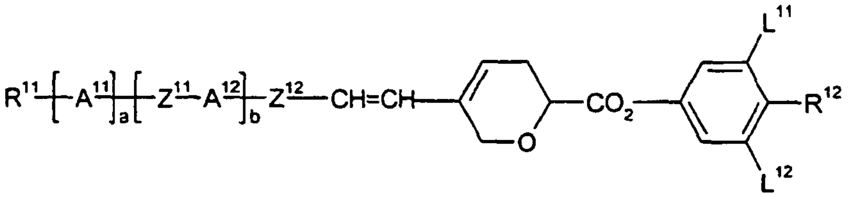

I-KBB

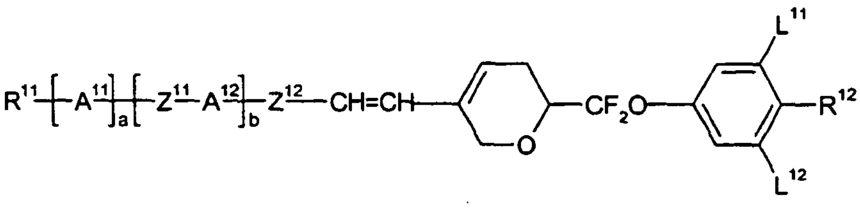

I-KBC

wobei a, b, $R^{11}$, $R^{12}$, $Z^{11}$, $Z^{12}$, $A^{11}$, $A^{12}$, $L^{11}$ und $L^{12}$ die gleiche Bedeutung wie für Formel I besitzen. $R^{11}$ ist vorzugsweise H, ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen.

Unter den Verbindungen der Formel I-KBA sind solche der Formeln I-KBAI, I-KBAII und I-KBAIII ganz besonders bevorzugt:

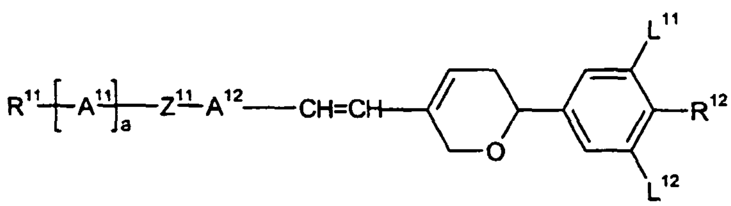

I-KBAI

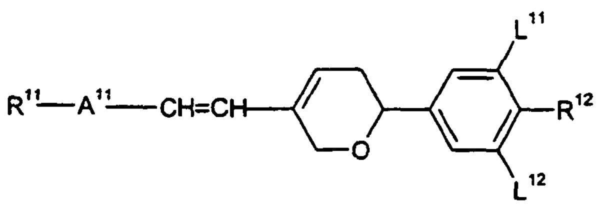

I-KBAII

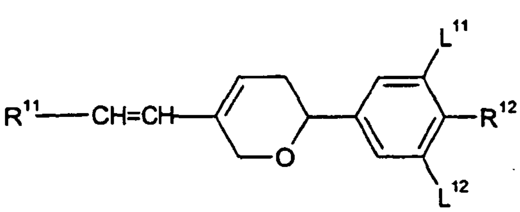

I-KBAIII

wobei a, $R^{11}$, $R^{12}$, $Z^{11}$, $A^{11}$, $A^{12}$, $L^{11}$ und $L^{12}$ die gleiche Bedeutung wie für Formel I besitzen. Beispielhafte Verbindungen der Formeln I-KBAI, I-KBAII und I-KBAIII sind unter anderem die oben genannten Verbindungen der Formeln I-DABIa, I-DBBIa beziehungsweise I-DCBIa.

Unter den Verbindungen der Formel I-KBB sind solche der Formeln I-KBBI, I-KBBII und I-KBBIII ganz besonders bevorzugt:

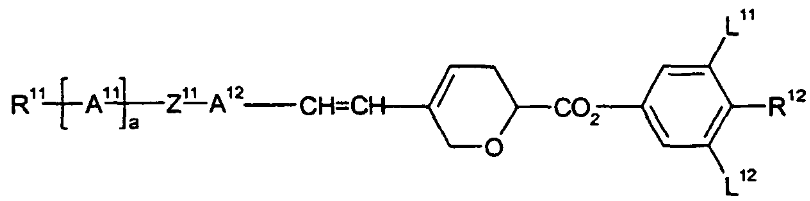

I-KBBI

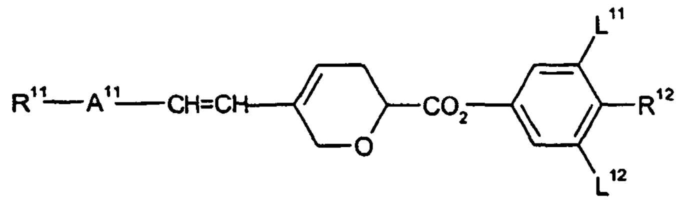

I-KBBII

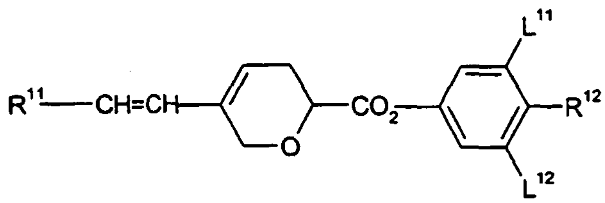

I-KBBIII

wobei a, $R^{11}$, $R^{12}$, $Z^{11}$, $A^{11}$, $A^{12}$, $L^{11}$ und $L^{12}$ die gleiche Bedeutung wie für Formel besitzen. Beispielhafte Verbindungen der Formeln I-KBBI, I-KBBII und I-KBBIII sind unter anderem die oben genannten Verbindungen der Formeln I-DABIIa, I-DBBIIa beziehungsweise I-DCBIIa.

Unter den Verbindungen der Formel I-KBC sind solche der Formeln I-KBCI, I-KBCII und I-KBCIII ganz besonders bevorzugt:

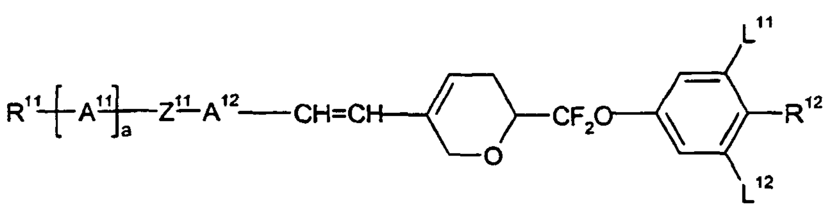

I-KBCI

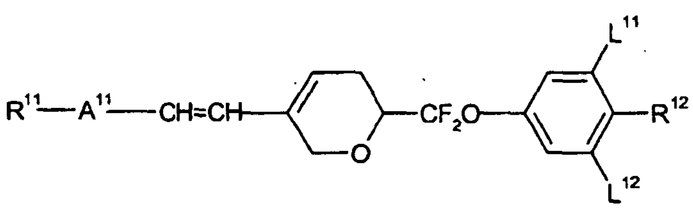

I-KBCII

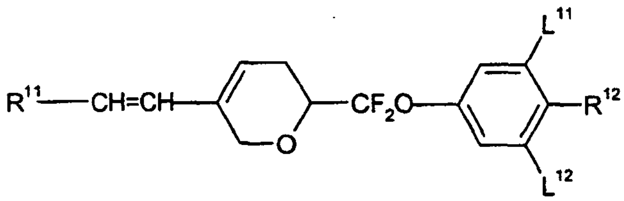

I-KBCIII

wobei a, $R^{11}$, $R^{12}$, $Z^{11}$, $A^{11}$, $A^{12}$, $L^{11}$ und $L^{12}$ die gleiche Bedeutung wie für Formel I besitzen. Beispielhafte Verbindungen der Formeln I-KBCI, I-KBCII und I-KBCIII sind unter anderem die oben genannten Verbindungen der Formeln I-DABIIIa, I-DBBIIIa beziehungsweise I-DCBIIIa.

Ferner sind solche Verbindungen der Formel I bevorzugt, die neben einem Ring $A^{15}$ eine Gruppe $A^{13}$-$Z^{14}$ und/oder $A^{14}$-$Z^{15}$ aufweisen (d.h. für die e = 1 und c und/oder d = 1), wobei $Z^{14}$ und $Z^{15}$ unabhängig voneinander für eine Einfachbindung oder eine Difluoroxymethylen-Brücke stehen:

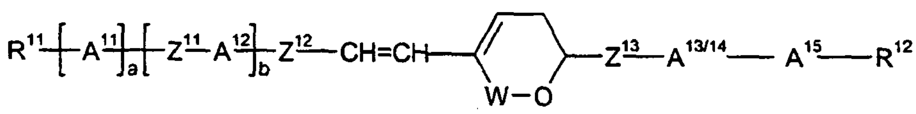

I-L

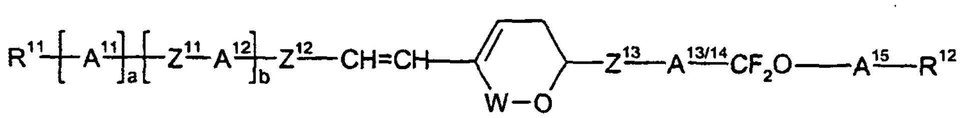

I-M

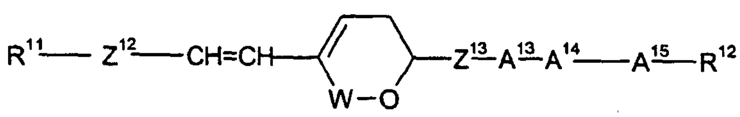

I-N

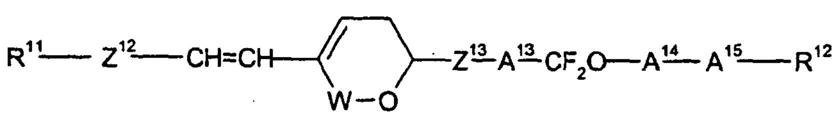

I-O

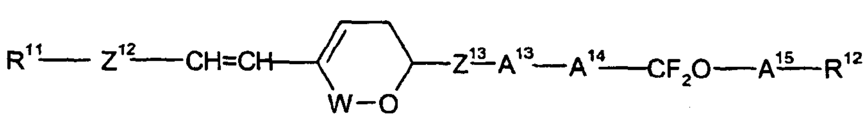

I-P

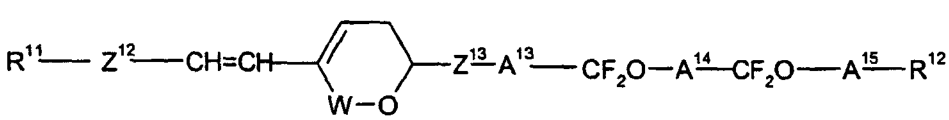

I-Q

wobei a, b, W, $R^{11}$, $R^{12}$, $Z^{11}$, $Z^{12}$, $Z^{13}$, $A^{11}$, $A^{12}$, $A^{13}$, $A^{14}$ und $A^{15}$ die gleiche Bedeutung wie in Formel I oben besitzen und $A^{13/14}$ bedeutet, dass entweder ein Ring $A^{13}$ oder ein Ring $A^{14}$ vorhanden ist. Es ist ferner bevorzugt, dass die Summe von a, b, c, d und e nicht größer als 3 ist, so dass a und b beide null sind, wenn - wie für die Verbindungen der Formeln I-N, I-O, I-P und I-Q - c = d = e = 1 gilt.

Bevorzugte Untergruppen der Formel I-L werden dabei von Verbindungen mit W = -C(=O)- (Formel I-LA) und insbesondere von Verbindungen mit W = $-CH_2-$ (Formel I-LB) gebildet.

Besonders bevorzugte Verbindungen der Formel I-LB sind Verbindungen der Formeln I-LBA, I-LBB und I-LBC:

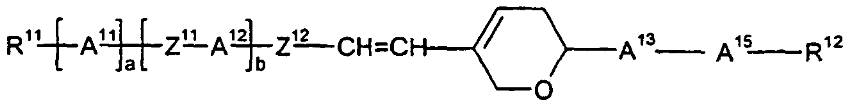

I-LBA

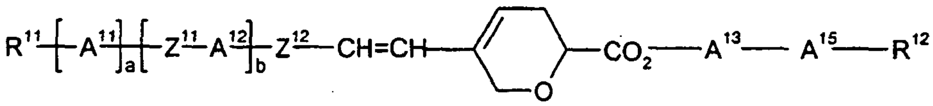

I-LBB

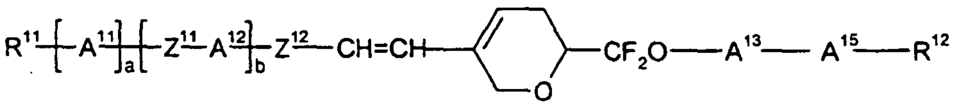

I-LBC

wobei a, b, $R^{11}$, $R^{12}$, $Z^{11}$, $Z^{12}$, $A^{11}$, $A^{12}$, $A^{13}$ und $A^{15}$ die gleiche Bedeutung wie in Formel I oben besitzen. $R^{11}$ ist vorzugsweise H, ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. Unter diesen Verbindungen sind jene der Formeln I-LBAI, I-LBAII, I-LBBI, I-LBBII, I-LBCI und I-LBCII ganz besonders bevorzugt:

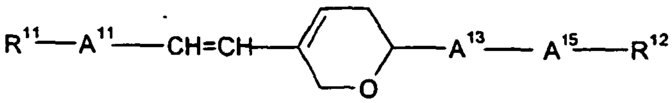

I-LBAI

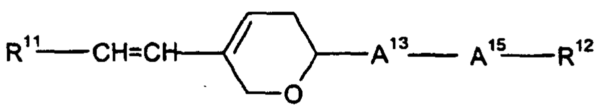

I-LBAII

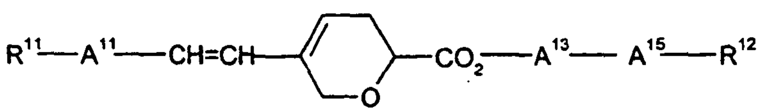

I-LBBI

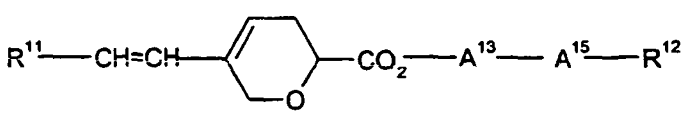

I-LBBII

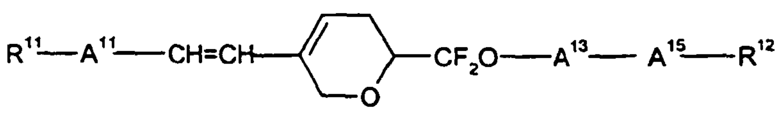

I-LBCI

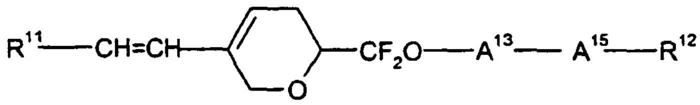

I-LBCII

wobei $R^{11}$, $R^{12}$, $A^{11}$, $A^{13}$ und $A^{15}$ die oben für Formel I angegebene Bedeutung besitzen. Beispielhafte Verbindungen der Formel I-LBAI sind unter anderem die oben genannten Verbindungen der Formeln I-DBBIb und I-DBBIe;
der Formel I-LBAII sind unter anderem die oben genannten Verbindungen der Formeln I-CBIb, I-CBIc, I-DCBIb, I-DCBIe;
der Formel I-LBBI sind unter anderem die oben genannten Verbindungen der Formel I-DBBIIb;
der Formel I-LBBII sind unter anderem die oben genannten Verbindungen der Formeln I-CBIIc, I-CBIId, I-DCBIIb, I-DCBIIc;
der Formel I-LBCI sind unter anderem die oben genannten Verbindungen der Formel I-DBBIIIb;
der Formel I-LBCII sind unter anderem die oben genannten Verbindungen der Formeln I-CBIIIb, I-DCBIIIb.

Bevorzugte Untergruppen von Verbindungen der Formel I-M werden von Verbindungen mit W = -C(=O)- (Formel I-MA) und insbesondere von Verbindungen mit W = $-CH_2-$ (Formel I-MB) gebildet.

Besonders bevorzugte Verbindungen der Formel I-MB sind Verbindungen der Formeln I-MBA und I-MBB:

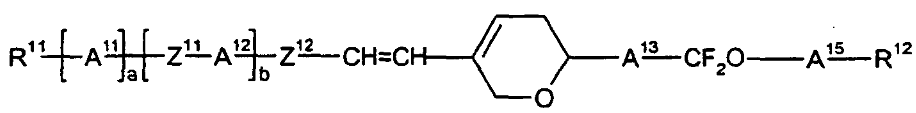

I-MBA

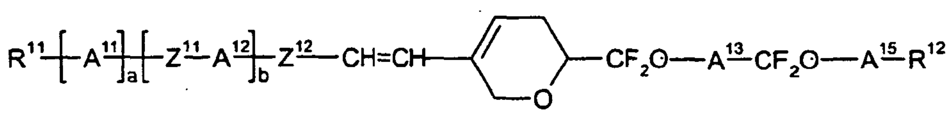

I-MBB

wobei a, b, $R^{11}$, $R^{12}$, $Z^{11}$, $Z^{12}$, $A^{11}$, $A^{12}$, $A^{13}$ und $A^{15}$ die gleiche Bedeutung wie in Formel I oben besitzen. $R^{11}$ ist vorzugsweise Wasserstoff, ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. $A^{15}$ in I-MBA und $A^{13}$ und $A^{15}$ in I-MBB sind bevorzugt aromatische Ringe (Phenylen beziehungsweise Naphthylen). Unter diesen Verbindungen sind jene der Formeln I-MBAI, I-MBAII, I-MBBI und I-MBBII ganz besonders bevorzugt:

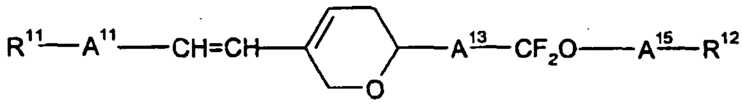

I-MBAI

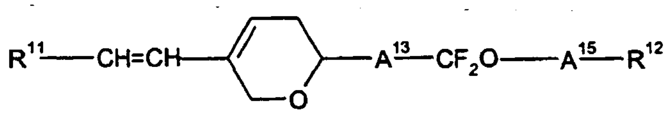

I-MBAII

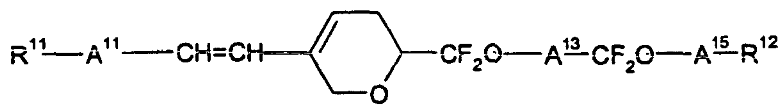

I-MBBI

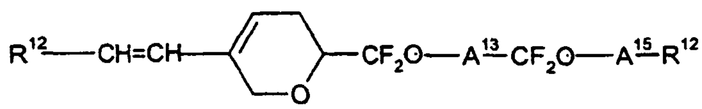

I-MBBII

wobei $R^{11}$, $R^{12}$, $A^{11}$, $A^{13}$ und $A^{15}$ die oben für Formel I angegebene Bedeutung besitzen. Beispielhafte Verbindungen der Formel 1-MBAI sind unter anderem die oben genannten Verbindungen der Formeln I-DBBId und I-DBBIg, und beispielhafte Verbindungen der Formel I-MBAII sind unter anderem die oben genannten Verbindungen der Formeln I-CBIe, I-CBIg, I-DCBId, I-DCBIg. Beispielhafte Verbindungen der Formel I-MBBI sind unter anderem Verbindungen der Formel I-MBBIa, und beispielhafte Verbindungen der Formel I-MBBIIa sind unter anderem die oben genannten Verbindungen der Formel I-CBIIIc sowie Verbindungen der Formel I-MBBIIa; die Reste $R^{11}$, $R^{12}$, $L^{11}$, $L^{12}$, $L^{13}$ und $L^{14}$ weisen die in Tabelle 8 unten angegebene Bedeutung auf.

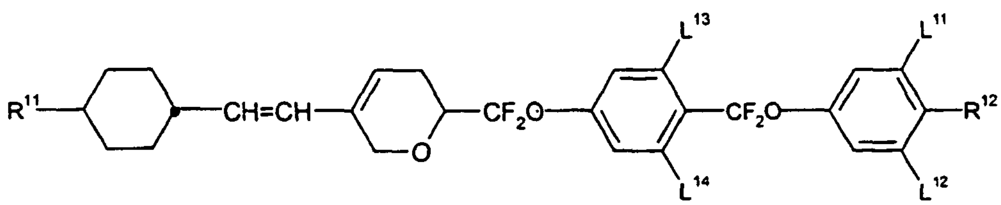

I-MBBIa

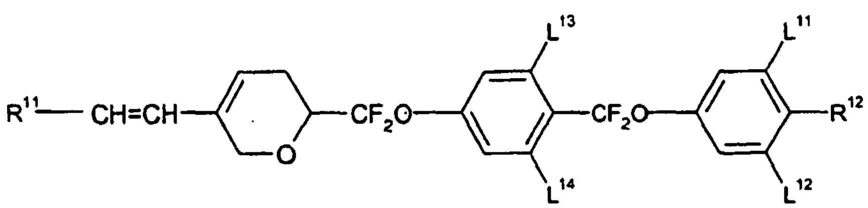

I-MBBIIa

Bevorzugte Untergruppen von Verbindungen der Formel I-N werden von Verbindungen mit W = -C(=O)- (Formel 1-NA) und insbesondere von Verbindungen mit W = $-CH_2-$ (Formel 1-NB) gebildet.

Besonders bevorzugte Verbindungen der Formel 1-NB sind Verbindungen der Formeln I-NBA, I-NBB und I-NBC:

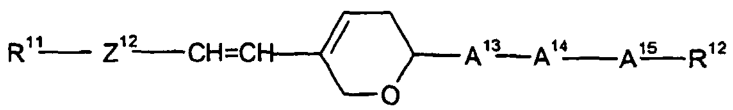

I-NBA

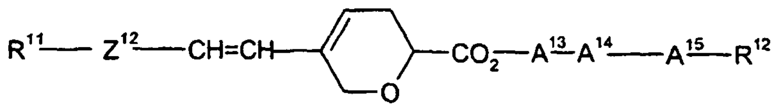

I-NBB

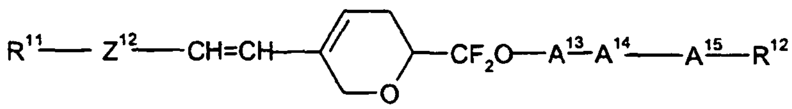

I-NBC

wobei $R^{11}$, $R^{12}$, $Z^{12}$, $A^{13}$, $A^{14}$ und $A^{15}$ die gleiche Bedeutung wie in Formel I oben besitzen. $R^{11}$ ist vorzugsweise H, ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. Unter diesen Verbindungen sind jene der Formeln I-NBAI, I-NBBI und 1-NBCI ganz besonders bevorzugt:

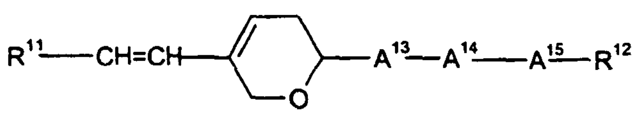

I-NBAI

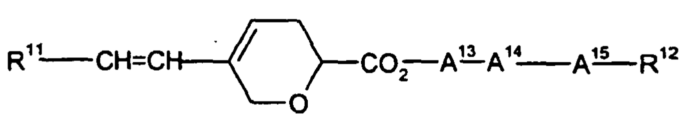

I-NBBI

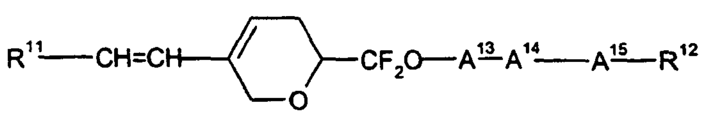

I-NBCI

wobei $R^{11}$, $R^{12}$, $A^{13}$, $A^{14}$ und $A^{15}$ die oben für Formel I angegebene Bedeutung besitzen. Beispielhafte Verbindungen der Formel I-NBAI sind unter anderem die oben genannten Verbindungen der Formel I-CBIh sowie Verbindungen der Formel I-NBAIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf. Beispielhafte Verbindungen der Formel I-NBBI sind unter anderem die oben genannten Verbindungen der Formeln 1-CBIIe und I-CBIIf sowie Verbindungen der Formeln I-NBBIa und I-NBBIb; die Reste $R^{11}$, $R^{12}$, $L^{11}$, $L^{12}$, $L^{13}$, $L^{14}$, $L^{15}$ und $L^{16}$ weisen die in Tabelle 8 beziehungsweise 9 unten angegebene Bedeutung auf. Beispielhafte Verbindungen der Formel I-NBCI sind unter anderem die oben genannten Verbindungen der Formel I-CBIIId sowie Verbindungen der Formel I-NBCIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf.

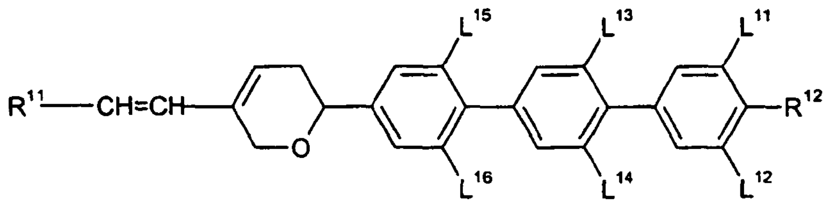

I-NBAIa

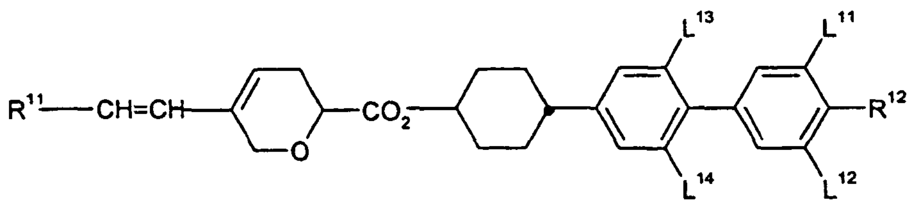

I-NBBIa

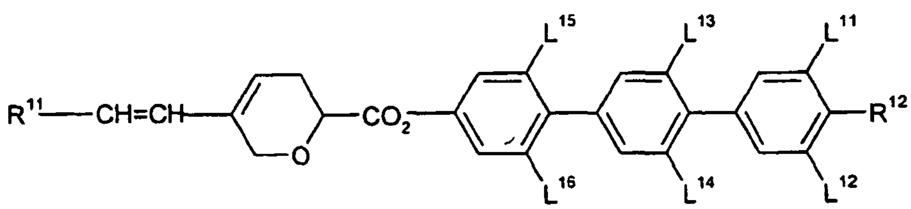

I-NBBIb

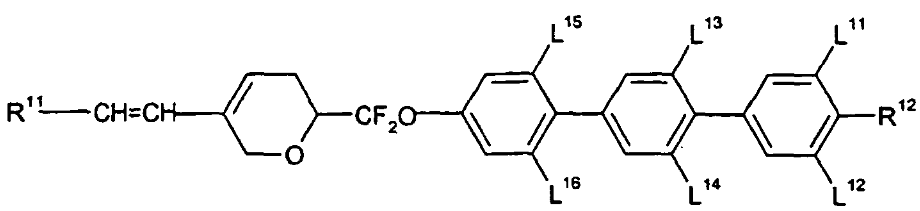

I-NBCIa

Bevorzugte Untergruppen der Formel I-O werden von Verbindungen mit W = -C(=O)- (Formel I-OA) und insbesondere von Verbindungen mit W = $-CH_2-$ (Formel I-OB) gebildet.

Besonders bevorzugte Verbindungen der Formel I-OB sind Verbindungen der Formeln I-OBA und I-OBB:

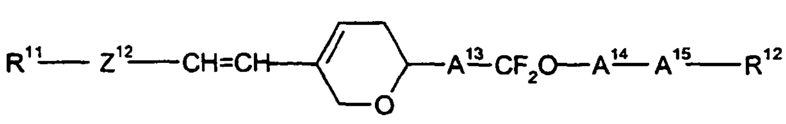

I-OBA

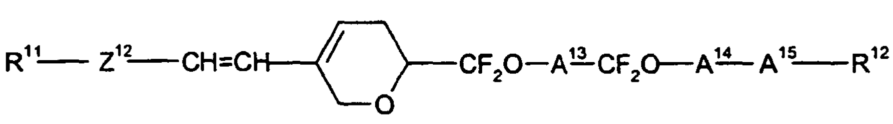

I-OBB

wobei $R^{11}$, $R^{12}$, $Z^{12}$, $A^{13}$, $A^{14}$ und $A^{15}$ die gleiche Bedeutung wie in Formel I oben besitzen. $R^{11}$ ist vorzugsweise H, ein geradkettiger Alkenyl- oder insbesondere Alkanylrest mit 1 bis 7 Kohlenstoffatomen, und $R^{12}$ ist vorzugsweise Halogen,

insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. Unter diesen Verbindungen sind jene der Formeln I-OBAI und I-OBBI ganz besonders bevorzugt:

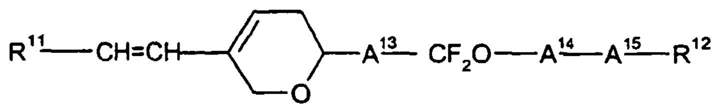

I-OBAI

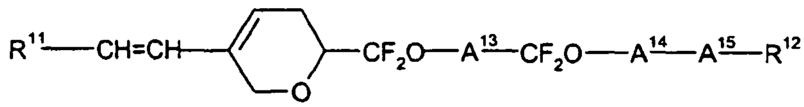

I-OBBI

wobei $R^{11}$, $R^{12}$, $A^{13}$, $A^{14}$ und $A^{15}$ die oben für Formel I angegebene Bedeutung besitzen.

Beispielhafte Verbindungen der Formel I-OBAI sind unter anderem die oben genannten Verbindungen der Formel I-CBIm sowie Verbindungen der Formel I-OBAIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf. Beispielhafte Verbindungen der Formel I-OBBI sind unter anderem die oben genannten Verbindungen der Formel I-CBIIIe sowie Verbindungen der Formel I-OBBIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf.

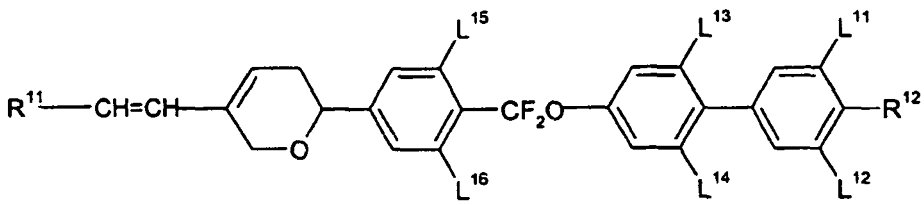

I-OBAIa

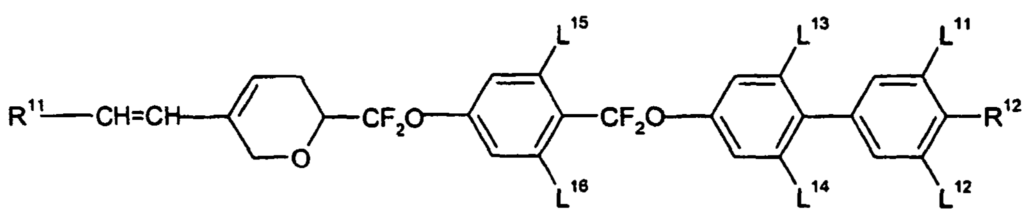

I-OBBIa

Bevorzugte Untergruppen der Formel I-P werden von Verbindungen mit W = -C(=O)- (Formel I-PA) und insbesondere von Verbindungen mit W = $-CH_2-$ (Formel I-PB) gebildet.

Besonders bevorzugte Verbindungen der Formel I-PB sind Verbindungen der Formeln I-PBA und I-PBB:

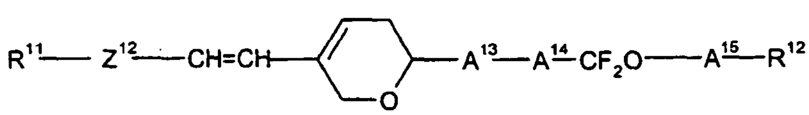

I-PBA

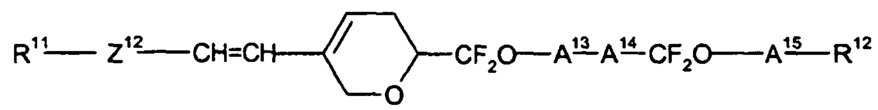

I-PBB

wobei $R^{11}$, $R^{12}$, $Z^{12}$, $A^{13}$, $A^{14}$ und $A^{15}$ die gleiche Bedeutung wie in Formel I oben besitzen. $R^{11}$ ist vorzugsweise H, ein geradkettiger Alkenyl- oder insbesondere Alkanylrest mit 1 bis 7 Kohlenstoffatomen, und $R^{12}$ ist vorzugsweise Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen. Unter diesen Verbindungen sind jene der Formeln I-PBAI und I-PBBI ganz besonders bevorzugt:

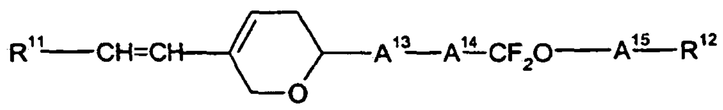

I-PBAI

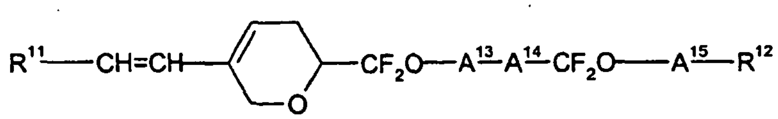

I-PBBI

wobei $R^{11}$, $R^{12}$, $A^{13}$, $A^{14}$ und $A^{15}$ die oben für Formel I angegebene Bedeutung besitzen. Beispielhafte Verbindungen der Formel I-PBAI sind unter anderem die oben genannten Verbindungen der Formeln I-CBIj und I-CBIn sowie Verbindungen der Formel I-PBAIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf. Beispielhafte Verbindungen der Formel I-PBBI sind unter anderem die oben genannten Verbindungen der Formel I-CBIIIf sowie Verbindungen der Formel I-PBBIa; die Reste $R^{11}$, $R^{12}$, $L^{11}$, $L^{12}$, $L^{13}$, $L^{14}$, $L^{15}$ und $L^{16}$ weisen die in Tabelle 9 unten angegebene Bedeutung auf.

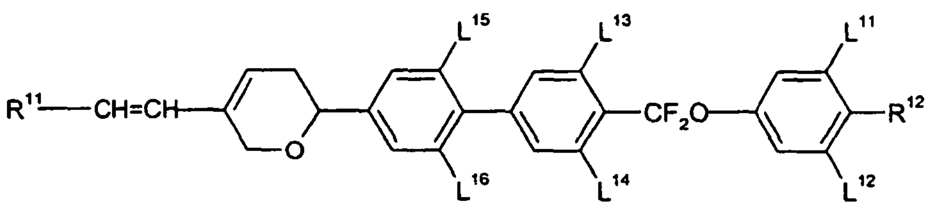

I-PBAIa

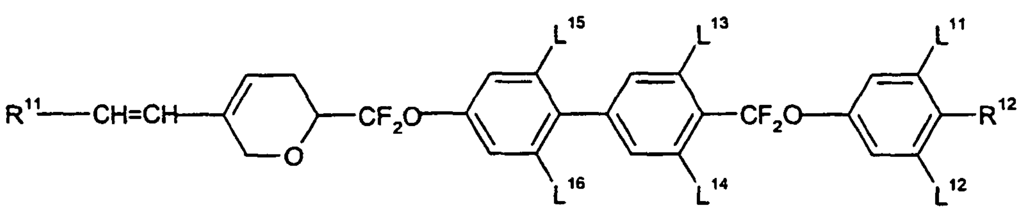

I-PBBIa

Bevorzugte Untergruppen der Formel I-Q werden von Verbindungen der Formel I-QA (mit W = -C(=O)-) und insbesondere von Verbindungen der Formel I-QB (mit W = $-CH_2-$) gebildet:

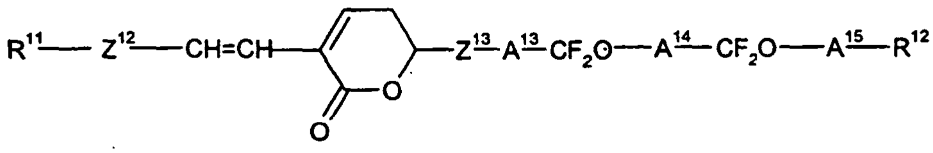

I-QA

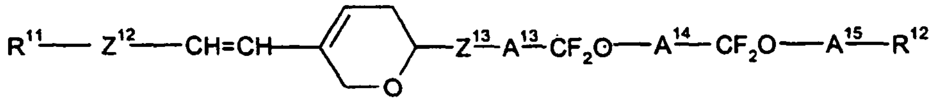

I-QB

wobei $R^{11}$, $R^{12}$, $Z^{12}$, $Z^{13}$, $A^{13}$, $A^{14}$ und $A^{15}$ die gleiche Bedeutung wie in Formel I oben besitzen.

Besonders bevorzugte Verbindungen der Formel I-QB sind Verbindungen der Formel I-QBA:

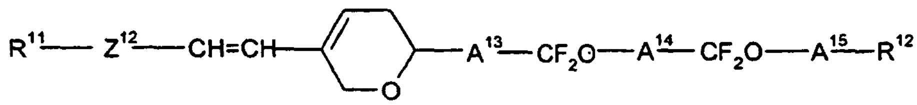

I-QBA

wobei $R^{11}$, $R^{12}$, $Z^{12}$, $A^{13}$, $A^{14}$ und $A^{15}$ die gleiche Bedeutung wie in Formel I oben besitzen. Unter diesen Verbindungen sind jene der Formeln I-QBAI ganz besonders bevorzugt:

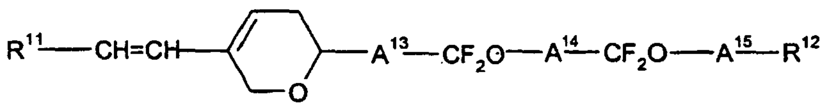

I-QBAI

wobei $R^{11}$, $R^{12}$, $A^{13}$, $A^{14}$ und $A^{15}$ die oben für Formel I angegebene Bedeutung besitzen. Beispielhafte Verbindungen der Formel I-QBAI sind unter anderem Verbindungen der Formeln I-QBAIa und I-QBAIb; die Reste $R^{11}$, $R^{12}$, $L^{11}$, $L^{12}$, $L^{13}$, $L^{14}$, $L^{15}$ und $L^{16}$ weisen die in Tabelle 8 beziehungsweise 9 unten angegebene Bedeutung auf.

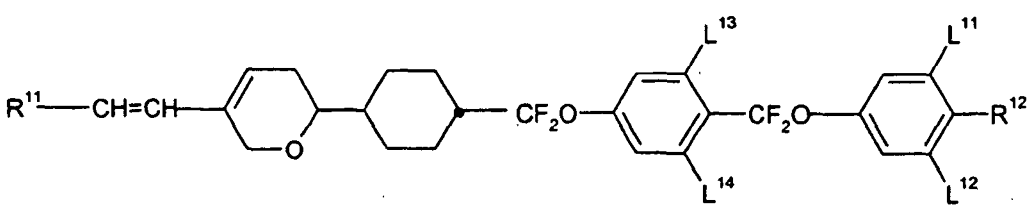

I-QBAIa

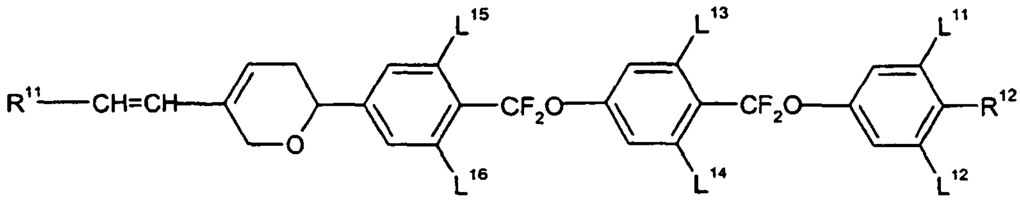

I-QBAIb

Tabelle 8

| I-MBBIa/I-MBBIIa/I-NBBIa/IQBAIa-Nr. | $L^{11}$ | $L^{12}$ | $L^{13}$ | $L^{14}$ | $R^{12}$ | $R^{11}$ |
|---|---|---|---|---|---|---|
| -1 | H | H | H | H | F | $CH_3$ |
| -2 | H | H | H | H | $CF_3$ | $CH_3$ |
| -3 | H | H | H | H | $OCF_3$ | $CH_3$ |
| -4 | H | H | H | H | $OCHF_2$ | $CH_3$ |
| -5 | F | H | H | H | F | $CH_3$ |
| -6 | F | H | H | H | $CF_3$ | $CH_3$ |
| -7 | F | H | H | H | $OCF_3$ | $CH_3$ |
| -8 | F | H | H | H | $OCHF_2$ | $CH_3$ |
| -9 | F | F | H | H | F | $CH_3$ |
| -10 | F | F | H | H | $CF_3$ | $CH_3$ |
| -11 | F | F | H | H | $OCF_3$ | $CH_3$ |
| -12 | F | F | H | H | $OCHF_2$ | $CH_3$ |
| -13 | H | H | H | H | F | $C_2H_5$ |
| -14 | H | H | H | H | $CF_3$ | $C_2H_5$ |
| -15 | H | H | H | H | $OCF_3$ | $C_2H_5$ |
| -16 | H | H | H | H | $OCHF_2$ | $C_2H_5$ |
| -17 | F | H | H | H | F | $C_2H_5$ |
| -18 | F | H | H | H | $CF_3$ | $C_2H_5$ |
| -19 | F | H | H | H | $OCF_3$ | $C_2H_5$ |
| -20 | F | H | H | H | $OCHF_2$ | $C_2H_5$ |
| -21 | F | F | H | H | F | $C_2H_5$ |
| -22 | F | F | H | H | $CF_3$ | $C_2H_5$ |
| -23 | F | F | H | H | $OCF_3$ | $C_2H_5$ |
| -24 | F | F | H | H | $OCHF_2$ | $C_2H_5$ |
| -25 | H | H | H | H | F | n-$C_3H_7$ |
| -26 | H | H | H | H | $CF_3$ | n-$C_3H_7$ |
| -27 | H | H | H | H | $OCF_3$ | n-$C_3H_7$ |
| -28 | H | H | H | H | $OCHF_2$ | n-$C_3H_7$ |
| -29 | F | H | H | H | F | n-$C_3H_7$ |
| -30 | F | H | H | H | $CF_3$ | n-$C_3H_7$ |
| -31 | F | H | H | H | $OCF_3$ | n-$C_3H_7$ |
| -32 | F | H | H | H | $OCHF_2$ | n-$C_3H_7$ |
| -33 | F | F | H | H | F | n-$C_3H_7$ |
| -34 | F | F | H | H | $CF_3$ | n-$C_3H_7$ |
| -35 | F | F | H | H | $OCF_3$ | n-$C_3H_7$ |
| -36 | F | F | H | H | $OCHF_2$ | n-$C_3H_7$ |
| -37 | H | H | H | H | F | n-$C_4H_9$ |
| -38 | H | H | H | H | $CF_3$ | n-$C_4H_9$ |
| -39 | H | H | H | H | $OCF_3$ | n-$C_4H_9$ |

(fortgesetzt)

| I-MBBIa/I-MBBIIa/I-NBBIa/IQBAIa-Nr. | $L^{11}$ | $L^{12}$ | $L^{13}$ | $L^{14}$ | $R^{12}$ | $R^{11}$ |
|---|---|---|---|---|---|---|
| -40 | H | H | H | H | $OCHF_2$ | $n-C_4H_9$ |
| -41 | F | H | H | H | F | $n-C_4H_9$ |
| -42 | F | H | H | H | $CF_3$ | $n-C_4H_9$ |
| -43 | F | H | H | H | $OCF_3$ | $n-C_4H_9$ |
| -44 | F | H | H | H | $OCHF_2$ | $n-C_4H_9$ |
| -45 | F | F | H | H | F | $n-C_4H_9$ |
| -46 | F | F | H | H | $CF_3$ | $n-C_4H_9$ |
| -47 | F | F | H | H | $OCF_3$ | $n-C_4H_9$ |
| -48 | F | F | H | H | $OCHF_2$ | $n-C_4H_9$ |
| -49 | H | H | H | H | F | $n-C_5H_{11}$ |
| -50 | H | H | H | H | $CF_3$ | $n-C_5H_{11}$ |
| -51 | H | H | H | H | $OCF_3$ | $n-C_5H_{11}$ |
| -52 | H | H | H | H | $OCHF_2$ | $n-C_5H_{11}$ |
| -53 | F | H | H | H | F | $n-C_5H_{11}$ |
| -54 | F | H | H | H | $CF_3$ | $n-C_5H_{11}$ |
| -55 | F | H | H | H | $OCF_3$ | $n-C_5H_{11}$ |
| -56 | F | H | H | H | $OCHF_2$ | $n-C_5H_{11}$ |
| -57 | F | F | H | H | F | $n-C_5H_{11}$ |
| -58 | F | F | H | H | $CF_3$ | $n-C_5H_{11}$ |
| -59 | F | F | H | H | $OCF_3$ | $n-C_5H_{11}$ |
| -60 | F | F | H | H | $OCHF_2$ | $n-C_5H_{11}$ |
| -61 | F | F | F | H | F | $CH_3$ |
| -62 | F | F | F | H | $CF_3$ | $CH_3$ |
| -63 | F | F | F | H | $OCF_3$ | $CH_3$ |
| -64 | F | F | F | H | $OCHF_2$ | $CH_3$ |
| -65 | F | F | F | H | F | $C_2H_5$ |
| -66 | F | F | F | H | $CF_3$ | $C_2H_5$ |
| -67 | F | F | F | H | $OCF_3$ | $C_2H_5$ |
| -68 | F | F | F | H | $OCHF_2$ | $C_2H_5$ |
| -69 | F | F | F | H | F | $n-C_3H_7$ |
| -70 | F | F | F | H | $CF_3$ | $n-C_3H_7$ |
| -71 | F | F | F | H | $OCF_3$ | $n-C_3H_7$ |
| -72 | F | F | F | H | $OCHF_2$ | $n-C_3H_7$ |
| -73 | F | F | F | H | F | $n-C_4H_9$ |
| -74 | F | F | F | H | $CF_3$ | $n-C_4H_9$ |
| -75 | F | F | F | H | $OCF_3$ | $n-C_4H_9$ |
| -76 | F | F | F | H | $OCHF_2$ | $n-C_4H_9$ |
| -77 | F | F | F | H | F | $n-C_5H_{11}$ |
| -78 | F | F | F | H | $CF_3$ | $n-C_5H_{11}$ |
| -79 | F | F | F | H | $OCF_3$ | $n-C_5H_{11}$ |
| -80 | F | F | F | H | $OCHF_2$ | $n-C_5H_{11}$ |

**Tabelle 9**

| I-NBAIa/I-NBBIb/I-NBCIa/I-OBAIa/ I-OBBIa/I-PBAIa/I-PBBIa/I-QBAIb- Nr. | $L^{15}$ | $L^{16}$ | $L^{13}$ | $L^{14}$ | $L^{11}$ | $L^{12}$ | $R^{11}$ | $R^{12}$ |
|---|---|---|---|---|---|---|---|---|
| -1 | H | H | H | H | H | H | $CH_3$ | F |
| -2 | H | H | H | H | H | H | $CH_3$ | $CF_3$ |
| -3 | H | H | H | H | H | H | $CH_3$ | $OCF_3$ |
| -4 | H | H | H | H | H | H | $CH_3$ | $OCHF_2$ |

(fortgesetzt)

| I-NBAIa/I-NBBIb/I-NBCIa/I-OBAIa/ I-OBBIa/I-PBAIa/I-PBBIa/I-QBAIb- Nr. | $L^{15}$ | $L^{16}$ | $L^{13}$ | $L^{14}$ | $L^{11}$ | $L^{12}$ | $R^{11}$ | $R^{12}$ |
|---|---|---|---|---|---|---|---|---|
| -5 | H | H | H | H | F | H | $CH_3$ | F |
| -6 | H | H | H | H | F | H | $CH_3$ | $CF_3$ |
| -7 | H | H | H | H | F | H | $CH_3$ | $OCF_3$ |
| -8 | H | H | H | H | F | H | $CH_3$ | $OCHF_2$ |
| -9 | H | H | H | H | F | F | $CH_3$ | F |
| -10 | H | H | H | H | F | F | $CH_3$ | $CF_3$ |
| -11 | H | H | H | H | F | F | $CH_3$ | $OCF_3$ |
| -12 | H | H | H | H | F | F | $CH_3$ | $OCHF_2$ |
| -13 | H | H | F | H | F | F | $CH_3$ | F |
| -14 | H | H | F | H | F | F | $CH_3$ | $CF_3$ |
| -15 | H | H | F | H | F | F | $CH_3$ | $OCF_3$ |
| -16 | H | H | F | H | F | F | $CH_3$ | $OCHF_2$ |
| -17 | H | H | F | F | F | F | $CH_3$ | F |
| -18 | H | H | F | F | F | F | $CH_3$ | $CF_3$ |
| -19 | H | H | F | F | F | F | $CH_3$ | $OCF_3$ |
| -20 | H | H | F | F | F | F | $CH_3$ | $OCHF_2$ |
| -21 | F | H | F | F | F | F | $CH_3$ | F |
| -22 | F | H | F | F | F | F | $CH_3$ | $CF_3$ |
| -23 | F | H | F | F | F | F | $CH_3$ | $OCF_3$ |
| -24 | F | H | F | F | F | F | $CH_3$ | $OCHF_2$ |
| -25 | H | H | F | H | F | H | $CH_3$ | F |
| -26 | H | H | F | H | F | H | $CH_3$ | $CF_3$ |
| -27 | H | H | F | H | F | H | $CH_3$ | $OCF_3$ |
| -28 | H | H | F | H | F | H | $CH_3$ | $OCHF_2$ |
| -29 | H | H | H | H | H | H | $C_2H_5$ | F |
| -30 | H | H | H | H | H | H | $C_2H_5$ | $CF_3$ |
| -31 | H | H | H | H | H | H | $C_2H_5$ | $OCF_3$ |
| -32 | H | H | H | H | H | H | $C_2H_5$ | $OCHF_2$ |
| -33 | H | H | H | H | F | H | $C_2H_5$ | F |
| -34 | H | H | H | H | F | H | $C_2H_5$ | $CF_3$ |
| -35 | H | H | H | H | F | H | $C_2H_5$ | $OCF_3$ |
| -36 | H | H | H | H | F | H | $C_2H_5$ | $OCHF_2$ |
| -37 | H | H | H | H | F | F | $C_2H_5$ | F |
| -38 | H | H | H | H | F | F | $C_2H_5$ | $CF_3$ |
| -39 | H | H | H | H | F | F | $C_2H_5$ | $OCF_3$ |
| -40 | H | H | H | H | F | F | $C_2H_5$ | $OCHF_2$ |
| -41 | H | H | F | H | F | F | $C_2H_5$ | F |
| -42 | H | H | F | H | F | F | $C_2H_5$ | $CF_3$ |
| -43 | H | H | F | H | F | F | $C_2H_5$ | $OCF_3$ |
| -44 | H | H | F | H | F | F | $C_2H_5$ | $OCHF_2$ |
| -45 | H | H | F | F | F | F | $C_2H_5$ | F |
| -46 | H | H | F | F | F | F | $C_2H_5$ | $CF_3$ |
| -47 | H | H | F | F | F | F | $C_2H_5$ | $OCF_3$ |
| -48 | H | H | F | F | F | F | $C_2H_5$ | $OCHF_2$ |
| -49 | F | H | F | F | F | F | $C_2H_5$ | F |
| -50 | F | H | F | F | F | F | $C_2H_5$ | $CF_3$ |
| -51 | F | H | F | F | F | F | $C_2H_5$ | $OCF_3$ |
| -52 | F | H | F | F | F | F | $C_2H_5$ | $OCHF_2$ |
| -53 | H | H | F | H | F | H | $C_2H_5$ | F |

(fortgesetzt)

| I-NBAIa/I-NBBIb/I-NBCIa/I-OBAIa/ I-OBBIa/I-PBAIa/I-PBBIa/I-QBAIb- Nr. | $L^{15}$ | $L^{16}$ | $L^{13}$ | $L^{14}$ | $L^{11}$ | $L^{12}$ | $R^{11}$ | $R^{12}$ |
|---|---|---|---|---|---|---|---|---|
| -54 | H | H | F | H | F | H | $C_2H_5$ | $CF_3$ |
| -55 | H | H | F | H | F | H | $C_2H_5$ | $OCF_3$ |
| -56 | H | H | F | H | F | H | $C_2H_5$ | $OCHF_2$ |
| -57 | H | H | H | H | H | H | n-$C_3H_7$ | F |
| -58 | H | H | H | H | H | H | n-$C_3H_7$ | $CF_3$ |
| -59 | H | H | H | H | H | H | n-$C_3H_7$ | $OCF_3$ |
| -60 | H | H | H | H | H | H | n-$C_3H_7$ | $OCHF_2$ |
| -61 | H | H | H | H | F | H | n-$C_3H_7$ | F |
| -62 | H | H | H | H | F | H | n-$C_3H_7$ | $CF_3$ |
| -63 | H | H | H | H | F | H | n-$C_3H_7$ | $OCF_3$ |
| -64 | H | H | H | H | F | H | n-$C_3H_7$ | $OCHF_2$ |
| -65 | H | H | H | H | F | F | n-$C_3H_7$ | F |
| -66 | H | H | H | H | F | F | n-$C_3H_7$ | $CF_3$ |
| -67 | H | H | H | H | F | F | n-$C_3H_7$ | $OCF_3$ |
| -68 | H | H | H | H | F | F | n-$C_3H_7$ | $OCHF_2$ |
| -69 | H | H | F | H | F | F | n-$C_3H_7$ | F |
| -70 | H | H | F | H | F | F | n-$C_3H_7$ | $CF_3$ |
| -71 | H | H | F | H | F | F | n-$C_3H_7$ | $OCF_3$ |
| -72 | H | H | F | H | F | F | n-$C_3H_7$ | $OCHF_2$ |
| -73 | H | H | F | F | F | F | n-$C_3H_7$ | F |
| -74 | H | H | F | F | F | F | n-$C_3H_7$ | $CF_3$ |
| -75 | H | H | F | F | F | F | n-$C_3H_7$ | $OCF_3$ |
| -76 | H | H | F | F | F | F | n-$C_3H_7$ | $OCHF_2$ |
| -77 | F | H | F | F | F | F | n-$C_3H_7$ | F |
| -78 | F | H | F | F | F | F | n-$C_3H_7$ | $CF_3$ |
| -79 | F | H | F | F | F | F | n-$C_3H_7$ | $OCF_3$ |
| -80 | F | H | F | F | F | F | n-$C_3H_7$ | $OCHF_2$ |
| -81 | H | H | F | H | F | H | n-$C_3H_7$ | F |
| -82 | H | H | F | H | F | H | n-$C_3H_7$ | $CF_3$ |
| -83 | H | H | F | H | F | H | n-$C_3H_7$ | $OCF_3$ |
| -84 | H | H | F | H | F | H | n-$C_3H_7$ | $OCHF_2$ |
| -85 | H | H | H | H | H | H | n-$C_4H_9$ | F |
| -86 | H | H | H | H | H | H | n-$C_4H_9$ | $CF_3$ |
| -87 | H | H | H | H | H | H | n-$C_4H_9$ | $OCF_3$ |
| -88 | H | H | H | H | H | H | n-$C_4H_9$ | $OCHF_2$ |
| -89 | H | H | H | H | F | H | n-$C_4H_9$ | F |
| -90 | H | H | H | H | F | H | n-$C_4H_9$ | $CF_3$ |
| -91 | H | H | H | H | F | H | n-$C_4H_9$ | $OCF_3$ |
| -92 | H | H | H | H | F | H | n-$C_4H_9$ | $OCHF_2$ |
| -93 | H | H | H | H | F | F | n-$C_4H_9$ | F |
| -94 | H | H | H | H | F | F | n-$C_4H_9$ | $CF_3$ |
| -95 | H | H | H | H | F | F | n-$C_4H_9$ | $OCF_3$ |
| -96 | H | H | H | H | F | F | n-$C_4H_9$ | $OCHF_2$ |
| -97 | H | H | F | H | F | F | n-$C_4H_9$ | F |
| -98 | H | H | F | H | F | F | n-$C_4H_9$ | $CF_3$ |
| -99 | H | H | F | H | F | F | n-$C_4H_9$ | $OCF_3$ |
| -100 | H | H | F | H | F | F | n-$C_4H_9$ | $OCHF_2$ |
| -101 | H | H | F | F | F | F | n-$C_4H_9$ | F |
| -102 | H | H | F | F | F | F | n-$C_4H_9$ | $CF_3$ |

(fortgesetzt)

| I-NBAIa/I-NBBIb/I-NBCIa/I-OBAIa/ I-OBBIa/I-PBAIa/I-PBBIa/I-QBAIb- Nr. | $L^{15}$ | $L^{16}$ | $L^{13}$ | $L^{14}$ | $L^{11}$ | $L^{12}$ | $R^{11}$ | $R^{12}$ |
|---|---|---|---|---|---|---|---|---|
| -103 | H | H | F | F | F | F | $n-C_4H_9$ | $OCF_3$ |
| -104 | H | H | F | F | F | F | $n-C_4H_9$ | $OCHF_2$ |
| -105 | F | H | F | F | F | F | $n-C_4H_9$ | F |
| -106 | F | H | F | F | F | F | $n-C_4H_9$ | $CF_3$ |
| -107 | F | H | F | F | F | F | $n-C_4H_9$ | $OCF_3$ |
| -108 | F | H | F | F | F | F | $n-C_4H_9$ | $OCHF_2$ |
| -109 | H | H | F | H | F | H | $n-C_4H_9$ | F |
| -110 | H | H | F | H | F | H | $n-C_4H_9$ | $CF_3$ |
| -111 | H | H | F | H | F | H | $n-C_4H_9$ | $OCF_3$ |
| -112 | H | H | F | H | F | H | $n-C_4H_9$ | $OCHF_2$ |
| -113 | H | H | H | H | H | H | $n-C_5H_{11}$ | F |
| -114 | H | H | H | H | H | H | $n-C_5H_{11}$ | $CF_3$ |
| -115 | H | H | H | H | H | H | $n-C_5H_{11}$ | $OCF_3$ |
| -116 | H | H | H | H | H | H | $n-C_5H_{11}$ | $OCHF_2$ |
| -117 | H | H | H | H | F | H | $n-C_5H_{11}$ | F |
| -118 | H | H | H | H | F | H | $n-C_5H_{11}$ | $CF_3$ |
| -119 | H | H | H | H | F | H | $n-C_5H_{11}$ | $OCF_3$ |
| -120 | H | H | H | H | F | H | $n-C_5H_{11}$ | $OCHF_2$ |
| -121 | H | H | H | H | F | F | $n-C_5H_{11}$ | F |
| -122 | H | H | H | H | F | F | $n-C_5H_{11}$ | $CF_3$ |
| -123 | H | H | H | H | F | F | $n-C_5H_{11}$ | $OCF_3$ |
| -124 | H | H | H | H | F | F | $n-C_5H_{11}$ | $OCHF_2$ |
| -125 | H | H | F | H | F | F | $n-C_5H_{11}$ | F |
| -126 | H | H | F | H | F | F | $n-C_5H_{11}$ | $CF_3$ |
| -127 | H | H | F | H | F | F | $n-C_5H_{11}$ | $OCF_3$ |
| -128 | H | H | F | H | F | F | $n-C_5H_{11}$ | $OCHF_2$ |
| -129 | H | H | F | F | F | F | $n-C_5H_{11}$ | F |
| -130 | H | H | F | F | F | F | $n-C_5H_{11}$ | $CF_3$ |
| -131 | H | H | F | F | F | F | $n-C_5H_{11}$ | $OCF_3$ |
| -132 | H | H | F | F | F | F | $n-C_5H_{11}$ | $OCHF_2$ |
| -133 | F | H | F | F | F | F | $n-C_5H_{11}$ | F |
| -134 | F | H | F | F | F | F | $n-C_5H_{11}$ | $CF_3$ |
| -135 | F | H | F | F | F | F | $n-C_5H_{11}$ | $OCF_3$ |
| -136 | F | H | F | F | F | F | $n-C_5H_{11}$ | $OCHF_2$ |
| -137 | H | H | F | H | F | H | $n-C_5H_{11}$ | F |
| -138 | H | H | F | H | F | H | $n-C_5H_{11}$ | $CF_3$ |
| -139 | H | H | F | H | F | H | $n-C_5H_{11}$ | $OCF_3$ |
| -140 | H | H | F | H | F | H | $n-C_5H_{11}$ | $OCHF_2$ |

Zu den bevorzugten Verbindungen der Erfindung zählen ferner Pyranderivate der Formel I, bei denen e 1 ist und $A^{15}$-$R^{12}$ für

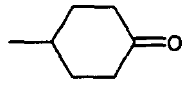

beziehungsweise

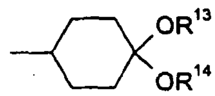

steht, wobei $R^{13}$ und $R^{14}$ unabhängig voneinander einen Alkanylrest mit 1 bis 7 Kohlenstoffatomen bedeutet oder zusammen für eine Alkylenbrücke mit 2 bis 7 Kohlenstoffatomen stehen. Die Cyclohexanone werden durch die Formel I-R und ihre Ketale durch Formel I-S veranschaulicht:

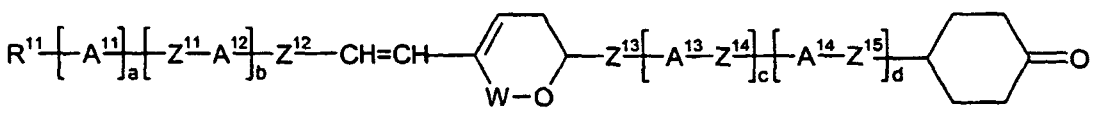

I-R

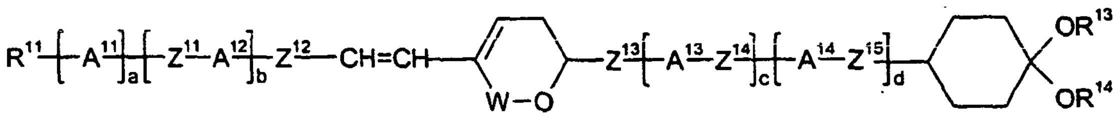

I-S

wobei $R^{11}$, $R^{13}$, $R^{14}$, a, b, c, d, $Z^{11}$, $Z^{12}$, $Z^{13}$, $Z^{14}$, $Z^{15}$, $A^{11}$, $A^{12}$, $A^{13}$ und $A^{14}$ die gleiche Bedeutung wie für Formel I oben besitzen. Bevorzugte Untergruppen von Verbindungen der Formeln I-R und I-S werden von Verbindungen mit W = -C(=O)- (Formel I-RA beziehungsweise I-SA) und insbesondere W = $-CH_2-$ (Formel I-RB beziehungsweise I-SB) gebildet.

Besonders bevorzugte Verbindungen der Formeln I-RB und I-SB sind Verbindungen der Formeln I-RBA, I-SBA, I-RBB und I-SBB:

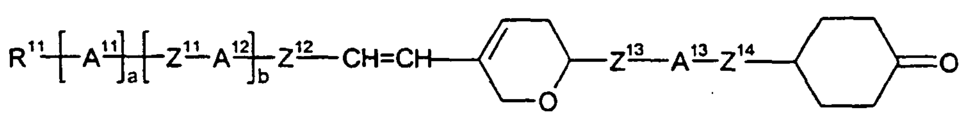

I-RBA

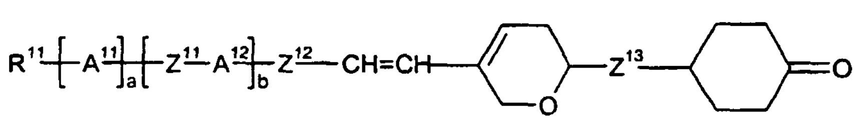

I-RBB

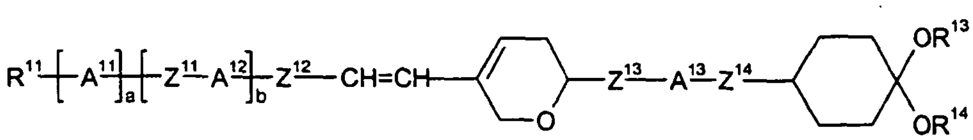

I-SBA

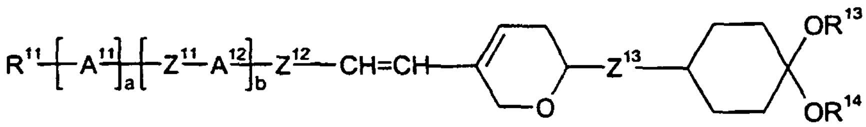

I-SBB

wobei $R^{11}$, $R^{13}$, $R^{14}$, a, b, $Z^{11}$, $Z^{12}$, $Z^{13}$, $Z^{14}$, $A^{11}$, $A^{12}$ und $A^{13}$ die gleiche Bedeutung wie für Formel I oben besitzen. $R^{11}$ ist vorzugsweise ein geradkettiger Alkenyl- oder insbesondere Alkanylrest mit 1 bis 7 Kohlenstoffatomen. $R^{13}$ und $R^{14}$ bedeuten bevorzugt jeweils Methyl oder Ethyl oder zusammen $-CH_2CH_2-$ oder $-CH_2C(CH_3)_2-CH_2-$. $Z^{12}$ und $Z^{14}$ stehen jeweils bevorzugt für eine Einfachbindung, während $Z^{13}$ vorzugsweise eine Einfachbindung, -C(=O)O- oder $-CF_2O-$ bedeutet. Unter diesen Verbindungen sind jene der Formeln I-RBAI, I-RBAII, I-RBBI und I-RBBII und die entsprechenden Ketale I-SBAI, I-SBAII, I-SBBI und I-SBBII ganz besonders bevorzugt:

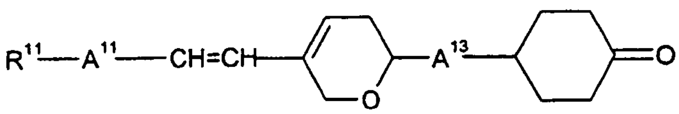

I-RBAI

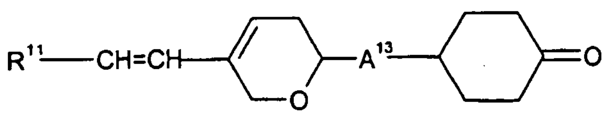

I-RBAII

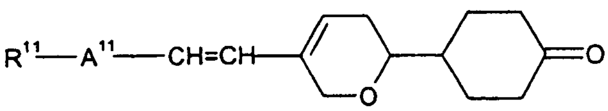

I-RBBI

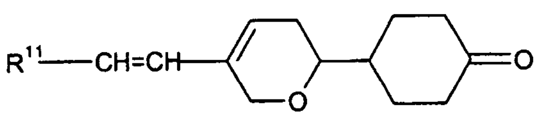

I-RBBII

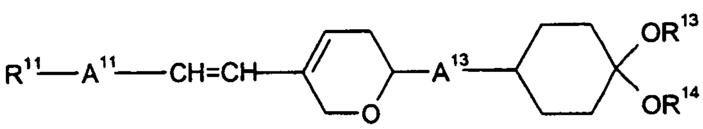

I-SBAI

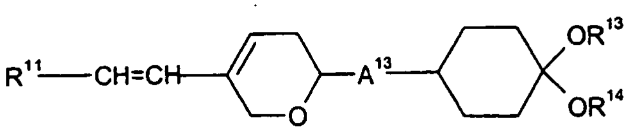

I-SBAII

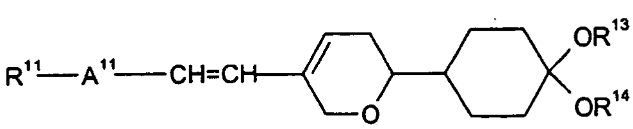

I-SBBI

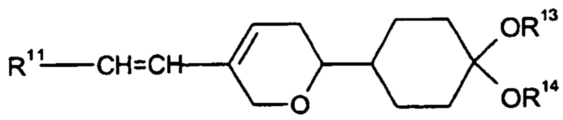

I-SBBII

wobei $R^{11}$, $R^{13}$, $R^{14}$, $A^{11}$ und $A^{13}$ die für Formel I angegebene Bedeutung aufweisen. Vorzugsweise steht $R^{11}$ für H, Alkanyl oder Alkenyl mit 1 bis 5 Kohlenstoffatomen, $A^{11}$ für 1,4-Cyclohexylen und $A^{13}$ für 1,4-Cyclohexylen oder gegebenenfalls in 3- und/oder 5-Position Fluor-substituiertes 1,4-Phenylen. $R^{13}$ und $R^{14}$ bedeuten bevorzugt jeweils Methyl oder Ethyl oder zusammen $-CH_2CH_2-$ oder $-CH_2C(CH_3)_2-CH_2-$.

Ein weiterer erfindungsgemäßer Gegenstand sind Verfahren zur Herstellung von Pyranderivaten, insbesondere der allgemeinen Formel I.

Ein erstes erfindungsgemäßes Verfahren ist dadurch gekennzeichnet, dass es als einen Verfahrensschritt eine Enin-Metathese-Reaktion eines Enins der allgemeinen Formel II zu einem erfindungsgemäßen Pyranderivat der Formel I in Gegenwart eines Metathese-Katalysators umfasst:

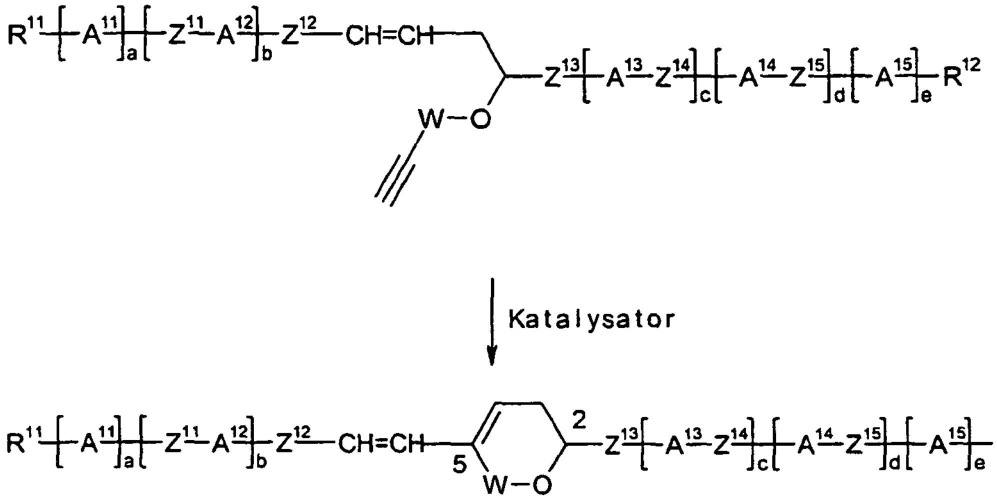


II

I

**Schema 1**

wobei a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ in den Formeln I und II die gleiche Bedeutung wie für Formel I oben haben; mit der Maßgabe, dass für den Fall der direkten Verknüpfung von $Z^{13}$ und $R^{12}$ zu $-Z^{13}-R^{12}$ $R^{12}$ H, Aralkyl oder Alkanyl bedeutet, wenn $Z^{13}$-C(=O)- bedeutet, $R^{12}$ Aralkyl oder Alkanyl bedeutet, wenn $Z^{13}$ -C(=O)-O-bedeutet, und $Z^{13}$ nicht $-CH_2O-$ oder $-CF_2O-$ bedeutet; dass für den Fall der direkten Verknüpfung von $Z^{14}$ und $R^{12}$ zu $-Z^{14}-R^{12}$ $R^{12}$ H, Aralkyl oder Alkanyl bedeutet, wenn $Z^{14}$ -C(=O)- bedeutet, $R^{12}$ Aralkyl oder Alkanyl bedeutet, wenn $Z^{14}$ -C(=O)-O- bedeutet, und $Z^{14}$ nicht $-CH_2O-$ oder $-CF_2O-$bedeutet; dass für den Fall der direkten Verknüpfung von $Z^{15}$ und $R^{12}$ zu $-Z^{15}-R^{12}$ $R^{12}$ H, Aralkyl oder Alkanyl bedeutet, wenn $Z^{15}$ -C(=O)- bedeutet, $R^{12}$ Aralkyl oder Alkanyl bedeutet, wenn $Z^{15}$ -C(=O)-O- bedeutet, und $Z^{15}$ nicht $-CH_2O-$ oder $-CF_2O-$ bedeutet.

Im allgemeinen wird das Pyranderivat der Formel überwiegend oder ausschließlich als E-Isomer bezüglich der exocylischen Doppelbindung erhalten.

Metathese-Reaktionen (auch häufig als Olefin-Metathese bezeichnet) zur Synthese von Carbocyclen (bzw. von (Poly-)Olefinen aus Carbocyclen) sind im Stand der Technik gut bekannt (siehe u.a. R. H. Grubbs und S. Chang, *Tetrahedron* 54 (1998) 4413; T. M. Trnka und R. H. Grubbs, Acc. Chem. Res. **2001**, *34*, 18; S. K. Armstrong, *J. Chem.* Soc., *Perkin Trans. I,* 1998, 371; A. Fürstner et al., *Chem. Eur. J.* **2001,** *7*, 3236, und dort zitierte Literaturstellen). Mit ihrer Hilfe können durch gleichzeitige Spaltung und Knüpfung von ungesättigten Kohlenstoff-Kohlenstoff-Bindungen insbesondere in Gegenwart ausgewählter Metall-Carben-Komplexe neue C-C-Bindungen und somit komplexere Moleküle aufgebaut werden.

Dabei kann man verschiedene ringschließende und ringöffnende Metathese-Reaktionstypen unterscheiden. Neben der hier nicht weiter interessierenden ringöffnenden polymerisierenden Metathese (ROMP; Ring-opening metathesis polymerization) sind insbesondere die (intramolekulare) ringschließende Metathese (RCM; Ring closing metathesis), die Kreuzmetathese (CM; Cross metathesis) und die Enin-Metathese (Enin) von besonderer Bedeutung. Die genannten Metathese-Typen sind in Schema 2 am Beispiel eines Kohlenwasserstoffs veranschaulicht:

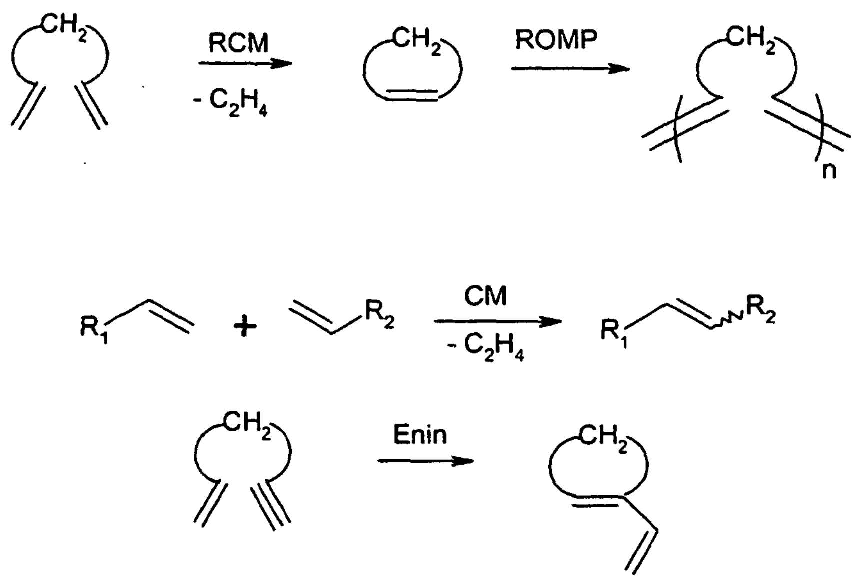


**Schema 2**

Die Metathese-Reaktionen werden bevorzugt durch so genannte Schrock (oder auch Grubbs-)Carben-Komplexe (Metall-Alkyliden-Komplexe) von Übergangsmetallen wie Wolfram, Molybdän und insbesondere Ruthenium katalysiert. Diese Komplexe weisen zumeist eine Struktur auf, die durch die folgende Formel COMP-A wiedergegeben werden kann (vgl. u.a. WO 96/04289, WO 97/06185, WO 99/00396 und WO 99/00397):

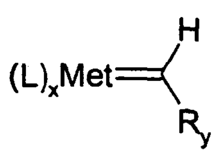

COMP-A

Dabei steht Met für ein Übergangsmetall, $(L)_x$ für mehrere gleiche oder verschiedene Liganden und Ry für einen organischen Rest, zumeist Aryl, Alkanyl oder Alkenyl.

Die genannten ringschließenden Metathese-Reaktionen sind auch eingesetzt worden, um heterocyclische Ringsysteme zu bilden und entsprechende Verbindungen darzustellen. So sind Stickstoff-Heterocyclen in großer Vielzahl durch Olefin-Metathese zugänglich. Auch Sauerstoff-Heterocyclen sind laut Literatur mit Hilfe dieser synthetischen Methodologie herstellbar, jedoch offenbar in deutlich geringerer struktureller Breite.

So sind von gut zugänglichen Ausgangsverbindungen ausgehende Metathese-Verfahren zur Synthese von Pyranderivaten, die sowohl einen exocyclischen Substituenten an einem Kohlenstoffatom der durch Metathese gebildeten endocyclischen C=C-Doppelbindung und eine 2,5-Disubstitution aufweisen

( 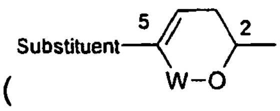 ),

als auch über technisch nützliche Eigenschaften verfügen und zum Beispiel als mesogene Materialien oder als Vorstufen zur Herstellung von anderen einen Pyranring enthaltenden Verbindungen mit mesogenen Eigenschaften Verwendung finden können, bislang im Stand der Technik nicht beschrieben worden. Möglicherweise ist das darauf zurückzuführen, dass die Ringschlussreaktion zu O-Heterocyclen unter Metathesebedingungen bis zur vorliegenden Erfindung gewöhnlich nicht oder nicht reproduzierbar gelungen ist, wenn eines der Kohlenstoffatome einer der reagierenden C=C-Doppelbindungen der Ausgangsverbindung(en) disubstituiert ist (vgl. auch S. K. Armstrong, *J. Chem.* Soc., *Perkin Trans. I,* 1998, 371, insbesondere S. 376).

Im Hinblick auf die Enin-Metathese von Pyranderivaten ist im Stand der Technik lediglich eine einzelne Synthese ausgehend von einem Alkin-1-ylboronat beschrieben worden (J. Renaud et al., *Angew. Chem.* **2000**, *112*, 3231), was jedoch zunächst die Herstellung des entsprechenden Boronats erforderlich macht.

Es ist daher besonders überraschend, dass die Enin-Metathese ausgehend von Verbindungen der Formel II in Gegenwart eines Metathese-Katalysators zuverlässig und effizient zu den erfindungsgemäßen Pyranderivaten der Formel führt.

Als Metathese-Katalysator für die Enin-Metathese finden bevorzugt die im Stand der Technik beschriebenen Übergangsmetall-Alkyliden-Komplexe der allgemeinen Formel COMP-A Verwendung (siehe u.a. R. H. Grubbs und S. Chang, *Tetrahedron* 54 (1998) 4413; T. M. Trnka und R. H. Grubbs, Acc. Chem. Res. **2001,** *34*, 18; S. K. Armstrong, *J. Chem. Soc.*, *Perkin Trans. I,* 1998, 371; A. Fürstner et al., *Chem. Eur. J.* **2001,** *7*, 3236, und dort zitierte Literaturstellen). Bevorzugt handelt es sich dabei um einen Komplex der Formel COMP-A mit Met = Wolfram, Molybdän oder Ruthenium. Ferner finden Oxo-Wolfram-Komplexe des Typs trans-$W(=O)Cl_2(Aryl)_2$ (mit Aryl vorzugsweise gleich 2,6-Dibromphenyl), die unter anderem von W. A Nugent et al., J. Am. Chem. Soc., 1995, 117, 8992, beschrieben worden sind, Verwendung als Metathese-Katalysatoren in den erfindungsgemäßen Verfahren.

Bevorzugte Molybdän-Metathese-Katalysatoren sind solche der Formeln COMP-Mo1, COMP-Mo2, COMP-Mo3 und COMP-Mo4, während Formel COMP-W1 einen bevorzugten Wolfram-Metathese-Katalysator zeigt:

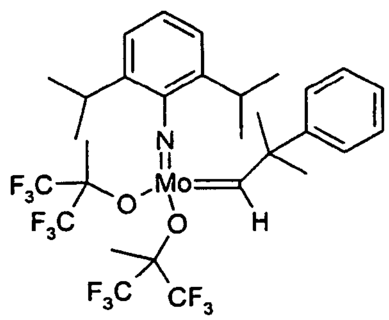

COMP-Mo1

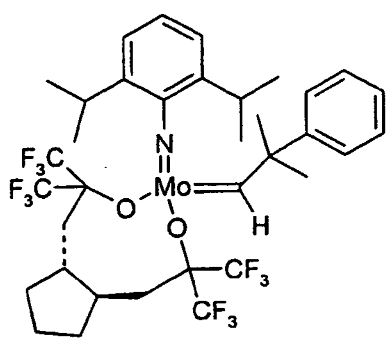

COMP-Mo2

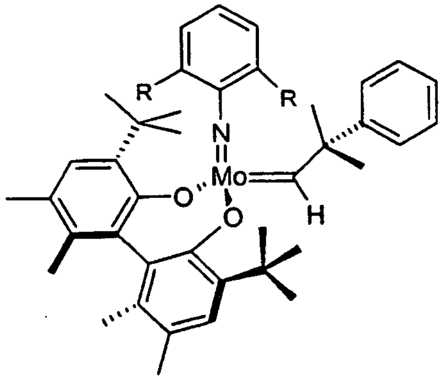

COMP-Mo3

a: R = i-Propyl
b: R = Methyl
c: R = Chlor

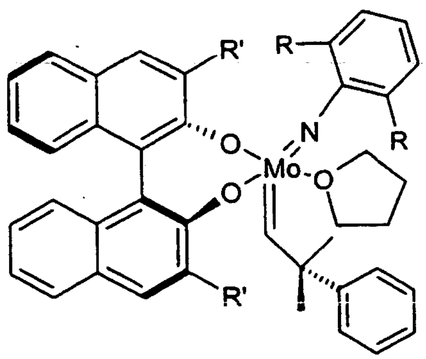

R' = 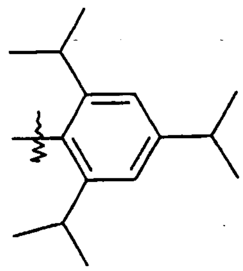

COMP-Mo4
R = i-Propyl

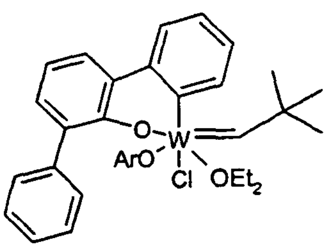

Ar = 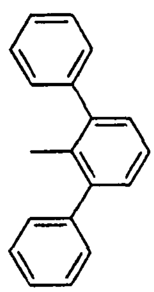

COMP-W1

Et = Ethyl

Besonders bevorzugte Komplexe zur Verwendung als Enin-Metathese-Katalysator in dem ersten erfindungsgemäßen Verfahren sind Ruthenium-Alkyliden-Komplexe des Typs COMP-RuA, die an sich literaturbekannt sind (siehe u.a. WO 96/04289, WO 97/06185, WO 99/00396, WO 99/00397, WO 99/29701, WO 99/51344, WO 00/15339, EP 1 022 282 A2, WO 00/58322, WO 00/71554, WO 02/14336, WO 02/14376, WO 02/083742):

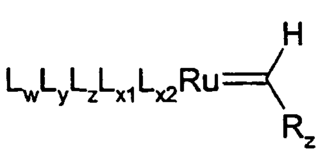

COMP-RuA

Vorzugsweise sind dabei $L_{x1}$ und/oder $L_{x2}$ anionische Liganden, bevorzugt Brom, Iod oder insbesondere Chlor,

gegebenenfalls auch O-Aralkyl, während $L_y$ und $L_z$ vorzugsweise andere, zumeist neutrale Liganden wie zum Beispiel $PPh_3$ (Ph = Phenyl), $P(i\text{-}Pr)_3$ (i-Pr = iso-Propyl), $PCy_3$ (Cy = Cyclohexyl), $P(Cp)_3$ (Cp = Cyclopentadienyl), unsubstituiertes oder substituiertes Pyridyl, $Im^1$, $Im^2$ oder über das Sauerstoffatom koordinierendes Alkoxy darstellen. $L_w$ steht für einen optional vorhandenen Liganden, d.h. w ist 0 oder 1, wobei dieser zumeist die gleiche Bedeutung hat wie $L_{x1}$, $L_{x2}$, $L_y$ und/oder $L_z$. $R_z$ steht für einen organischen Rest, insbesondere Aryl, Alkanyl oder Alkenyl.

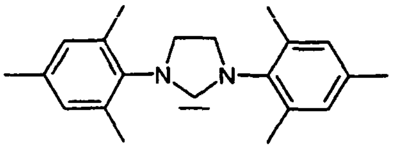

$Im^1$

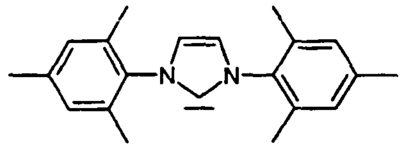

$Im^2$

Diese Ruthenium-Komplexe sind im allgemeinen wenig empfindlich für Luftsauerstoff und tolerieren sowohl Feuchtigkeitsspuren als auch geringe Verunreinigungen. Unter den Ruthenium-Alkyliden-Komplexen COMP-RuA sind beispielhaft solche der folgenden Formeln COMP-Ru1 bis COMP-Ru13 zu nennen:

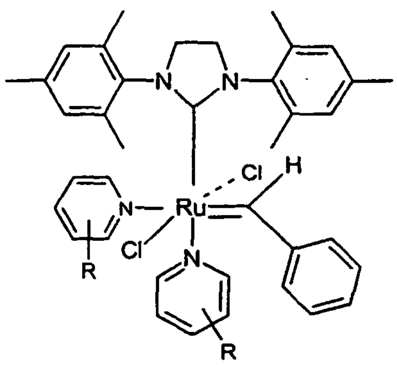

COMP-Ru1

a: R = H
b: R = 3-Br
c: R = 4-Phenyl

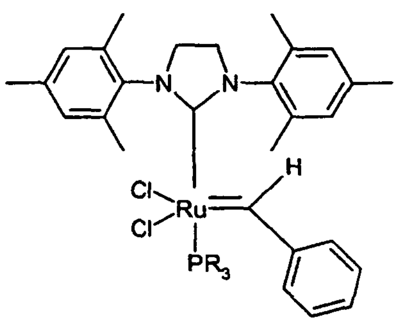

COMP-Ru2

a: R = Cy
b: R = Phenyl
c: R = p-$CF_3$-Phenyl

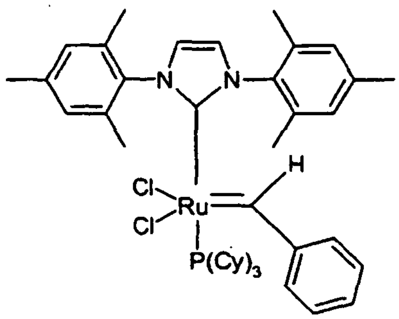

COMP-Ru3

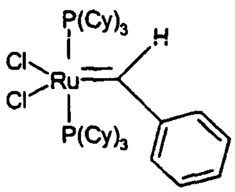

COMP-Ru4

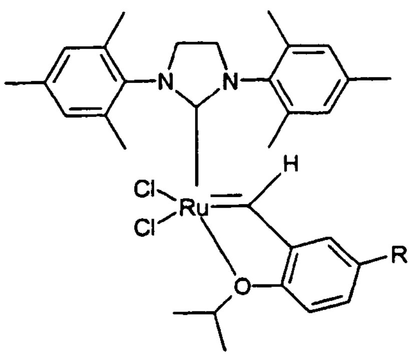

COMP-Ru5

a: R = H
b: R = Br
c: R = $NO_2$

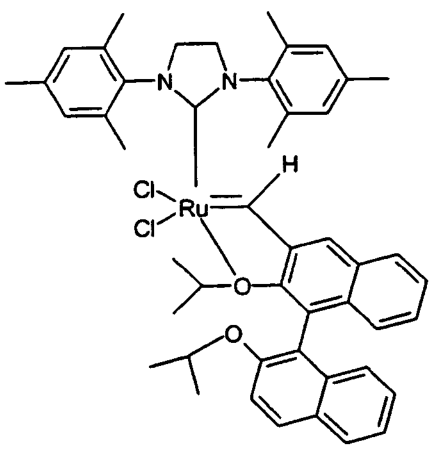

COMP-Ru6

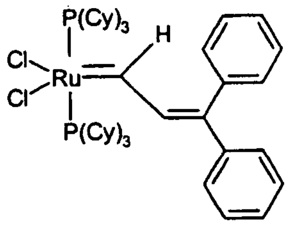

COMP-Ru7

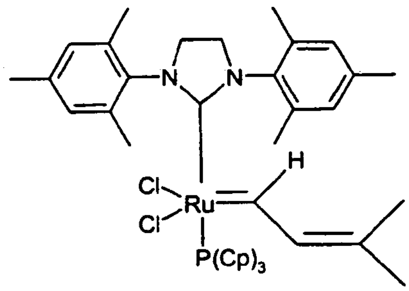

COMP-Ru8

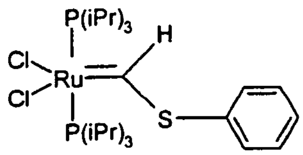

COMP-Ru9

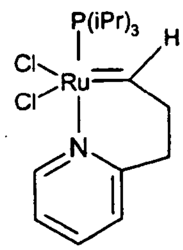

COMP-Ru10

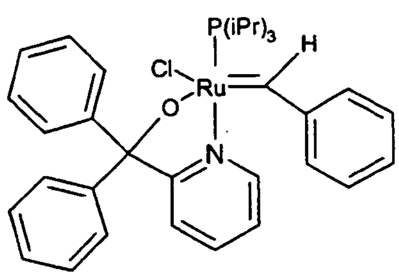

COMP-Ru11

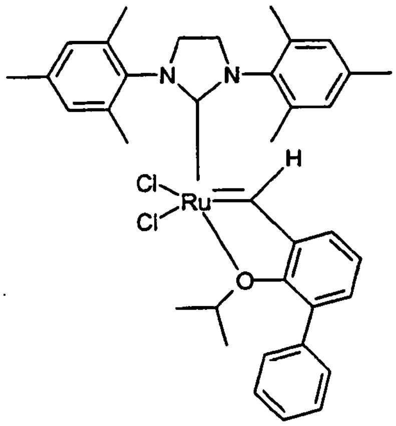

COMP-Ru12

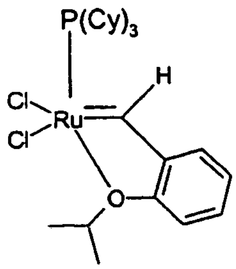

COMP-Ru13

Die als Metathese-Katalysatoren eingesetzten Alkyliden-Komplexe werden entweder nach literaturbekannten Verfahren hergestellt (siehe neben WO 96/04289, WO 97/06185, WO 99/00396, WO 99/00397, WO 99/29701, WO 99/51344, WO 00/15339, EP 1 022 282 A2, WO 00/58322, WO 00/71554, WO 02/14336, WO 02/14376, WO 02/083742 u.a. auch R. H. Grubbs und S. Chang, *Tetrahedron* 54 (1998) 4413; T. M. Trnka und R. H. Grubbs, Acc. Chem. Res. **2001,** *34*, 18; S. K. Armstrong, *J. Chem. Soc., Perkin Trans. I,* 1998, 371; A. Fürstner et al., *Chem. Eur. J.* **2001,** *7*, 3236; J. A. Love et al., Angew. Chem. 2002, 114, 4207; K. Grela et al., Angew. Chem. 2002, 114, 4210; G. S. Weatherhead et al., Tetrahedron Lett. 41 (2000) 9553, J. S. Kingsbury et al., J. Am. Chem. Soc. 1999, 121, 791, und dort angegebene Literaturstellen) oder sind kommerziell erhältlich, zum Beispiel von Sigma-Aldrich, Inc. (USA), oder Strem Chemicals Inc. (Kehl, Deutschland).

Einige dieser Katalysatoren können auch an immobilisierenden Trägern, zum Beispiel aus Polystyrol (z.B. COMP-Ru3: M. Ahmed et al., Synlett 2000, 1007; oder COMP-Ru5a und davon abgeleitete Komplexe: St. Randl et al., Synlett 2001, 1547) oder Glas (z.B. COMP-Ru5 und davon abgeleitete Komplexe: J. S. Kingsbury et al., Angew. Chem. 2001, *113*, 4381), angebracht sein.

Ganz besonders bevorzugt wird als Katalysator für den Enin-Metathese-Schritt des ersten erfindungsgemäßen Verfahrens ein Metall-Komplex, ausgewählt aus der Gruppe bestehend aus Komplexen der Formeln COMP-Ru1, -Ru2, -Ru3, -Ru4, -Ru5, -Ru6, -Ru7, -Ru8, -Ru9, -Ru10, -Ru11, -Ru12, -Ru13, insbesondere der Formeln COMP-Ru2a, COMP-Ru3, COMP-Ru4, COMP-Ru5a und COMP-Ru13 verwendet. Üblicherweise beträgt der Gehalt an Katalysator von 0,01 bis 10 mol-% (bezogen auf das Enin), bevorzugt 0,1 bis 5 mol-%, insbesondere 0,5 mol-% bis 2,5 mol-%.

Die Enin-Metathese des erfindungsgemäßen Verfahrens wird unter für solche Metallkomplex-katalysierten Reaktionen üblichen Bedingungen ausgeführt. Die Reaktion erfolgt lösungsmittelfrei oder in einem geeigneten Solvens. Als Lösungsmittel wird ein inertes Lösungsmittel, bevorzugt ein aromatisches und/oder halogeniertes organisches Lösungsmittel und insbesondere Toluol, Benzol, Chlorbenzol oder Dichlormethan verwendet. Die Reaktion erfolgt in der Regel bei Temperaturen von Raumtemperatur bis Siedetemperatur des Lösungsmittels (z.B. ca. 20 °C bis 40 °C (Dichlormethan) beziehungsweise bis 80°C oder 110°C (Toluol)). Bevorzugt sind Reaktionstemperaturen zwischen 20 und 60 °C. Die Reaktionszeit ist an sich nicht kritisch, da die Enin-Metathese im allgemeinen unter vollständiger Umsetzung des Edukts der Formel II abläuft und das gewünschte Pyranderivat der Formel I in guten Ausbeuten ergibt. Üblicherweise beträgt die Reaktionsdauer von 30 min bis 8 Tagen, bevorzugt von 1 h bis 3 Tagen, insbesondere von 4 h bis 24 h, wobei die genaue Reaktionszeit vom Fachmann in Abhängigkeit vom jeweiligen Katalysator, seiner Menge und Konzentration und vom Verlauf der Umsetzung ausgewählt werden kann.

Es ist ferner vorteilhaft, den Katalysator dem Reaktionsgemisch portionsweise zuzusetzen, was eine bessere Steuerung der Umsetzung erlaubt und zu einer Einsparung an Katalysator-Gesamtmenge führt. Ferner ist es in einigen Fällen vorteilhaft, bei der Enin-Metathese neben dem eigentlichen Metathese-Katalysator einen Co-Katalysator, zum Beispiel Kupfer(1)bromid, in Mengen bis zu ca. 5 Äquivalenten (bezogen auf den Metathese-Katalysator) einzusetzen.

Ferner betrifft die vorliegende Erfindung ein zweites erfindungsgemäßes Verfahren zur Herstellung von Pyranen, das durch einen Verfahrenschritt gekennzeichnet ist, der eine Kreuz-Metathese-Reaktion eines Dihydropyranderivats der allgemeinen Formel I-C mit einem Alken der Formel IIIa oder IIIb

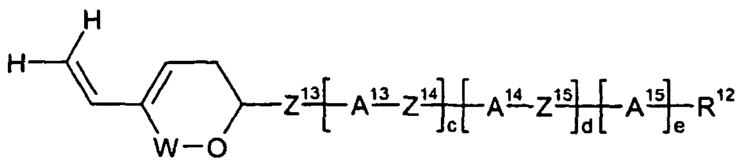

I-C

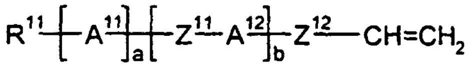

IIIa

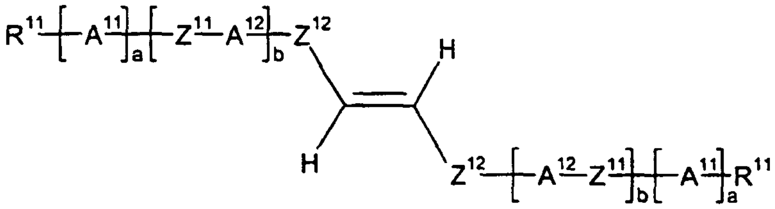

IIIb

unter Bildung eines Pyranderivats der Formel I

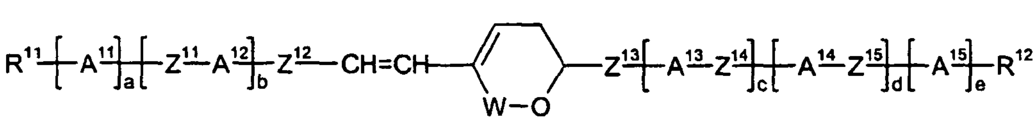

I

in Gegenwart eines Metathese-Katalysators umfasst, wobei a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ in den Formeln I, I-C, IIIa und IIIb die gleiche Bedeutung wie für Formel I oben haben;

mit der Maßgabe, dass für den Fall der direkten Verknüpfung von $Z^{13}$ und $R^{12}$ zu -$Z^{13}$-$R^{12}$ $R^{12}$ H, Aralkyl oder Alkanyl bedeutet, wenn $Z^{13}$ -C(=O)-bedeutet, $R^{12}$ Aralkyl oder Alkanyl bedeutet, wenn $Z^{13}$ -C(=O)-O- bedeutet, und $Z^{13}$ nicht -$CH_2O$- oder -$CF_2O$- bedeutet; dass für den Fall der direkten Verknüpfung von $Z^{14}$ und $R^{12}$ zu -$Z^{14}$-$R^{12}$ $R^{12}$ H, Aralkyl oder Alkanyl bedeutet, wenn $Z^{14}$ -C(=O)- bedeutet, $R^{12}$ Aralkyl oder Alkanyl bedeutet, wenn $Z^{14}$ -C(=O)-O- bedeutet, und $Z^{14}$ nicht -$CH_2O$- oder -$CF_2O$- bedeutet; dass für den Fall der direkten Verknüpfung von $Z^{15}$ und $R^{12}$ zu -$Z^{15}$-$R^{12}$ $R^{12}$ H, Aralkyl oder Alkanyl bedeutet, wenn $Z^{15}$ -C(=O)- bedeutet, $R^{12}$ Aralkyl oder Alkanyl bedeutet, wenn $Z^{15}$ -C(=O)-O- bedeutet, und $Z^{15}$ nicht -$CH_2O$- oder -$CF_2O$- bedeutet.

Im allgemeinen wird das Pyranderivat der Formel I überwiegend oder ausschließlich als E-Isomer bezüglich der exocylischen Doppelbindung erhalten.

Das Alken der Formel IIIb weist an der zentralen C=C-Doppelbindung trans-Konfiguration auf. In der erfindungsgemäßen Metathese-Reaktion setzt 1 Mol-Äquivalent dieses trans-Alkens unter Bruch der zentralen C=C-Doppelbindung 2 Äquivalente eines reaktiven Intermediats (das auch als "$R^{11}$-[-$A^{11}$-]$_a$-[-$Z^{11}$-$A^{12}$-]$_b$-$Z^{12}$-CH=" umschrieben werden kann) frei, die dann mit 2 Äquivalenten der Verbindung der Formel I-C abreagieren. Das bedeutet, dass bei Verwendung eines Alkens der Formel IIIb in dem erfindungsgemäßen Verfahren pro einem Mol des Pyranderivats der Formel I-C nur 0,5 Mol des Alkens der Formel IIIb eingesetzt werden müssen.

Im Zusammenhang mit der vorliegenden Erfindung umfaßt der Ausdruck "Alken der Formel III" beziehungsweise"Verbindung der Formel III" - soweit nicht im Einzelfall etwas anderes angegeben ist - sowohl Alkene der Formel IIIa als auch Alkene der Formel IIIb.

Dieses zweite erfindungsgemäße Verfahren erlaubt unter Anwendung der oben in allgemeiner Form erläuterten Kreuz-Metathese (CM) ebenfalls die Synthese von Pyranderivaten mit exocyclischer C=C-Doppelbindung und 2,5-Disubstitution, welche vorteilhafte technische Merkmale wie mesogene Eigenschaften aufweisen und/oder als Ausgangsverbindungen für die Herstellung weiterer, zum Beispiel mesogener Verbindungen mit einem zentralen Pyranring dienen können.

In einer besonders bevorzugten Ausführungsform der erfindungsgemäßen Verfahren wird zunächst in einer Enin-Metathese-Reaktion eine Verbindung der Formel I-C hergestellt, welche anschließend mittels erfindungsgemäßer Kreuz-Metathese in die entsprechende erfindungsgemäße Verbindung der Formel I überführt wird (wobei es bevorzugt ist, dass $R^{11}$ in Formel III und Formel I dann nicht H ist):

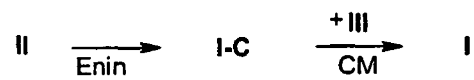


**Schema 3**

Diese erfindungsgemäße Reaktionssequenz kann als "Eintopf"-Synthese ausgeführt werden, d.h. das Enin-Metathese-Produkt I-C wird nicht isoliert, sondern direkt mit dem Alken III und gegebenenfalls weiterem Metathese-Katalysator versetzt. Ferner ist es möglich, bereits der Ausgangsreaktionsmischung aus dem Enin der Formel II und dem Metathese-Katalysator das Alken der Formel III zuzusetzen, ohne dass in nennenswertem Maße die Bildung der erfindungsgemäßen Verbindung der Formel I via I-C beeinträchtigt wird; offenbar reagiert das Enin der Formel II unter den gewählten Metathese-Bedingungen signifikant schneller intramolekular unter Bildung von I-C als intermolekular mit dem Alken 111. Im allgemeinen wird die Reaktionssequenz vorzugsweise unter Isolierung und gegebenenfalls Aufreinigung des Pyranderivats der Formel I-C vor der weiteren Umsetzung durchgeführt.

Als Metathese-Katalysator für die Kreuz-Metathese finden die gleichen Oxo-Wolfram- beziehungsweise Übergangsmetall-Alkyliden-Komplexe Verwendung, die für die oben beschriebene Enin-Metathese eingesetzt werden. Besonders bevorzugt wird für den Kreuz-Metathese-Schritt des zweiten erfindungsgemäßen Verfahrens ein Ruthenium-Alkyliden-Komplex, ganz besonders bevorzugt ausgewählt aus der Gruppe bestehend aus Komplexen der Formeln COMP-Ru1, -Ru2, -Ru3, -Ru4, -Ru5, -Ru6, -Ru7,- Ru8, -Ru9, -Ru10, -Ru11, -Ru12, -Ru13, insbesondere der Formeln COMP-Ru2a, COMP-Ru3, COMP-Ru4, COMP-Ru5a, COMP-Ru6 und COMP-Ru13 verwendet. Üblicherweise beträgt der Gehalt an Katalysator von 0,01 bis 10 mol-% (bezogen auf Verbindung I-C), bevorzugt 0,1 bis 5 mol-%, insbesondere 0,5 mol-% bis 2,5 mol-%.

Die Kreuz-Metathese wird unter für solche Metallkomplex-katalysierten Reaktionen üblichen Bedingungen ausgeführt. Die Reaktion erfolgt lösungsmittelfrei oder in einem geeigneten Solvens. Als Lösungsmittel wird ein inertes Lösungsmittel, bevorzugt ein aromatisches und/oder halogeniertes organisches Lösungsmittel und insbesondere Toluol, Benzol, Chlorbenzol oder Dichlormethan verwendet. Die Reaktion erfolgt in der Regel bei Temperaturen von Raumtemperatur bis Siedetemperatur des Lösungsmittels (z.B. ca. 20 ˚C bis 40 ˚C (Dichlormethan) beziehungsweise bis 80 ˚C oder 110 ˚C (Toluol)). Sie kann auch lösungsmittelfrei sowie gegebenenfalls in einem Autoklaven unter erhöhtem Druck, zum Beispiel 2 bis 10 bar, ausgeführt werden. Die Reaktionszeit ist an sich nicht kritisch, da die Kreuz-Metathese im allgemeinen unter vollständiger Umsetzung des Edukts der Formel I-C abläuft und das gewünschte Pyranderivat der Formel I in guten Ausbeuten ergibt. Üblicherweise beträgt die Reaktionsdauer von 30 min bis 8 Tagen, bevorzugt von 1 h bis 3 Tagen, insbesondere von 4 h bis 24 h, wobei die genaue Reaktionszeit vom Fachmann in Abhängigkeit vom jeweiligen Katalysator, seiner Menge und Konzentration und vom Verlauf der Umsetzung ausgewählt werden kann.

Die in der Kreuz-Metathese-Reaktion des erfindungsgemäßen Verfahrens eingesetzten Alkene der Formel III sind entweder käuflich erhältlich oder werden nach an sich bekannten Methoden dargestellt, wie sie in der Literatur (zum Beispiel den Standardwerken wie Houben-Weyl, Methoden der Organischen Chemie, Georg-Thieme-Verlag, Stuttgart) beschrieben sind, und zwar unter Reaktionsbedingungen, die für die dort genannten Umsetzungen bekannt und geeignet sind. Dabei kann man auch von an sich bekannten, hier nicht näher erwähnten Varianten Gebrauch machen.

Im Hinblick auf die erfindungsgemäßen Verbindungen der Formel I, bei denen es sich um Lactone handelt (Verbindungen der Formel I-A), wird der Enin-Metathese-Schritt der erfindungsgemäßen Verfahren ausgehend von den entsprechenden Propiolsäureestern II-A ausgeführt. Diese sind gemäß Schema 4 durch Veresterung eines käuflich erhältlichen aktivierten Propiolsäurederivats V-I (mit beispielsweise X = Methoxy, Ethoxy, Propoxy, t-Butoxy, Chlor, Brom oder Anhydrid-Rest, insbesondere Acetoxy) mit einem entsprechenden Homoallylalkohol V-II (dessen Herstellung unten in Schema 5 dargestellt ist) zugänglich. Dieser Verfahrensschritt verläuft unter für derartige Esterbildungsreaktionen üblichen Bedingungen (vgl. u.a. J. March: Advanced Organic Chemistry; John Wiley & Sons, New York u.a., 3. Aufl., 1985, S. 346-351, 0-22, 0-23 und 0-24).

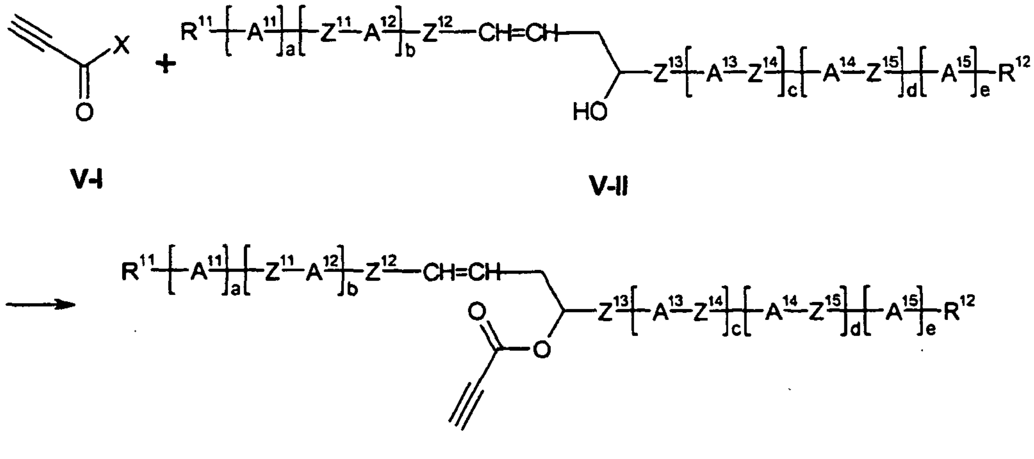

V-I

V-II

II-A

**Schema 4**

wobei a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ die gleiche Bedeutung wie in Formel I oben besitzen und X wie oben definiert ist und bevorzugt Brom oder Methoxy bedeutet.

Die Homoallylalkohole der Formel V-II und die Propiolsäureester der Formel II-A sind sie ihrerseits als nützliche Zwischenverbindungen zur Synthese der erfindungsgemäßen Verbindungen der Formel I jeweils ein Gegenstand der vorliegenden Erfindung.

Lactone der Formel 1-A mit a = b = 0, $Z^{11}$ = Einfachbindung und $R^{11}$ = H (welche auch durch die Formel I-CA dargestellt werden können), können, sofern gewünscht, in dem Kreuz-Metathese-Schritt des zweiten erfindungsgemäßen Verfahrens mit entsprechenden Alkenen der Formel III zu den korrespondierenden Pyranderivaten der Formel I-A umgesetzt werden, wobei die so erhaltenen Reaktionsprodukte dann einen von H verschiedenen Substituenten an der exocyclischen Doppelbindung in Formel I-A aufweisen, wie dies beispielsweise bei Verbindungen der Formel I-DAA der Fall ist.

Ferner ist es bei Auswahl geeigneter Reste, Ringe beziehungsweise funktioneller Gruppen für $R^{12}$, $A^{13}$, $A^{14}$, $A^{15}$, $Z^{13}$, $Z^{14}$ beziehungsweise $Z^{15}$ möglich, die "rechte" Seitenkette des Pyranderivats I-A ($-Z^{13}-[A^{13}-Z^{14}]_c-[A^{14}-Z^{15}]_d-[A^{15}]_e-R^{12}$) ganz oder teilweise nach der ringschließenden Enin-Metathese in das jeweilige Molekül einzuführen. Hierbei stehen grundsätzlich die gleichen synthetischen Methoden zur Verfügung, die auch zur weiteren Derivatisierung der erfindungsgemäßen Pyrane der Formel I-B angewendet werden und weiter unten detaillierter beschrieben sind, wobei der Fachmann die auf Grund der Lactonfunktion des zentralen O-Heterocyclus gegebenenfalls erforderlichen oder empfehlenswerten Anpassungen ohne weiteres vornehmen kann.

Für Verbindungen der Formel I-A (beziehungsweise der Formel I mit W = - C(=O)-) ist es ferner bevorzugt, dass in den erfindungsgemäßen Verfahren nach dem jeweiligen Metathese-Reaktionsschritt als ein weiterer Reaktionsschritt eine Reduktionsreaktion zur Überführung des Pyranderviats der Formel I, worin W -C(=O)- bedeutet und $Z^{13}$, $Z^{14}$ und $Z^{15}$ nicht -C(O)- bedeuten, in ein Pyranderivat der Formel I, worin W eine Methylen-Gruppe ist (d.h. ein Pyran der Formel I-B), ausgeführt wird. Diese Reduktionsreaktion kann mit geeigneten Reduktionsmitteln, zum Beispiel Diisobutylaluminiumhydrid (DIBAH) oder Bortrifluorid-Etherat + Lithiumaluminumhydrid oder Lithiumborhydrid oder Natriumborhydrid (siehe J. March: Advanced Organic Chemistry; John Wiley & Sons, New York u.a., 3. Aufl., 1985, S. 1100, 9-41), ausgeführt werden. Dabei werden auch andere im Molekül vorhandene Carboxy-Funktionen (C(=O)O) zu Ether-Funktionen ($CH_2O$) reduziert.

Die für die erfindungsgemäße Herstellung jener erfindungsgemäßen Verbindungen der Formel I, bei denen es sich um Pyrane handelt (Verbindungen der Formel I-B), erforderlichen Ausgangsverbindungen und Vorstufen sind auf verschiedenen synthetischen Wegen zugänglich, sofern sie nicht auch kommerziell erhältlich sind. Zum Teil werden Verbindungen der Formel I-B durch chemische Modifizierung anderer Verbindungen der Formel I-B oder - wie oben dargelegt - durch Reduktion von Verbindungen der Formel I-A hergestellt.

So sind die Ausgangsverbindungen der Formel II mit W = $-CH_2-$ (= Formel II-B), die ihrerseits als nützliche Zwischenverbindungen zur Herstellung der erfindungsgemäßen Verbindungen der Formel I-B Gegenstand der vorliegenden Erfindung sind, im allgemeinen von Verbindungen der Formel V-III ausgehend gemäß Schema 5 zugänglich. Anstelle des Allylbromids V-III können andere Allylhalogenide, insbesondere Allylchloride, eingesetzt werden.

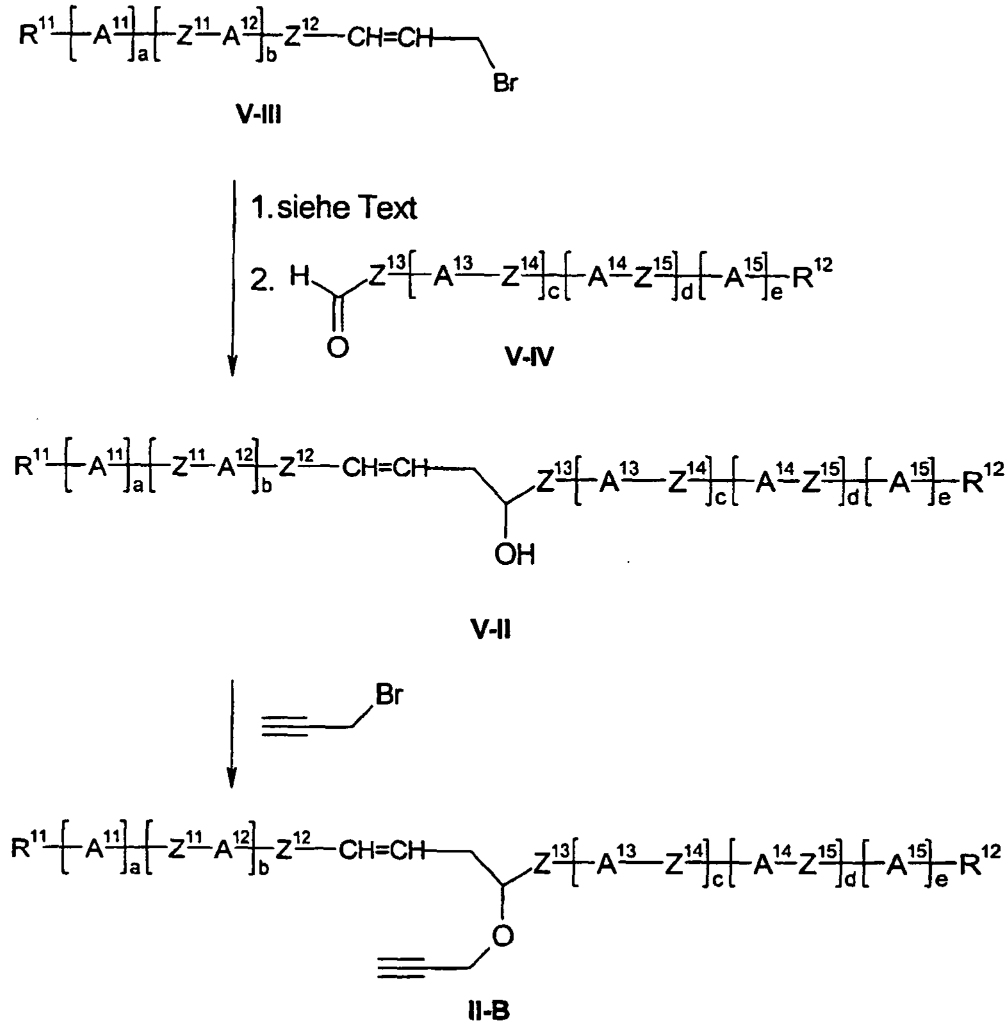


**Schema 5**

Dabei kann die erforderliche Aktivierung des Allylbromids V-III (beziehungsweise entsprechender Allylhalogenide) in Reaktionsschritt 1 auf unterschiedliche Weise erfolgen. Beispielsweise wird durch Zugabe von V-III zu Indium-Pulver in einem geeigneten Reaktionsmedium, zum Beispiel Wasser oder Wasser/Tetrahydrofuran, zunächst eine entsprechende intermediäre Indium-Allylverbindung (nach T.-P. Loh et al., Tetrahedron Letters 42 (2001) 8701 und 8705) gebildet, die in Schritt 2 mit dem Aldehyd V-IV unter Bildung des korrespondierenden Homoallylalkohols V-II reagiert. Das Allylbromid V-III kann auch mit Lithium oder einer Lithium-organischen Base oder mit Magnesium, zumeist im Überschuß, oder einer reaktiven Grignard-Base unter Halogen-Metall-Austausch in die entsprechende intermediäre Lithium- beziehungsweise Magnesiumbromid-Allylverbindung überführt werden, die dann mit dem Aldehyd V-IV zu dem Homoallylalkohol V-II reagiert.

Enthält der Aldehyd V-IV zusätzlich eine funktionelle Gruppe, die wie die aldehydische Carbonylfunktion mit der Allyl-Grignard- beziehungsweise Allyl-Lithium-Verbindung reagieren kann, zum Beispiel eine Esterfunktion oder eine Nitrilfunktion, so ist es vorteilhaft, die intermediäre Lithiumbeziehungsweise Magnesiumbromid-Allylverbindung in bekannter Weise mit Zink- oder Titansalzen umzumetallieren, da die entsprechenden Zinkbeziehungsweise Titan-Allylverbindungen chemoselektiv nur mit der aldehydischen Carbonyl-Funktion und nicht mit der Ester-Carboxybeziehungsweise der CN-Funktion reagieren.

Ferner können auch Allylderivate anderer Metalle und Halbmetalle, zum Beispiel Allylderivate von Chrom, Zinn, Zink, Samarium, Bor und Silicium, zur Herstellung der erfindungsgemäßen Homoallylverbindungen V-II eingesetzt werden. Ausgehend von Allylmesylaten, die anstelle von Brom in Formel V-III einen Mesylatrest ($-OSO_2CH_3$) tragen, sind mittels Transmetallierung mit z.B. $LiSn(butyl)_3$ die entsprechenden Allyl-Stannane zugänglich, die auch durch Umsetzung des Allylbromids V-III mit Zinn(II)chlorid und Kaliumiodid in Wasser (vgl. V.V. Samoshin et al., Tetrahedron Lett. 43 (2002) 6329) oder mit Zinnmetall unter Einwirken von Ultraschall und Wasser (vgl. P. C. Andrews, Tetrahedron Lett. 43 (2002) 7541) hergestellt und mit dem Aldehyd V-IV zum Homoallylalkohol V-II umgesetzt werden können. Entsprechend sind unter anderem mit Zinkstaub in Tetrahydrofuran Zinkallylverbindungen (vgl. B. C. Ranu et al., Tetrahedron Lett. 36 (1995), 4885), mit $SmI_2$ in Tetrahydrofuran Samariumallylverbindungen (vgl. B. Hamann-Gaudinet et al., Tetrahedron Lett. 38 (1997) 6585) beziehungsweise mit $Cr(II)Cl_2/Mn$ Chromallylverbindungen erhältlich, die mit einem Aldehyd V-IV zu Homoallylalkoholen V-II umgesetzt werden können.

Der Homoallylalkohol V-II wird dann mit Propargylbromid in das Enin II-B überführt. Der Etherbildungs-Reaktionsschritt erfolgt im allgemeinen unter Bedingungen der Williamson-Ether-Synthese (s. z.B. J. March: Advanced Organic Chemistry; John Wiley & Sons, New York u.a., 3. Aufl., 1985, S. 342, 0-14), d.h. unter basischen Reaktionsbedingungen und bei Temperaturen von Raumtemperatur bis ca. 60 ˚C in geeigneten Lösungsmitteln, z.B. Ethern wie Tetrahydrofuran (THF), Methyl-tert.-butylether (MTBE), Dioxan oder Dimethoxyethan. Eine besonders bevorzugte Variante dieser Reaktion arbeitet mit granuliertem NaOH in THF in Gegenwart eines Phasentransferkatalysators und von wenig Wasser, wobei das Propargylbromid und der Homoallylalkohol der Formel V-II für 4 bis 48 h auf 40 ˚C bis 60 ˚C erwärmt werden. Diese Variante eignet sich besonders für diejenigen Homoallylalkohole der Formel V-II, die keinen Carboxylfunktion aufweisen (also Homoallylalkohole der Formel V-II, in denen $Z^{13}$, $Z^{14}$ und $Z^{15}$ nicht $CO_2$ bedeuten). Alternativ kann als Base Natriumhydrid, bevorzugt in einem organischen Lösunsmittel, eingesetzt werden.

Das so gebildete Enin der Formel II-B wird dann - nach gegebenenfalls erforderlicher Reinigung - in der Enin-Metathese-Reaktion des ersten erfindungsgemäßen Verfahrens in die erfindungsgemäße Verbindung der Formel I-B (= Formel I mit W = $-CH_2-$) überführt.

Wird in der Synthese gemäß Schema 5 als Ausgangsverbindung V-III käuflich erhältliches Allylbromid eingesetzt (d.h. a = b = 0, $Z^{12}$ = Einfachbindung und $R^{11}$ = H in Formel V-III), so werden über das entsprechende Enin der Formel II-B mittels Enin-Metathese-Reaktion schließlich die erfindungsgemäßen Verbindungen der Formel I-C erhalten. Diese können dann im zweiten erfindungsgemäßen Verfahren mit einem Alken der Formel III via Kreuz-Metathese-Reaktion zu den entsprechenden Verbindungen der Formel umgesetzt werden. Diese (konvergente) Vorgehensweise kann sich im Vergleich zum ersten erfindungsgemäßen Verfahren (mit linearer Synthesestrategie) unter Umständen als vorteilhaft erweisen, wenn beispielsweise das Alken III leichter zugänglich ist als das substituierte Allylbromid V-III.

Da die Aldehyde der Formel V-IV am Carbonyl-Kohlenstoffatom ein prochirales Zentrum aufweisen, wird bei der Umsetzung mit dem aus der Verbindung der Formel V-III gebildeten aktivierten Allylderivat zu dem Homoallylalkohol V-II ein Chiralitätszentrum am die Hydroxyfunktion tragenden Kohlenstoffatom gebildet. Im allgemeinen entsteht dabei ein Racemat der optischen Antipoden des Homoallylalkohols V-II. Es ist jedoch auch möglich, eines der optischen Isomeren des Homoallylalkohols V-II stereoselektiv herzustellen oder alternativ aus der racemischen Mischung zu isolieren.

Die stereoselektive Synthese erfolgt bevorzugt durch katalytische asymmetrische Allylierung des Aldehyds der Formel V-IV mit einer von Verbindung V-III abgeleiteten Allylzinnverbindung, zumeist dem entsprechenden Allyltributylstannan, in Gegenwart eines chiralen Katalysators. Als chirale Katalysatoren eignen sich insbesondere Komplexe chiraler Binaphtol- (BINOL-)Verbindungen mit Zirkonium (z.B. (*R*,*R*)- oder (*S*,*S*)-BINOL-Zr(O=tert.-Butyl)$_4$: M. Kurosu et al., Tetrahedron Lett. 43 (2002) 1765) beziehungsweise Titan (z.B. Bis(((*S*)(napthoxy)(isopropxy)-titanoxid: H. Hanawa et al., J. Am. Chem. Soc. **2003,** *125*, 1708) oder entsprechende Boronate (vgl. z.B. S. Thormeier et al., J. Organomet. Chem. 657 (2002) 136). Grundsätzlich ist so - je nach Wahl des chiralen Katalysators - das R- oder das S-Isomere selektiv zugänglich.

Die Isolierung der Enantiomeren aus der racemischen Mischung erfolgt nach üblichen Verfahren, zum Beispiel mittels Kristallisation mit einer chiralen Base oder Chromatographie an einem chiralen Säulenmaterial.

Durch Verwendung eines so zugänglichen enantiomerenreinen Homoallylalkohols der Formel V-II wird über die Zwischenstufe II-A beziehungsweise II-B nach der erfindungsgemäßen Enin-Metathese und ggf. der erfindungsgemäßen Kreuzmetathese-Reaktion das entsprechende Pyranderivat der Formel I, das in 2-Position ein asymmetrisches Zentrum aufweist, in enantiomerenreiner Form erhalten. Eine weitere Möglichkeit, einen der optischen Antipoden des Pyranderivats der Formel I stereoselektiv zu erhalten, besteht in der Verwendung eines chiralen Metathese-Katalysators,

z.B. COMP-Mo3 oder COMP-Mo4, die von G. S. Weatherhead et al., Tetrahedron Lett. 41 (2000) 9553, beschrieben und für stereoselektive ringschließende Metathesereaktionen eingesetzt worden sind.

Die Verbindungen der Formel V-III sind, soweit sie nicht kommerziell erhältlich sind, ausgehend von den entsprechenden Aldehyden der Formel

$R^{11}$-$[A^{11}]_a$-$[Z^{11}$-$A^{12}]_b$-$Z^{12}$-CHO V-V

zugänglich: In einer Wittig-Horner-Reaktion (s. z.B. J. March: Advanced Organic Chemistry; John Wiley & Sons, New York u.a., 3. Aufl., 1985, S. 845, 6-47) mit beispielsweise $(EthylO)_2P(=O)CH_2CO_2$Ethyl erfolgt die Überführung in den Ester $R^{11}$-$[A^{11}]_a$-$[Z^{11}$-$A^{12}]_b$-$Z^{12}$-CH=CH-$CH_2CO_2$Ethyl, der nach Reduktion mit z.B. DIBAH und Bromierung des so hergestellten Allylalkohols mit beispielsweise Brom/Triphenylphosphan das gewünschte Allylbromid V-III ergibt.

Aus dem Aldehyd der Formel V-V ist mittels Wittig-Reaktion (s. z.B. J. March: Advanced Organic Chemistry; John Wiley & Sons, New York u.a., 3. Aufl., 1985, S. 845, 6-47) auch das entsprechende Alken der Formel III erhältlich.

Die Aldehyde der Formel V-IV sind als solche literaturbekannt (siehe z.B. EP 0 122 389 A2). Die Synthese von Aldehyden der Formel V-IV mit $Z^{13}$ = $CF_2O$ erfolgt dabei zum Beispiel ausgehend von dem Säurechlorid

EthylO-C(=O)-C(=O)-Cl V-VI

durch Umsetzung mit zunächst NaS-$(CH_2)_3$-SH. Der dabei entstehende Thiolthioester wird mit Trifluormethansulfonsäure (in Analogie zu den Verfahren gemäß P. Kirsch et al., Angew. Chem. **2001**, *113*, 1528, und WO 01/64667) zu dem entsprechenden Bis(alkylthio)carbenium-Salz

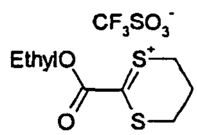

V-VII

umgesetzt, das dann einer oxidativen Fluordesulfurierung (gemäß P. Kirsch et al., Angew. Chem. **2001**, *113*, 1528, und WO 01/64667) unterworfen wird, indem das Bis(alkylthio)carbenium-Salz der Formel V-VII zunächst bei tiefen Temperaturen mit $NEt_3 \cdot 3$ HF (Et = Ethyl) und einem Alkohol der Formel

HO-$[A^{13}$-$Z^{14}]_c$-$[A^{14}$-$Z^{15}]_d$-$[A^{15}]_e$-$R^{12}$ V-VIII,

dann mit 1,3-Dibrom-5,5-dimethylhydanthoin (DBH) oder N-Bromsuccinimid (NBS) oder Brom und schließlich mit wäßriger Lauge zum Ester

Ethyl-O-C(=O)-$CF_2O$-$[A^{13}$-$Z^{14}]_c$-$[A^{14}$-$Z^{15}]_d$-$[A^{15}]_e$-$R^{12}$ V-IX

umgesetzt wird. Die abschließende Einführung der Aldehyd-Funktion unter Ergeben des Aldehyds der Formel V-IV erfolgt entweder durch direkte Reduktion des Esters mit einem geeigneten Reduktionsmittel wie Diisobutylaluminiumhydrid in einem inerten Solvens, zum Beispiel n-Heptan, oder über die Reduktion des Esters zum entsprechenden Alkohol und anschließende Oxidation zum Aldehyd mit einem geeigneten Oxidationsmittel, zum Beispiel mit Dess-Martin-Reagenz.

Eine alternative Syntheseroute, die sich vor allem für die Darstellung von Aldehyden der Formel V-IV eignet, in denen die Difluoroxymethylen-Brücke mit einem aromatischen Rest verknüpft ist, geht von dem Ester Ethyl-O-C(=O)-$CF_2$Br aus, der mit einem geeigneten Phenolat in Gegenwart von beispielsweise Hexamethylenphosphortribromid oder unter $Pd^0$-Komplex-Katalyse unter Bildung der $CF_2O$-Brücke und nach abschließender Reduktion der Esterfunktion in den gewünschten Aldehyd V-IV überführt wird.

Sofern es sich bei dem Aldehyd der Formel V-IV um ein Phenanthrenderivat der Formel

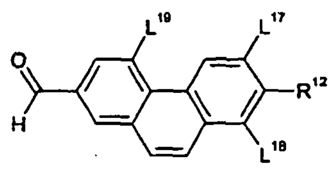

handelt, so ist dieses nach folgendem Schema 6 zugänglich:

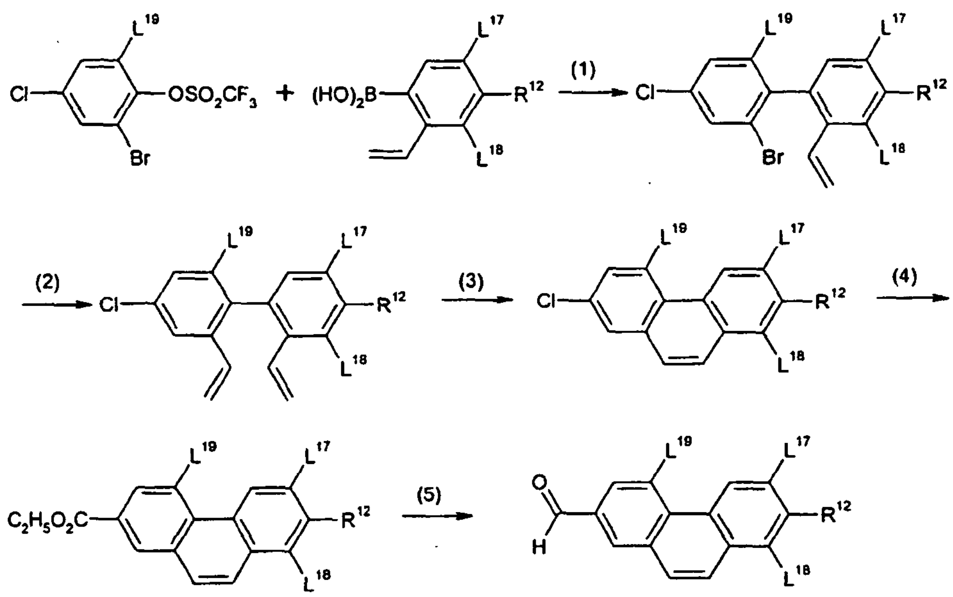


**Schema 6**

In Schritt (1) erfolgt eine C-C-Kupplung unter $Pd^0$-Katalyse zum Biphenyl, das in Schritt (2) mit Vinylmagnesiumbromid oder Vinylzinkbromid in Gegenwart eines Palladium-Komplexes ($PdCl_2$·dppf) in das Divinylderivat überführt wird. In Schritt (3) erfolgt eine intramolekulare Kreuzmetathese zum Phenanthrenderivat in Gegenwart eines Ruthenium-Alkyliden-Komplexes COMP-RuA, bevorzugt COMP-Ru2a-c oder COMP-Ru4. Das Chlorphenanthren wird anschließend in Schritt (4) mit CO/Ethanol bei 70 °C und 5 bar in Gegenwart von $PdCl_2$·[2P(Cyclohexyl)$_3$] als Katalysator in den Ethylester überführt, der nach abschließender Reduktion mit DIBAH in Schritt (5) den gewünschten Phenanthrenaldehyd ergibt. Zur Herstellung des Phenanthrenaldehyds der Formel

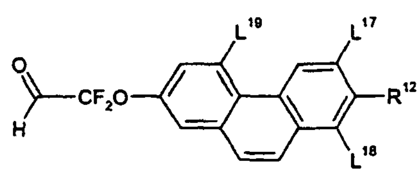

setzt man in Schritt (1) von Schema 6 anstelle der Chlor-substituierten Verbindung die korrespondierende Benzyloxyverbindung

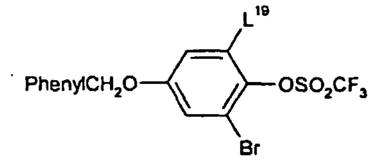

ein, die entsprechend in den Schritten (1) bis (4) in die korrespondierende Benzyloxy-substituierte Phenanthrenverbindung überführt wird. Nach reduktiver Abspaltung der Benzyloxyschutzgruppe (mit Wasserstoff und Pd-C) wird das resultierende Hydroxyphenanthren wie oben dargelegt mit Ethyl-O-C(=O)-$CF_2$Br und nach abschließender Reduktion der Esterfunktion in den gewünschten Aldehyd überführt. Die Ausgangsverbindungen für diese Phenanthrensynthesen sind entweder kommerziell oder nach bekannten Synthesemethoden leicht zugänglich.

Es ist selbstverständlich, dass zum einen Vorstufen und Ausgangsverbindungen der Formeln II-B und III für erfindungsgemäße Verbindungen der Formel I-B aus anderen geeigneten Vorstufen und Ausgangsverbindungen der Formeln II-B und III hergestellt werden können, und zum anderen auch erfindungsgemäße Verbindungen der Formel I-B nach erfolgtem Ringschluss in andere erfindungsgemäße Pyranderivate der Formel I-B umgewandelt werden können. Als besonders nützlich haben sich dabei Carbonsäurederivate erwiesen.

Wird zum Beispiel in der Synthese gemäß Schema 5 als Aldehyd V-IV Glyoxalethylester eingesetzt, so erhält man das Enin der Formel II-B mit $Z^{13}$-$R^{12}$ = $CO_2$Ethyl. Dieser Enin-Carbonsäurethylester kann nun zunächst einer Enin-Metathese-Reaktion unter Bildung des entsprechenden Pyranderivats der Formel I-B (beziehungsweise - in Abhängigkeit von der Bedeutung von $R^{11}$, $A^{11}$, $A^{12}$, $Z^{11}$, $Z^{12}$, a und b - der Formeln I-CBIIa oder I-JB) unterworfen werden. Durch Umesterung des Esters I-JB mit Synthesebausteinen des Typs HO-$[A^{13}$-$Z^{14}]_c$-$[A^{14}$-$Z^{15}]_d$-$[A^{15}]_e$-$R^{12}$ können dann andere erfindungsgemäße Verbindungen der Formel I-B erhalten werden. Eine entsprechende Umesterung kann aber auch, falls gewünscht, vor der Enin-Metathese-Reaktion mit der Vorstufe II-B ausgeführt werden.

Wird mit dem Glyoxalsäureester Allylbromid als Allylverbindung V-III umgesetzt, ergibt sich als weiterer Syntheseweg neben der Umesterung vor der Enin-Metathese beziehungsweise nach der Enin-Metathese zu dem Ester der Formel I-CBIIa die Kreuz-Metathese-Reaktion von I-CBIIa mit einem Alken der Formel III, gegebenenfalls gefolgt von einer weiteren Veresterung.

Die erfindungsgemäßen Carbonsäuren der Formeln I-CBIIa-1 und I-JB mit $R^{12}$ = H, die aus den entsprechenden Estern durch basische oder saure Verseifung dargestellt werden, können eingesetzt werden, um über das korrespondierende Bis(alkythio)carbeniumsalz und anschließende oxidative Fluordesulfurierung (vgl. WO 01/64667) das entsprechende $CF_2O$ verbrückte Pyranderivat der Formel I-B (z.B. Verbindungen der Formeln I-CBIII, I-DAB und I-HB) zu erhalten. Die oxidative Fluordesulfurierung ist natürlich auch bei solchen erfindungsgemäßen Verbindungen der Formel I-B anwendbar, bei denen die Carboxyl-Funktion nicht direkt mit dem zentralen Pyranring verknüpft ist, d.h. wenn $Z^{14}$ oder $Z^{15}$ = -$CO_2$H bedeutet; auf diese Weise werden dann -$CF_2$O-$[A^{14}$-$Z^{15}]_d$-$[A^{15}]_e$-$R^{12}$- beziehungsweise -$CF_2$O-$A^{15}$-$R^{12}$-Reste eingeführt.

Aus den erfindungsgemäßen Carbonsäureestern der Formeln I-CBIIa und I-JB mit $R^{12} \neq$ H sind entweder durch direkte Reduktion beispielsweise mit einem geeigneten Metallhydrid oder zweistufig durch Reduktion zum primären Alkohol und anschließende schonende Oxidation mit beispielsweise Dess-Martin-Reagenz erfindungsgemäße Aldehyde (Formel I-B mit $Z^{13}$-$R^{12}$ = -C(=O)-H; z.B. I-CBIVa-1) zugänglich.

Aus dem so erhaltenen Aldehyd I-CBIVa-1 sind beispielsweise mittels Wittig- oder Wittig-Horner-Reaktion mit Molekülen des Typs $R^x$-$[A^{13}$-$Z^{14}]_c$-$[A^{14}$-$Z^{15}]_d$-$[A^{15}]_e$-$R^{12}$ (wobei $R^x$ beispielsweise für $(Phenyl)_3$P=CH- oder $(EthylO)_2$P(=O)-$CH_2$- steht) die entsprechenden Verbindungen der Formel I-CBV (mit $Z^{13}$ = -CH=CH-) zugänglich. Entsprechend können auch die erfindungsgemäßen Verbindungen der Formel I-B mit $Z^{14}$-$R^{12}$ oder $Z^{15}$-$R^{12}$ = -CHO hergestellt und in solche mit $Z^{14}$ oder $Z^{15}$ = -CH=CH- umgewandelt werden.

Die Herstellung von erfindungsgemäßen Ketonen beispielsweise der Formel I-CBIVa erfolgt z.B. durch Umsetzung des Carbonsäureesters I-CBIIa-3 mit einem geeigneten metallorganischen Reagenz, zum Beispiel mit einer Verbindung $R^{12}$-Mg-Br nach Grignard (siehe z.B. J. March: Advanced Organic Chemistry, John Wiley & Sons, New York u.a., 3. Aufl., 1985, S. 434, 0-107). Analog sind auch die weiteren Verbindungen der Formel I-CBIV durch Umsetzung mit einem geeigneten metallorganischen Reagenz Met*-$[A^{13}$-$Z^{14}]_c$-$[A^{14}$-$Z^{15}]_d$-$[A^{15}]_e$-$R^{12}$ (wobei "Met*" zum Beispiel Br-Mg oder Li bedeutet) zugänglich. Sofern $Z^{14}$ und $Z^{15}$ nicht $CO_2$ bedeuten und $R^{12}$ keine Carboxylfunktion enthält, sind die Ketone der Formel I-CBIV durch Reduktion der entsprechenden Ester der Formel I-CBII erhältlich. Analog können Keto-Funktionen auch als $Z^{14}$ oder $Z^{15}$ eingeführt werden.

Auch erfindungsgemäße Verbindungen der Formel I-RB sind gut für weitere Derivatisierungen geeignet. Durch Umsetzung mit beispielsweise Trimethylsilyl-1,3-dithian in THF in Gegenwart von n-Butyllithium (in Anlehnung an das Verfahren von J. Mtynarski und A. Banaszek, Tetrahedron **55** (1999) 2785) oder analog zu den Verfahren von P. Kirsch et al., Angew. Chem. **2001**, *113*, 1528, sind die entsprechenden Ketendithioketale (mit dem Strukturbestandteil

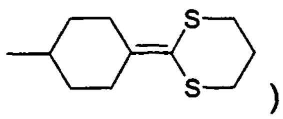

zugänglich. Oxidative Fluordesulfurierung (nach P. Kirsch et al., Angew.

Chem. **2001**, *113*, 1528) ergibt dann erfindungsgemäße Verbindungen der Fomel I-B mit

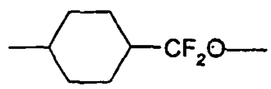

als Strukturbestandteil.

Die erfindungsgemäßen Cyclohexanone der Formel I-RB können auch mit einem geeigneten Grignard-Reagenz, zum Beispiel des Typs Br-Mg-$Z^{14}$-$A^{14}$-$R^{12}$ mit $Z^{14}$ beispielsweise -$CH_2$- oder -$CH_2CH_2$-, zu den entsprechenden tertiären Alkoholen, in diesem Beispiel mit dem Strukturelement

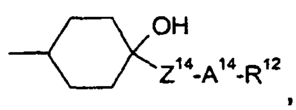

umgesetzt werden. Anschließende Reduktion des Alkohols mit Triethylsilan und Bortrifluorid-Etherat ergibt das korrespondierende 1,4-disubstiuerte Cyclohexan-Derivat (im angegebenen

Beispiel mit dem Strukturelement

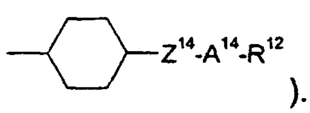

Erfindungsgemäße Verbindungen der Formel I, die einen endständigen Phenylring mit $R^{12}$ = Halogen, insbesondere Brom oder lod, aufweisen, zum Beispiel entsprechende Verbindungen der Formeln I-DABIa, I-DBBIa oder I-K, können ebenfalls als Ausgangsverbindungen zur Herstellung weiterer erfindungsgemäßer Verbindungen der Formel I eingesetzt werden. So kann - nach Metallierung unter Metall-Halogen-Austausch beispielsweise mit einer metallorganischen Base wie n-Butyllithium - die intermediäre metallierte Verbindung mit verschiedenen Reagenzien weiter umgesetzt werden, so z.B. mit $CO_2$ unter Bildung der korrespondierenden Carbonsäure, mit Borsäureestern oder verwandten Borverbindungen unter Bildung der korrespondierenden Aryl-Bor-Verbindungen, oder in Gegenwart geeigneter Katalysatoren mit Reaktionspartnern, die C-C-Kreuzkupplungsreaktionen beispielsweise vom Typ der Heck- oder der Suzuki-Reaktion eingehen.

Auch die genannten Aryl-Bor-Verbindungen oder die halogenierten Verbindungen selbst können in derartigen literaturbekannten Kreuzkupplungsreaktionen (siehe z.B. N. Miyaura, A. Suzuki, Chem. Rev. **1995**, 95, 2457) umgesetzt werden. Ferner sei angemerkt, dass auch bestimmte Vorstufen und Ausgangsverbindungen der erfindungsgemäßen Verfahren, zum Beispiel solche, in denen ein aromatischer Ring $A^{13}$ direkt mit einem aromatischen Ring $A^{14}$ (beziehungsweise $A^{15}$) über $Z^{14}$ (beziehungsweise $Z^{15}$) = Einfachbindung verknüpft ist, mit Hilfe dieser Kreuzkupplungsreaktionen hergestellt werden können.

Weiter ist es bevorzugt, in den erfindungsgemäßen Verfahren nach dem/den Metathese-Reaktionsschritt/en sowie dem gegebenenfalls durchzuführenden Reduktionsschritt vom Lacton der Formel I-A zu dem korrespondierenden Dihydropyran der allgemeinen Formel I-B als einen weiteren Reaktionsschritt eine katalytische Hydrierung zu einem Pyran der allgemeinen Formel IV durchzuführen:

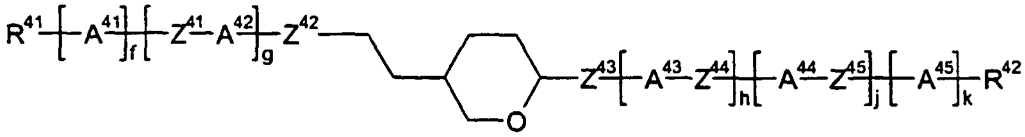

IV

worin

f, g, h, j und k jeweils unabhängig voneinander 0 oder 1 sind;

$R^{41}$ H, einen unsubstituierten oder einfach oder mehrfach gleich oder verschieden mit Halogen oder -CN substituierten gesättigten Alkylrest mit 1 bis 15 Kohlenstoffatomen bedeutet, wobei in diesem Rest auch eine oder mehrere $CH_2$-Gruppen durch -O-, -S-, -C(O)-O- und/oder -O-C(O)- so ersetzt sein können, dass Heteroatome (O, S) nicht direkt miteinander verknüpft sind;

$R^{42}$ H, Halogen, -CN, -NCS, Aralkyl, -O-Aralkyl oder einen unsubstituierten oder einfach oder mehrfach gleich oder verschieden mit Halogen oder -CN substituierten gesättigten Alkylrest mit 1 bis 15 C-Atomen bedeutet, wobei in diesem Rest auch eine oder mehrere $CH_2$-Gruppen durch -O-, -S-, -C (O)-O- und/ oder -O- C (O)- so ersetzt sein können, dass Heteroatome (O, S) nicht direkt miteinander verknüpft sind;

$Z^{41}$ und $Z^{42}$ unabhängig voneinander eine Einfachbindung, $-CH_2-$, $-CH_2CH_2-$, $-CF_2CH_2-$, $-CH_2CF_2-$ oder $-CF_2CF_2-$ bedeuten;

$Z^{43}$, $Z^{44}$ und $Z^{45}$ jeweils unabhängig voneinander eine Einfachbindung, $-CH_2CH_2-$, $-CF_2CH_2-$, $-CH_2CF_2-$, $-CF_2CF_2-$, $-CH_2O-$,$CF_2O-$ -C(O)- oder -C(O)-O- bedeuten;

$A^{41}$ und $A^{42}$ unabhängig voneinander für

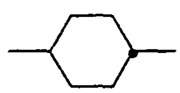

oder

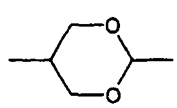

stehen;

$A^{43}$ und $A^{44}$ unabhängig voneinander für

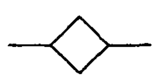

,

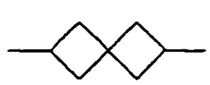

,

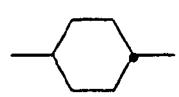

,

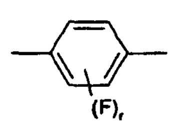

,

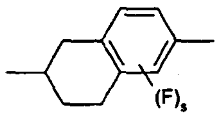 oder 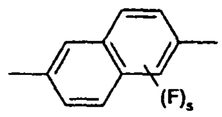

stehen;

$A^{45}$ für

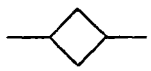, 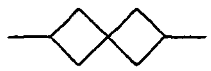, 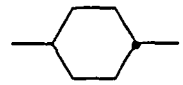,

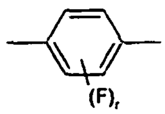, 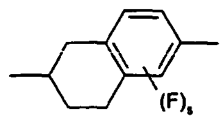 oder 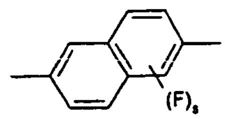

steht

oder

$A^{45}$-$R^{42}$ zusammen für

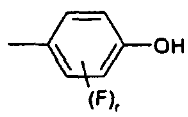, 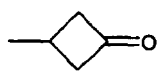, 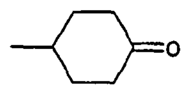

oder

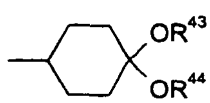

steht;

oder

$Z^{43}$-[-$A^{43}$-$Z^{44}$-]$_h$-[-$A^{44}$-$Z^{45}$-]$_j$-[-$A^{45}$-]$_k$-$R^{42}$ für

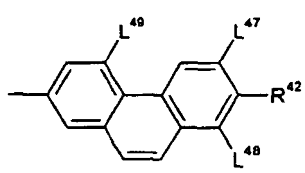

oder

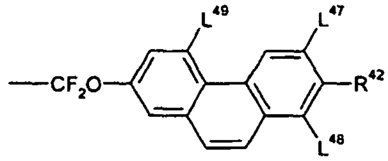

steht, wobei $R^{42}$ wie oben definiert ist und $L^{47}$, $L^{48}$ und $L^{49}$ unabhängig voneinander H oder F bedeuten;

r 0, 1, 2, 3 oder 4 ist;

s 0, 1, 2 oder 3 ist;

$R^{43}$ und $R^{44}$ unabhängig voneinander einen Alkanylrest mit 1 bis 7 Kohlenstoffatomen bedeutet oder zusammen für eine Alkylenbrücke mit 2 bis 7 Kohlenstoffatomen stehen;

mit der Maßgabe,
dass für den Fall der direkten Verknüpfung von $Z^{43}$ und $R^{42}$ zu $-Z^{43}-R^{42}$ $R^{42}$ H, Aralkyl oder Alkanyl bedeutet, wenn $Z^{43}$ -C(=O)- bedeutet, $R^{42}$ Aralkyl oder Alkanyl bedeutet, wenn $Z^{43}$ -C(=O)-O- bedeutet, und $Z^{43}$ nicht $-CH_2O-$ oder $-CF_2O-$ bedeutet;
dass für den Fall der direkten Verknüpfung von $Z^{44}$ und $R^{42}$ zu $-Z^{44}-R^{42}$ $R^{42}$ H, Aralkyl oder Alkanyl bedeutet, wenn $Z^{44}$ -C(=O)- bedeutet, $R^{42}$ Aralkyl oder Alkanyl bedeutet, wenn $Z^{44}$ -C(=O)-O- bedeutet, und $Z^{44}$ nicht $-CH_2O-$ oder $-CF_2O-$ bedeutet;
dass für den Fall der direkten Verknüpfung von $Z^{45}$ und $R^{42}$ zu $-Z^{45}-R^{42}$ $R^{42}$ H, Aralkyl oder Alkanyl bedeutet, wenn $Z^{45}$ -C(=O)- bedeutet, $R^{42}$ Aralkyl oder Alkanyl bedeutet, wenn $Z^{45}$ -C(=O)-O- bedeutet, und $Z^{45}$ nicht $-CH_2O-$ oder $-CF_2O-$ bedeutet.

Verbindungen der Formel IV sind vorzugsweise mesogen und insbesondere flüssigkristallin.

Als Ausgangsverbindungen zur Herstellung eines Pyrans in der allgemeinen Formel IV können grundsätzlich alle erfindungsgemäßen Verbindungen der Formel I mit W = $-CH_2-$ (d. h. Verbindungen der Formel I-B) eingesetzt werden. Dabei werden neben der (endocyclischen) C=C-Doppelbindung in dem zentralen Pyranring und der (exocyclischen) C=C-Doppelbindung am zentralen Pyranring auch weitere gegebenenfalls vorhandene aliphatische C=C-Doppelbindungen der Verbindung der Formel zu einer C-C-Einfachbindung hydriert. So kann beispielsweise ausgehend von einer Verbindung der Formel I-B durch die katalytische Hydrierung die entsprechende Verbindung der Formel IV-B erhalten werden, wobei die Bedeutung von f, g, h, j, k, $R^{41}$, $R^{42}$, $Z^{41}$, $Z^{42}$, $Z^{43}$, $Z^{44}$, $Z^{45}$, $A^{41}$, $A^{42}$, $A^{43}$, $A^{44}$ und $A^{45}$ der Bedeutung von a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ beziehungsweise $A^{15}$ entspricht unter der Maßgabe, dass, wenn $Z^{11}$, $Z^{12}$, $Z^{13}$, $Z^{14}$ und/oder $Z^{15}$ -CH=CH- bedeuten beziehungsweise $R^{11}$ und/oder $R^{12}$ eine -CH=CH-Gruppe enthalten, diese aliphatischen -CH=CH-Gruppen in $CH_2$-$CH_2$-Gruppen überführt werden. Analog werden aus den erfindungsgemäßen Verbindungen der Formel 1-CB die entsprechenden hydrierten Verbindungen IV-CB, aus I-DAB IV-DAB, aus I-DBB IV-DBB, aus I-DCB IV-DCB, aus I-EB IV-EB, aus I-FB IV-FB, aus I-GB IV-GB, aus I-HB IV-HB, aus I-JB IV-JB, aus I-KB IV-KB, aus I-LB IV-LB, aus I-MB IV-MB, aus I-NB IV-NB, aus I-OB IV-OB, aus I-PB IV-PB, aus I-QB IV-QB, aus I-RB IV-RB und aus I-SB IV-SB erhalten.

Die katalytische Hydrierung erfolgt üblicherweise bei einem Wasserstoff-Partialdruck von 1 bar bis 10 bar. Als Hydrierungskatalysatoren werden üblicherweise Übergangsmetall-Katalysatoren aus Nickel, Platin oder Palladium, wie zum Beispiel Raney-Nickel, 5% oder 10% Platin auf Kohlenstoff und 5% Palladium auf Aktivkohle, in einem geeigneten, Lösungsmittel, wie zum Beispiel n-Heptan, Toluol, Ethylacetat, Ethanol, Methanol oder THF, eingesetzt. Die Reaktionszeit ist an sich nicht kritisch; üblicherweise wird die Hydrierung bis zur vollständigen Umsetzung der jeweiligen Ausgangsverbindung ausgeführt. Die Reaktionstemperatur liegt im allgemeinen im Bereich zwischen Raumtemperatur und 100 ˚C.

Bei der Hydrierung des Pyranderivats I zu dem Pyranderivat IV wird zusätzlich zu dem Asymmetriezentrum in 2-Position des Pyranrings ein weiteres chirales Zentrum in 5-Position gebildet. Sofern als Ausgangsverbindung für die katalytische Hydrierung zur Bildung des Tetrahydropyrans IV ein solches Pyranderivat der Formel I eingesetzt wird, das nach entsprechender, oben beschriebener stereoselektiver Synthese oder Aufreinigung in enantiomerenreiner (oder enantiomerenangereicherter) Form vorliegt, werden üblicherweise nur 2 der 4 theoretisch denkbaren Diastereomeren von IV erhalten (in Abhängigkeit von der absoluten Konfiguration am C-2-Atom mit 2R,5R- und 2R,5S- oder mit 2S,5R- und 2S,5S- Konfiguration). Diese beiden Isomeren, die sich zueinander als Diastereomeren verhalten, können mittels herkömmlicher Verfahren wie fraktionierter Kristallisation oder Chromatographie voneinander getrennt werden, so dass das Tetrahydropyran der Formel IV auch in enantiomerenreiner Form erhalten werden kann. Die isomerenreinen Verbindungen der Formel IV finden ebenso wie die isomerenreinen Verbindungen der Formel I unter anderem Verwendung

als chirale Dotierstoffe für nematische flüssigkristalline Medien, die die für verschiedene elektrooptische Anwendungen erforderliche verdrillte Anordnung der in den flüssigkristallinen Medien enthaltenen Verbindungen bewirken.

Selbstverständlich können Verbindungen der Formel IV ihrerseits in andere Verbindungen der Formel IV überführt werden, und zwar mittels der gleichen synthetischen Verfahren, die oben insbesondere für die Verbindungen der Formel I-B ausführlich erläutert worden sind. Wie auch im Falle der erfindungsgemäßen Verbindungen der Formel I-B, die eine Carboxylfunktion aufweisen (zum Beispiel I-CBIIa), eignen sich hierfür besonders Carbonsäureester der Formel IV, insbesondere solche der Formeln IV-CBIla und IV-JB. Sie können beispielsweise zur entsprechenden freien Carbonsäure verseift, durch Hydrierung debenzyliert oder Palladium-katalysiert desallyliert und dann weiter derivatisiert werden, um zum Beispiel die Einführung einer Difluoroxymethylenbrücke zu ermöglichen. Unter vielen anderen Derivatisierungsmöglichkeiten soll hier auch die Möglichkeit erwähnt werden, den Ester der Formel IV-CBIIa (oder der Formel IV-JB) zum entsprechenden Aldehyd zu reduzieren, welcher seinerseits via Wittig- oder Wittig-Horner-Reaktion unter Einführung einer aliphatischen C=C-Doppelbindung umgesetzt werden kann.

Ferner sind Verbindungen der Formel IV-RB nicht nur direkt durch Hydrierung von entsprechenden Verbindungen der Formel 1-RB zugänglich. Sie können zum Beispiel auch in der Weise hergestellt werden, dass ein Pyranderivat der Formel 1-B mit einem endständigem Phenylring mit $R^{12}$ = 0-Aralkyl, wie z.B. Verbindungen der Formel I-CBIa oder I-DCBIa mit $R^{12}$ = 0-Aralkyl, zunächst unter gleichzeitiger Debenzylierung zu der korrespondierenden Verbindung der Formel IV-B mit endständigem Phenolring hydriert wird, um sie anschließend mit geeigneten Reduktionsmitteln (siehe z.B. J. March: Advanced Organic Chemistry; John Wiley & Sons, New York u.a., 3. Aufl., 1985, S. 700, 5-11) in die entsprechende Verbindung der Formel IV-RB mit endständigem Cyclohexanonring zu überführen. Für die beispielhaften Verbindungen wird dieses Vorgehen durch Schema 7 wiedergegeben. Die Verbindungen der Formel IV-RB sind insbesondere für die Einführung von $CF_2O$-Brücken geeignet, deren Einzelheiten oben für die Umsetzung der ebenfalls erfindungsgemäßen Cyclohexanonderivate der Formel I-RB beschrieben sind.

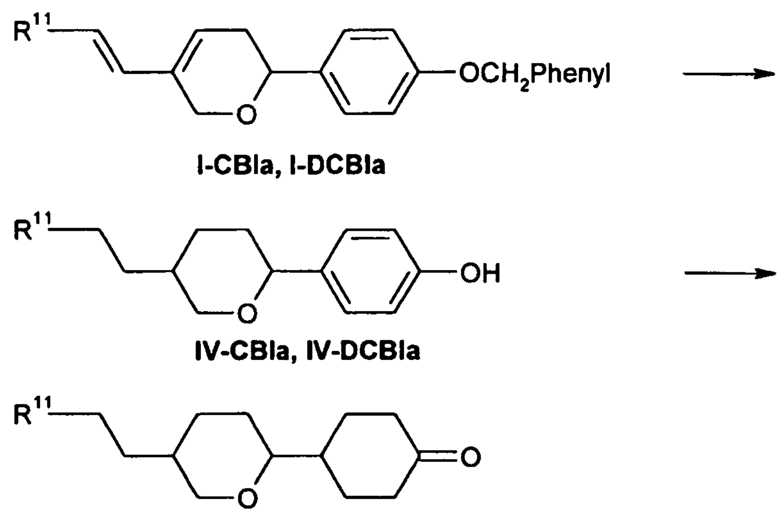

**I-CBIa, I-DCBIa**

**IV-CBIa, IV-DCBIa**

**IV-RBa**

## Schema 7

Aufgrund der 2,5-Disubstitution des zentralen Pyranrings kann die Verbindung der Formel IV sowohl als cis-als auch als trans-Isomer vorliegen. Mitunter wird das im allgemeinen für viele Verwendungen bevorzugte trans-Isomer als einziges Produkt der Hydrierung erhalten. Fällt das Pyran der Formel IV vorwiegend als cis-Isomer oder als Gemisch beider Isomeren an, so wird das bevorzugte trans-Isomer durch Behandlung mit einer starken Base, zum Beispiel Kalium-tert.-butylat in N-Methylpyrrolidon, oder mit einer starken Säure, zum Beispiel Schwefelsäure in Dioxan, aus dem cis-Isomeren erhalten.

Die vorliegende Erfindung betrifft ferner die Verwendung eines Pyranderivats der obigen Formel I und bevorzugt der Formel I-B als Bestandteil eines flüssigkristallinen Mediums, das insbesondere in elektrooptischen Anzeigevorrichtungen wie TN-, STN- und Aktiv-Matrix-Displays eingesetzt wird. Bei dieser elektrooptischen Anzeigevorrichtung handelt es sich beispielsweise um Displays von Mobilfunkgeräten, Bildschirme von portablen Computern (Notebooks) und TFT-Flachbildschirme.

Vor- und nachstehend bedeuten Prozentangaben Gewichtsprozent. Alle Temperaturen sind in Grad Celsius angegeben. Fp. bedeutet Schmelzpunkt, Klp. Klärpunkt. Ferner bedeuten K beziehungsweise C = kristalliner Zustand,

N = nematische Phase, S = smektische Phase und I = isotrope Phase. $S_c$ bedeutet eine smektisch C Phase, $S_B$ eine smektisch B Phase, $S_A$ eine smektisch A Phase. $\Delta n$ bezeichnet die optische Anisotropie ($\Delta n = n_e - n_o$, wobei $n_e$ der Brechungsindex des außerordentlichen und $n_o$ der Brechungsindex des ordentlichen Strahls ist) (589 nm, 20 °C). $\Delta\varepsilon$ bezeichnet die dielektrische Anisotropie ($\Delta\varepsilon = \varepsilon_{\parallel} - \varepsilon_{\perp}$, wobei $\varepsilon_{\parallel}$ die Dielektrizitätskonstante parallel zu den Moleküllängsachsen und $\varepsilon_{\perp}$ die Dielektrizitätskonstante senkrecht dazu bedeutet) (1 kHz, 20°C). Die optischen Daten wurden bei 20 °C gemessen, sofern nicht ausdrücklich etwas anderes angegeben wird. Die Rotationsviskosität $\gamma 1$ [mPa.s] wurde ebenfalls bei 20 °C bestimmt. Zur experimentellen Bestimmung der physikalischen Parameter wurde gemäß "Licristal, Physical Properties Of Liquid Crystals, Description of the measurement methods", Hrsg. W. Becker, Merck KGaA, Darmstadt, überarbeitete Ausgabe, 1998, verfahren, wobei die Eigenschaften von Einzelverbindungen zum Teil nach Messung einer definierten Menge der Verbindung (zumeist 5 oder 10 Gew.-%) in einer definierten Host-Mischung mit bekannten Eigenschaften und anschließende Extrapolation ermittelt wurden.

Die beigefügten Beispiele veranschaulichen die vorliegende Erfindung weiter, ohne sie in irgendeiner Weise zu beschränken.

## Beispiele

Die in den beispielhaften Synthesen eingesetzten Ausgangsverbindungen, Reagenzien und Lösungsmittel wurden entweder käuflich erworben oder nach literaturbekannten Verfahren hergestellt. Die beispielhaften Synthesen wurden üblicherweise in trockenen Apparaturen unter Feuchtigkeitsausschluss und - sofern die betreffende Umsetzung dies erforderte - auch unter Schutzgasatmosphäre zum Luftausschluss ausgeführt.

Der Verlauf von Reaktionen wurde im allgemeinen mittels Dünnschichtchromatographie oder Gaschromatographie überwacht. Die Reaktionsprodukte wurden nach üblichen Verfahren aufgearbeitet und gereinigt, beispielsweise mittels Säulenchromatographie beziehungsweise Kristallisation. Ihre strukturelle Identität wurde mittels Massenspektrometrie und $^1$H-NMR-Spektroskopie gesichert. Ausbeuten sind nicht optimiert.

### Beispiel 1 - Enin-Metathese-Reaktion

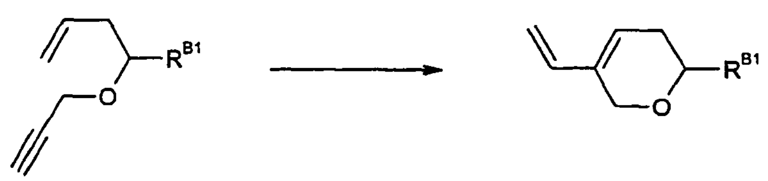

B 1.1 B 1.2

### Allgemeine Arbeitsvorschrift B1 (AAV-B1)

Ausgangsverbindung B1.1 (1 mol), gelöst in Dichlormethan, wird unter Stickstoff-Atmosphäre portionsweise mit Bis(tricyclohexylphosphin)-benzylidenrutheniumdichlorid (COMP-Ru4) (0,5 mol; von Strem Chemicals Inc., Kehl, Deutschland) bei Raumtemperatur versetzt und 72 h gerührt. Das Reaktionsgemisch wird konzentriert und einer Säulenchromatographie an Kieselgel unterworfen. Das Produkt B1.2 wird aus Heptan oder Ethanol umkristallisiert und mittels MS und $^1$H-NMR charakterisiert. Ausbeuten: 50-75%.

Nach AAV-B1 wurden folgende Verbindungen der Formel B1.2 mit den in Tabelle B1 genannten Bedeutungen für $R^{B1}$ hergestellt:

**Tabelle B1**

| Verbindung B1.2-Nr. | $R^{B1}$ | Verbindung B1.2-Nr. | $R^{B1}$ |
|---|---|---|---|
| -1 | 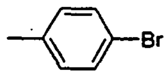 | -2 | 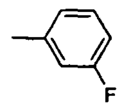 |
| -3 | 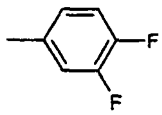 | -4 | 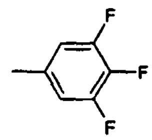 |
| -5 | 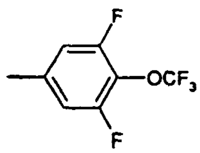 | -6 | 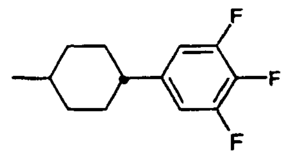 |
| -7 | 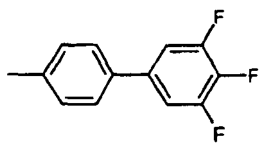 | -8 | 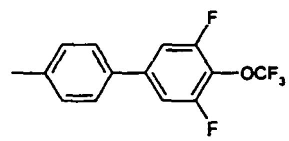 |
| -9 | 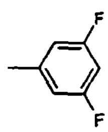 | -10 | 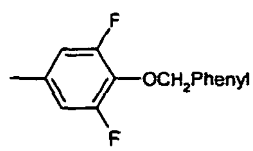 |
| -11 | 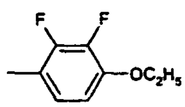 | -12 | 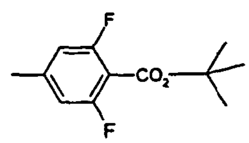 |
| -13 | 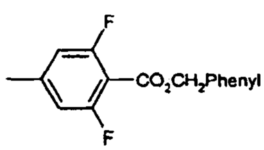 | -14 | 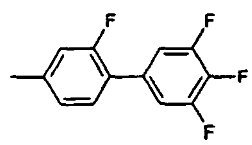 |
| -15 | 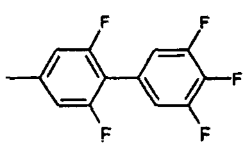 | -16 | 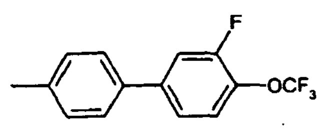 |
| -17 | 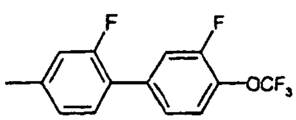 | -18 | 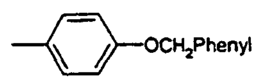 |

(fortgesetzt)

| Verbindung B1.2-Nr. | $R^{B1}$ | Verbindung B1.2-Nr. | $R^{B1}$ |
|---|---|---|---|
| -19 | 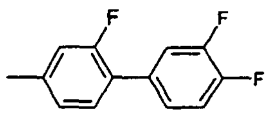 | -20 | 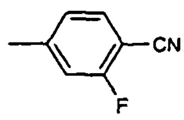 |
| -21 | 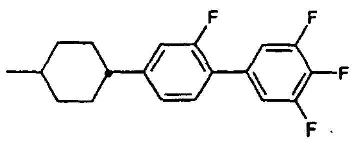 | -22 | 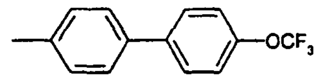 |
| -23 | 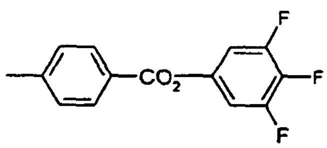 | | |

**Beispiel 2 - Kreuz-Metathese-Reaktion**

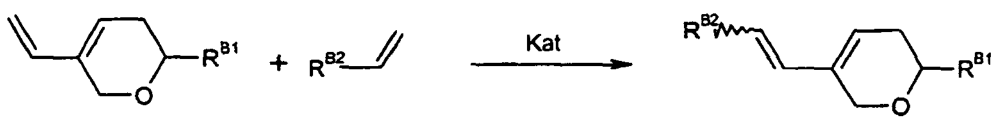


B 1.2 B 2.1 B 2.2

**Allgemeine Arbeitsvorschrift B2a (AAV-B2a)**

Ausgangsverbindung B1.2 (0,05 mol) und Tricyclohexylphospin(1,3-bis(2,4,6-trimethylphenyl)-4,5-dihydroimidazol-2-yliden-benzylidenrutheniumdichlorid (COMP-Ru2) (0,001 mol; von Strem Chemicals Inc., Kehl, Deutschland) werden in Dichlormethan gelöst und dann mit Prop-1-en (B2.1-1) bei ca. 5 bar 20 h gerührt. Das Reaktionsgemisch wird konzentriert und einer Säulenchromatographie an Kieselgel unterworfen. Das Produkt B2.2 wird als E-Isomer erhalten und aus Heptan oder Ethanol umkristallisiert und mittels MS und $^1$H-NMR charakterisiert. Ausbeuten: 45-75 %. Anstelle von Propen kann auch trans-But-2-en eingesetzt werden.

**Allgemeine Arbeitsvorschrift B2b (AAV-B2b)**

Ausgangsverbindung B1.2 (0,05 mol), das Alken B2.1 (0,05 mol) und Tricyclohexylphospin(1,3-bis(2,4,6-trimethylphenyl)-4,5-dihydroimidazol-2-yliden-benzylidenrutheniumdichlorid (COMP-Ru2) oder Bis(tricyclohexylphosphin)benzylidenrutheniumdichlorid (COMP-Ru4) (0,01 mol; von Strem Chemicals Inc., Kehl, Deutschland) werden in Toluol gelöst und 24 bis 48 h unter Rückfluss gerührt. Das Reaktionsgemisch wird konzentriert und einer Säulenchromatographie an Kieselgel unterworfen. Das Produkt B2.2 wird als E-Isomer erhalten und aus Heptan oder Ethanol umkristallisiert und mittels MS und $^1$H-NMR charakterisiert. Ausbeuten: 45-75 %. Anstelle des Alkens B 2.1 mit endständiger C=C-Doppelbindung kann auch das entsprechende trans-Alken

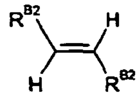

(0,5 Äquivalente, bezogen auf das Pyranderivat B 1.2) eingesetzt werden.

Nach AAV-B2a beziehungsweise AAV-B2b wurden die in Tabelle B2 genannten Verbindungen hergestellt:

**Tabelle B2**

| Verbindung B1.2-Nr. | Alken B2.1-Nr. | Katalysator | Produkt B2.2-Nr. | $R^{B1}$ | $R^{B2}$ |
|---|---|---|---|---|---|
| -1 | -1 | COMP-Ru2 | -1 | 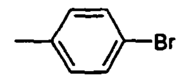 | $CH_3$- |
| -1 | -2 | COMP-Ru2 | -2 | 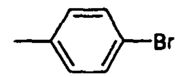 | n-$C_4H_9$- |
| -4 | -3 | COMP-Ru2 oder COMP-Ru4 | -3 | 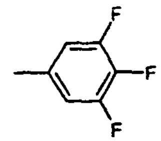 | 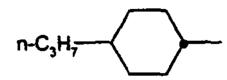 |
| -6 | -4 | COMP-Ru2 | -4 | 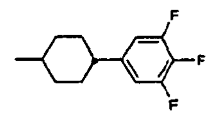 | n-$C_3H_7$ |
| -5 | -3 | COMP-Ru4 | -5 | 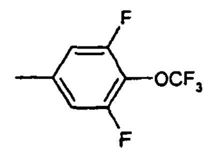 | 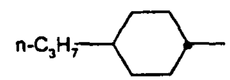 |
| -7 | -1 | COMP-Ru2 | -6 | 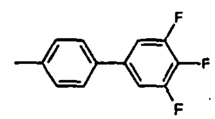 | $CH_3$ |
| -8 | -1 | COMP-Ru2 | -7 | 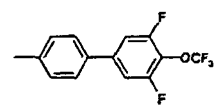 | $CH_3$ |

(fortgesetzt)

| Verbindung B1.2-Nr. | Alken B2.1-Nr. | Katalysator | Produkt B2.2-Nr. | $R^{B1}$ | $R^{B2}$ |
|---|---|---|---|---|---|
| -6 | -1 | COMP-Ru2 | -8 | 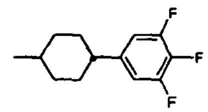 | $CH_3$ |
| -14 | -1 | COMP-Ru2 | -9 | 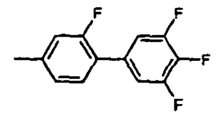 | $CH_3$ |
| -16 | -1 | COMP-Ru2 | -10 | 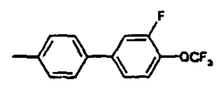 | $CH_3$ |
| B3.1-5 | -1 | COMP-Ru2 | -11 | 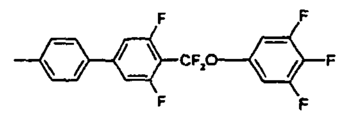 | $CH_3$ |
| -19 | -1 | COMP-Ru2 | -12 | 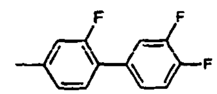 | $CH_3$ |
| -21 | -1 | COMP-Ru2 | -13 | 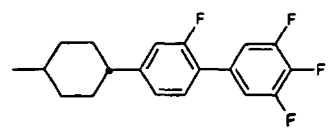 | $CH_3$ |
| -20 | -1 | COMP-Ru2 | -14 | 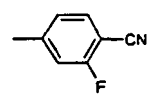 | $CH_3$ |

(fortgesetzt)

| Verbindung B1.2-Nr. | Alken B2.1-Nr. | Katalysator | Produkt B2.2-Nr. | $R^{B1}$ | $R^{B2}$ |
|---|---|---|---|---|---|
| analog B3.1-5 | -1 | COMP-Ru2 | -15 | 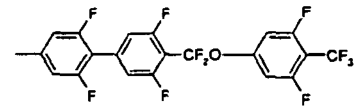 | $CH_3$ |
| analog B3.1-5 | -1 | COMP-Ru2 | -16 | 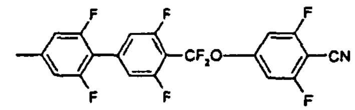 | $CH_3$ |
| analog B3.1-5 | -1 | COMP-Ru2 | -17 | 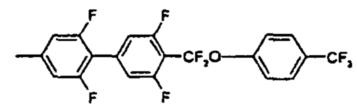 | $CH_3$ |
| analog B3.1-5 | -1 | COMP-Ru2 | -18 | 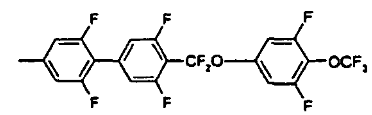 | $CH_3$ |
| analog B3.1-5 | -1 | COMP-Ru2 | -19 | 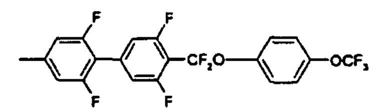 | $CH_3$ |

**Beispiel 3 - Derivatisierungen**

a)

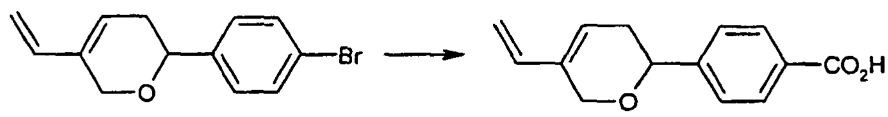

B 1.2-1

B 3.1-1

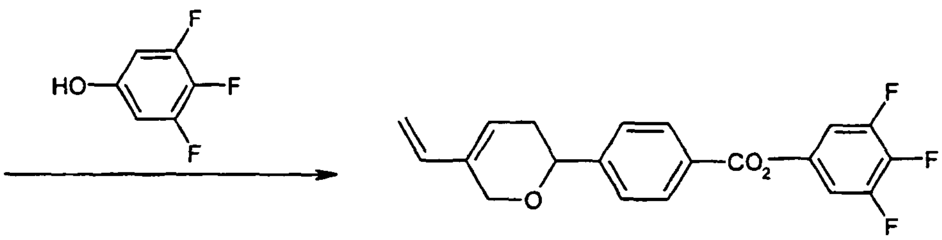

B 3.1-2

**Allgemeine Arbeitsvorschrift B3a (AAV-B3a)**

Verbindung B1.2-1 (0,3 mol) wird in THF gelöst und auf-78˚C gekühlt. Eine 15 %-ige Lösung von n-Butyllithium in n-Hexan (0,3 mol) wird zugetropft. Man rührt eine Stunde nach und gießt dann auf wasserfreies festes Kohlendioxid (ca. 6 mol) und arbeitet im Sauren wäßrig auf. Die organische Phase wird konzentriert und das Rohprodukt B3.1-2 aus Aceton umkristallisiert. Ausbeute: 62 %.

Zu einer Lösung von B3.1-1 (68 mmol), 3,4,5-Trifluorphenol (68 mmol) und 4,4-Dimethylaminopyridin (DMAP) (2 mmol) in Toluol wird unter Wasserbadkühlung N,N-Dicyclohexylcarbodiimid (76 mmol) zugetropft. Es wird bei Raumtemperatur für 18 h nachgerührt und dann mit Oxalsäuredihydrat (10 mmol) versetzt und 1 h gerührt. Nach Filtration wird das Reaktionsgemisch konzentriert und an Kieselgel chromatographiert. Das Rohprodukt B3.1-2 wird aus Aceton umkristallisiert. Ausbeute: 80 %.

b)

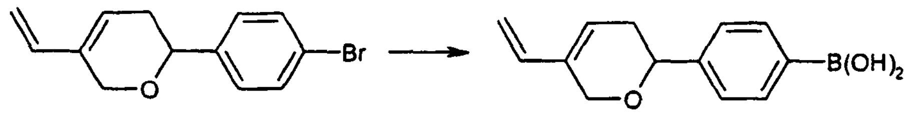

B 1.2-1 B 3.1-3

B 3.1-3 +

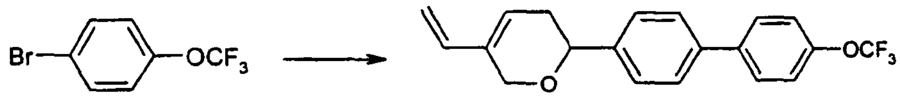

B 3.1-4

B 3.1-3 +

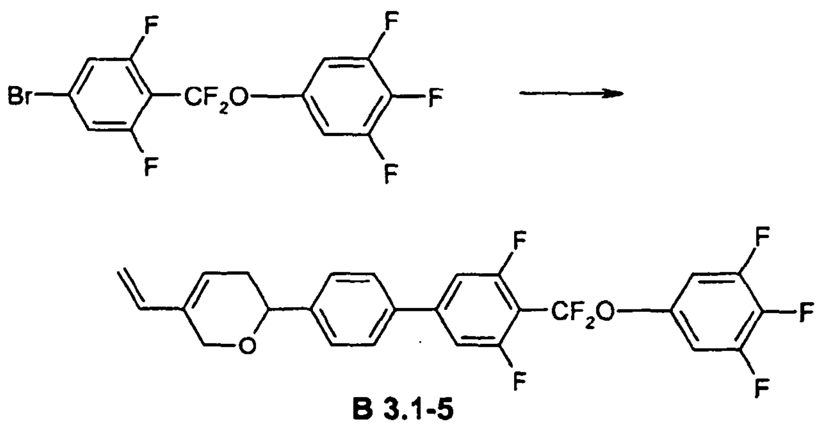

B 3.1-5

**Allgemeine Arbeitsvorschrift B3b (AAV-B3b)**

Magnesium-Späne (0,3 mol) in siedendem THF werden mit Bromethan (15 mmol) und einigen Tropfen einer Lösung von Verbindung B1.2-1 in THF versetzt; anschließend wird die restliche Lösung von Verbindung B1.2-1 (insgesamt 0,3 mol) in THF unter Rückfluß zugetropft. Nach Ende der Zugabe und 1 h Nachrühren wird diese Reaktionslösung bei -10 °C zu einer Lösung von Trimethylborat (0,36 mmol) in Heptan zugetropft. Es wird mit Salzsäure auf pH 5 eingestellt und wäßrig aufgearbeitet. Nach Trocknen und Einengen des Rohprodukts B3.1-3 wird aus Heptan umkristallisiert. Ausbeute: 82-96 %.

Eine Lösung aus Natriummetaborat-octahydrat (75 mmol), Bis(triphenylphosphin)-palladium(II)-chlorid (2 mmol) und Hydraziniumhydroxid (2 mmol) in Wasser/THF wird hergestellt. Hierzu werden Verbindung B3.1-3 (100 mmol) und das Arylbromid (100 mmol) hinzugegeben und für 3 h zum Sieden erhitzt. Nach wäßriger Aufarbeitung und Chromatographie an Kieselgel wird Verbindung B3.1-4 (30 % Ausbeute) beziehungsweise B3.1-5 (35 % Ausbeute) erhalten. In Analogie hierzu sind weitere Verbindungen mit abweichendem Substitutionsmuster an den Phenylringen zugänglich, deren Struktur sich unter anderem aus Tabelle B2 ergibt.

**Beispiel 4 - Hydrierungen**

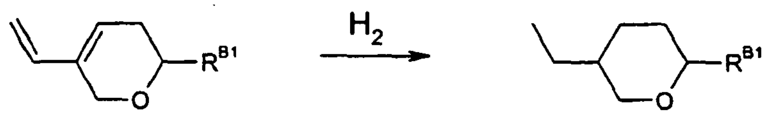


**B 1.2, B 3.1** **B 4.1**

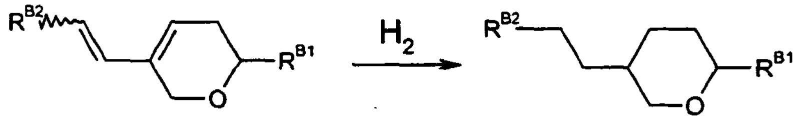


**B 2.2** **B 4.2**

**Allgemeine Arbeitsvorschrift B4 (AAV-B4)**

Die Verbindungen B1.2, B3.1 beziehungsweise B2.2 werden in Gegenwart von 5 % Platin auf Kohlenstoff oder 5 % Palladium auf Kohlenstoff (ca. 10 Gew.-%, bezogen auf die zu hydrierende Verbindung) in Heptan oder Tetrahydrofuran bei einem Wasserstoff-Druck von 1 bis 10 bar zu den entsprechenden Verbindungen B4.1 beziehungsweise B4.2 hydriert. Nach üblicher Aufarbeitung wird durch Säulenchromatographie, Destillation beziehungsweise Umkristallisation und gegebenenfalls nach Isomerisierung mit 30 mol-% Kalium-tert.-butylat in N-Methylpyrrolidon unter Kühlen und Rühren und nachfolgender Neutralisation das gewünschte trans-Pyran-Isomer erhalten. Die Tabellen B3 und B4 geben die entsprechenden Verbindungen wieder.

**Tabelle B3**

| Produkt B4.1-Nr. | $R^{B1}$ | Produkt B4.1-Nr. | $R^{B1}$ |
|---|---|---|---|
| -1 | 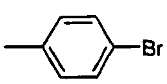 | -2 | 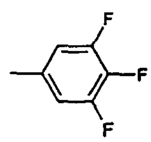 |
| -3 | 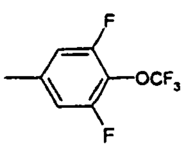 | -4 | 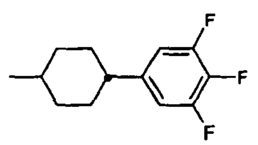 |
| -5 | 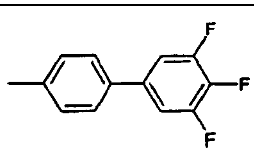 | -6 | 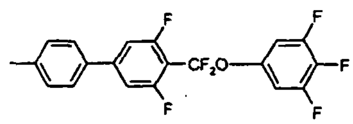 |
| -7 | 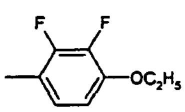 | -8 | 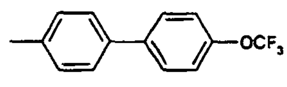 |
| -9 | 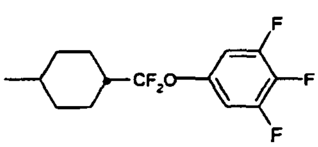 | -10 | 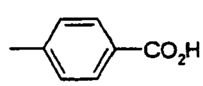 |

(fortgesetzt)

| Produkt B4.1-Nr. | $R^{B1}$ | Produkt B4.1-Nr. | $R^{B1}$ |
|---|---|---|---|
| -11 | 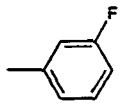 | -12 | 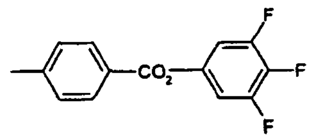 |
| -13 | 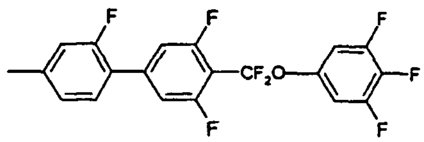 | -14 | 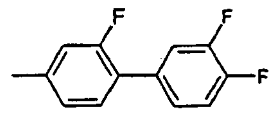 |
| -15 | 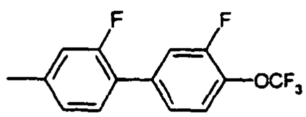 | -16 | 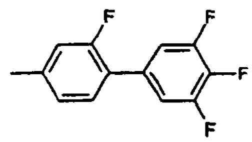 |

**Tabelle B4**

| Produkt B4.2-Nr. | $R^{B1}$ | $R^{B2}$ |
|---|---|---|
| -1 | 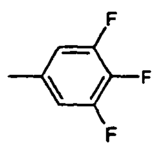 | 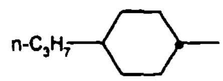 |
| -2 | 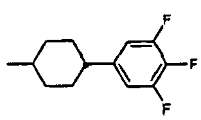 | $n\text{-}C_3H_7$ |
| -3 | 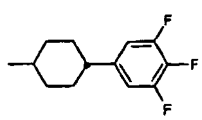 | $n\text{-}C_4H_9$ |
| -4 | 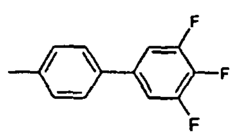 | $CH_3$ |
| -5 | 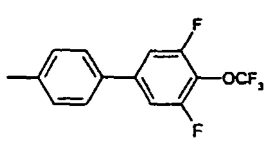 | $CH_3$ |
| -6 | 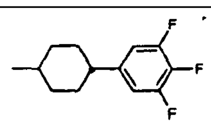 | $CH_3$ |

(fortgesetzt)

| Produkt B4.2-Nr. | $R^{B1}$ | $R^{B2}$ |
|---|---|---|
| -7 | 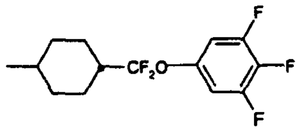 | $CH_3$ |
| -8 | 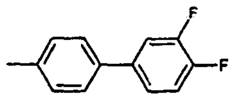 | $CH_3$ |
| -9 | 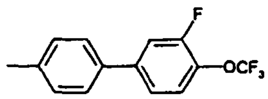 | $CH_3$ |
| -10 | 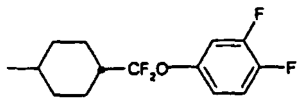 | $CH_3$ |
| -11 | 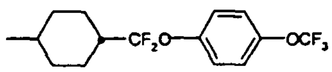 | $CH_3$ |
| -12 | 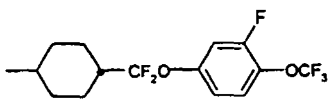 | $CH_3$ |
| -13 | 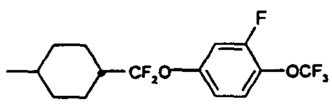 | $C_2H_5$ |
| -14 | 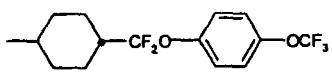 | $C_2H_5$ |
| -15 | 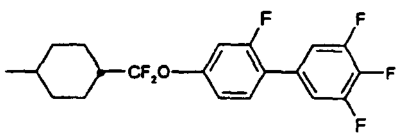 | $CH_3$ |
| -16 | 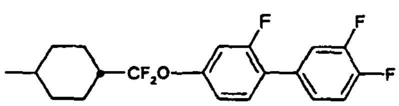 | $CH_3$ |
| -17 | 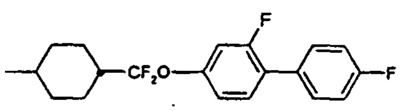 | $CH_3$ |
| -18 | 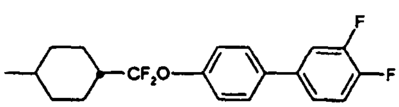 | $CH_3$ |

(fortgesetzt)

| Produkt B4.2-Nr. | $R^{B1}$ | $R^{B2}$ |
|---|---|---|
| -19 | 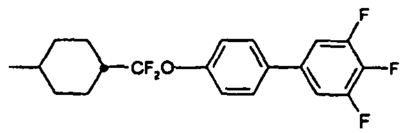 | $CH_3$ |
| -20 | 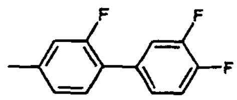 | $CH_3$ |
| -21 | 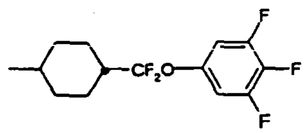 | $n\text{-}C_3H_7$ |
| -22 | 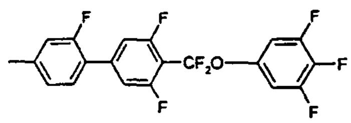 | $CH_3$ |
| -23 | 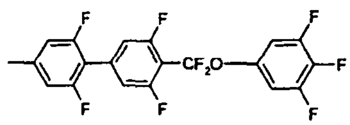 | $CH_3$ |
| -24 | 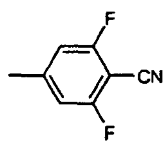 | $CH_3$ |
| -25 | 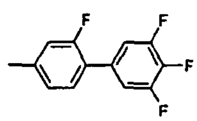 | $CH_3$ |
| -26 | 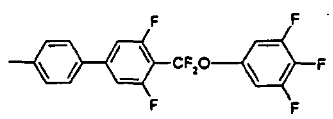 | $CH_3$ |
| -27 | 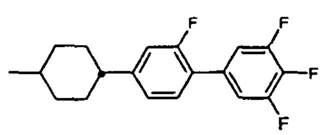 | $CH_3$ |
| -28 | 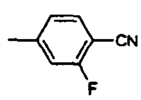 | $CH_3$ |
| -29 | 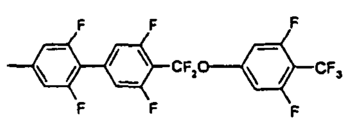 | $CH_3$ |

(fortgesetzt)

| Produkt B4.2-Nr. | $R^{B1}$ | $R^{B2}$ |
|---|---|---|
| -30 | 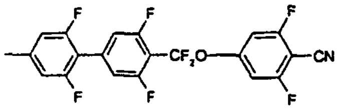 | $CH_3$ |
| -31 | 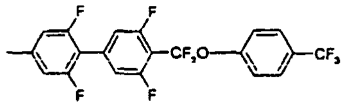 | $CH_3$ |
| -32 | 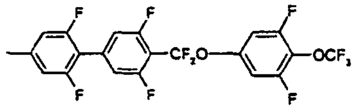 | $CH_3$ |
| -33 | 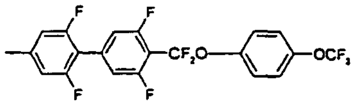 | $CH_3$ |

**Beispiel 5**

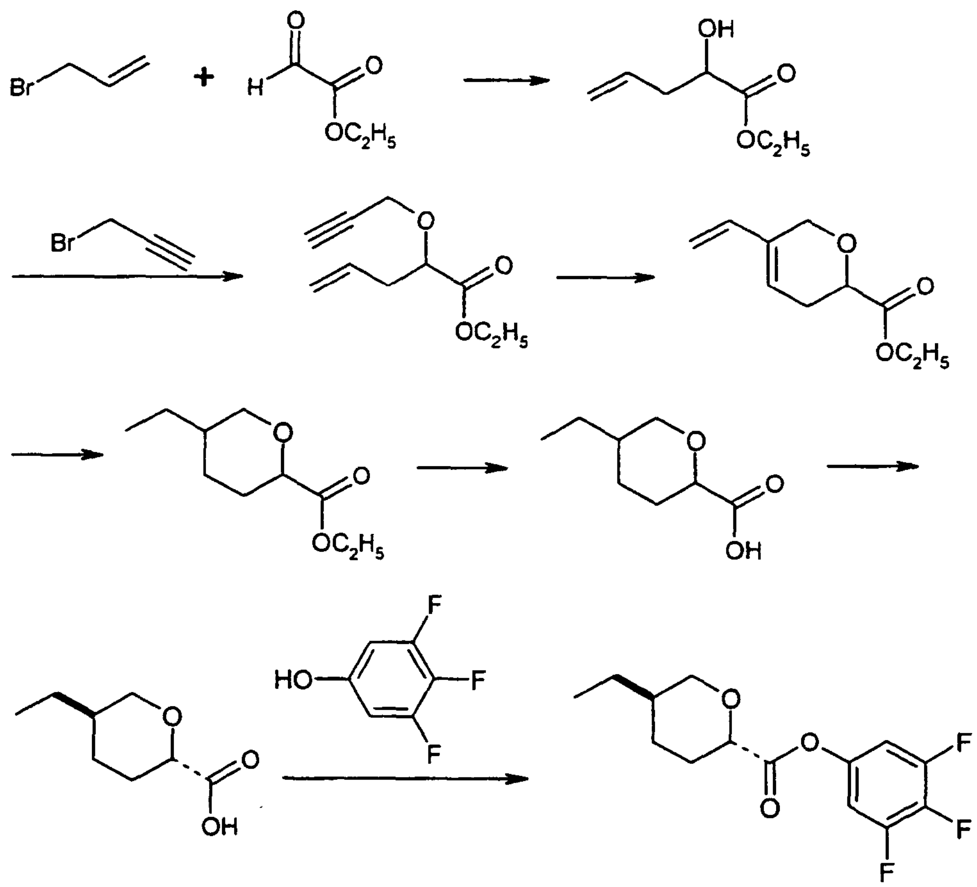

B5. 1-1

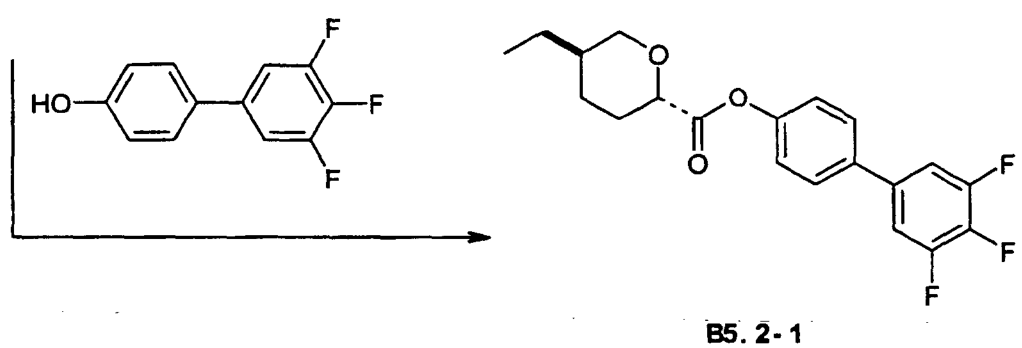

B5. 2-1

Indium-Pulver (200 mmol) wird in Wasser vorgelegt und mit Glyoxalsäureethylester (400 mmol) versetzt. Unter Rühren wird Allylbromid (600 mmoi) zugetropft. Nach Rühren über Nacht wird Ethylacetat (300 m!) hinzugegeben und nochmals 30 min gerührt. Die wäßrige Phase wird mit Ethylacetat extrahiert. Nach Trocknen der vereinigten organischen Phasen wird die Lösung konzentriert unter Erhalt des α-Hydroxyesters (Ausbeute 44 %) in für die weitere Umsetzung ausreichender Reinheit.

Alternativ wird der Glyoxalsäureester in Toluol/Wasser vorgelegt und nacheinander mit Indium-Pulver und Allybromid versetzt und gerührt. Nach Aufarbeitung, Trocknen und Konzentrieren wird der α-Hydroxyester erhalten, der ohne weitere Reinigung im nächsten Reaktionsschritt eingesetzt wird.

Ethyl-2-hydroxypent-4-enoat (139 mmol) wird in Toluol gelöst und bei -20 ˚C mit Natriumhydrid (139 mmol) portionsweise versetzt. Nach 1 h Rühren wird Propargylbromid (146 mmol) zugegeben. Man erwärmt auf Raumtemperatur, kühlt erneut auf -20 ˚C, setzt feuchtes THF zu und extrahiert mit Diethylether. Nach Trocknen, Einengen und Vakuumdestillation wird der gewünschte Propargylether als racemisches Gemisch der Enantiomeren erhalten.

2-Propinyloxy-pent-4-encarbonsäureethylester (64 mmol) wird unter Inertgasatmosphäre mit Bis(tricyclohexylphosphin)benzylidenrutheniumdichlorid COMP-Ru4 (1 mmol) in Dichlormethan versetzt und 8 Tage gerührt. Die Reaktionsmischung wird an Kieselgel chromatographiert und ergibt den gewüschten Dihydropyranester. Dieser wird

unter Raney-Nickel-Katalyse hydriert. Der hydrierte Ester wird mit Natriumhydroxid in 1,4-Dioxan verseift und das cis/trans-Isomerengemisch der 5-Ethyltetrahydropyran-2-carbonsäure in der KOH-Schmelze zum trans-Produkt isomerisiert. Nach Umkristallisieren aus Toluol wird mit 3,4,5-Trifluorphenol in Gegenwart von Dicydocarbodiimid und DMAP der Ester B5.1-1 beziehungsweise mit 3,4,5-Trifluor-4'-hydroxybiphenylder Ester B5.2-1 erhalten.

Alternativ wird die Enin-Metathese in Gegenwart von 1 mmol COMP-Ru4 und 5 mmol Kupfer(I)bromid als Cokatalysator ausgeführt.

**Beispiel 6 - Vorstufen**

**a1)**

**a) Allgemeine Arbeitsvorschrift B 6a1 (AAV** B6a1)

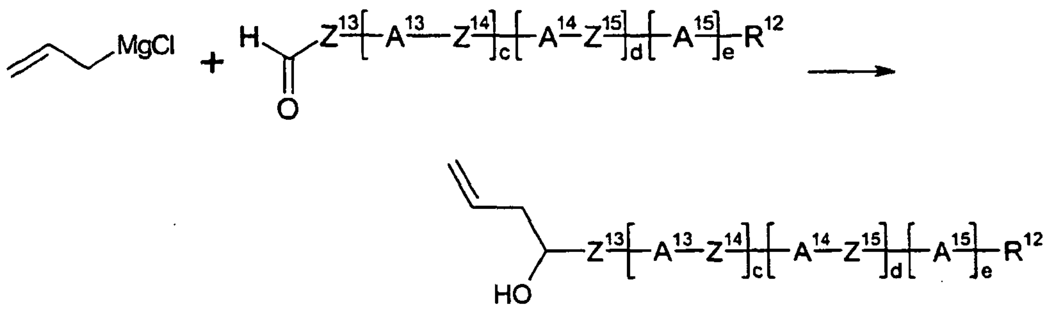

200 ml (2 mol) Allylmagnesiumchlorid in Diethylether (Aldrich Co.) werden unter schwacher Eiskühlung mit dem Aldehyd, gelöst in 100 ml THF, tropfenweise versetzt und dann 4 Stunden bei Raumtemperatur gerührt. Die Redaktionsmischung wird anschließend in 100 ml 0,5 N HCl eingegossen und fünf Minuten gerührt. Die organische Phase wird abgetrennt, die wässrige Phase zweimal mit Methyl-tert.-butylether extrahiert. Die vereinigten organischen Extrakte werden mit Wasser nachgewaschen, getrocknet, filtriert und eingeengt. Das quantitativ erhaltene Rohprodukt kann aufgrund seiner Reinheit direkt in der nachfolgenden Umsetzung verwendet werden.

**a2)**

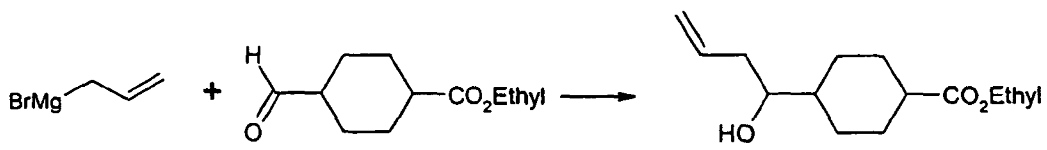

**Allgemeine Arbeitsvorschrift B6a2 (AAV-B6a2)**

Allylmagnesiumbromid in Ether (3,1 mol; von Sigma-Aldrich Co.) wird unter Inertgasatmosphäre vorgelegt und unter Kühlung auf ca. 10°C tropfenweise mit einer Lösung von Zinkbromid (3,1 mol) in Tetrahydrofuran versetzt. Die entstandene Suspension wird bei dieser Temperatur nachgerührt und dann tropfenweise mit einer Lösung des Aldehyds (3,1 mol) in Diethylether versetzt. Nach Rühren über Nacht wird in verdünnte Salzsäure gegossen und wäßrig aufgearbeitet. Die organische Phase wird nach dem Trocknen zu einem Öl konzentriert, das ohne weitere Reinigung zur Herstellung des korrespondierenden Propargylethers eingesetzt wird. Ausbeute: 82 %. Entsprechend werden weitere erfindungsgemäße Homallylalkohole der allgemeinen Formel V-II hergestellt.

Diese Reaktionsführung mit Ummetallierung eignet sich insbesondere für für die Umsetzung von Aldehyden, die neben der aldehydischen Carbonylfunktion eine gegenüber Grignard-Reagenzien reaktive funktionelle Gruppe wie

eine Carbonsäureester- oder Nitrilgruppe enthalten. Die Ummetallierung ist in der Regel überflüssig, wenn eine Carbonsäure- oder Nitrilgruppe-Funktionalität im jeweiligen Aldehyd nicht vorhanden ist, und kann daher aus syntheseökonomischen Gründen entfallen.

**b)**

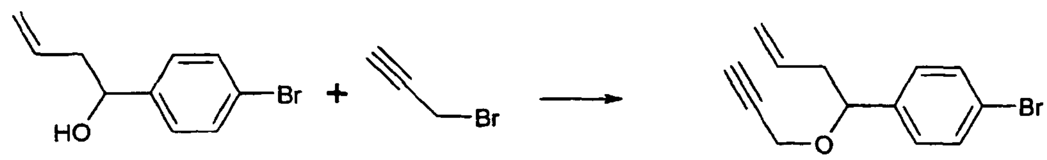

**Allgemeine Arbeitsvorschrift B6b (AAV-B6b)**

Festes Natriumhydroxid (1,6 mol) wird in Tetrahydrofuran vorgelegt und mit wenig Wasser versetzt. Dann wird N-Cetyl-N,N,N-trimethylammoniumbromid zugegeben, anschließend eine Lösung des Homoallylalkohols (0,8 mol) in Tetrahydrofuran und schließlich Propargylbromid (1,32 mol) bei Raumtemperatur zugetropft. Es wird auf ca. 45 ˚C erwärmt und 16 h bei dieser Temperatur gerührt. Dann wird die Reaktionsmischung auf Eiswasser gegossen und wie üblich aufgearbeitet. Nach Trocknen und Konzentrieren der organischen Phase wird an Kieselgel chromatographiert. Die Produktfraktion liefert nach Abdestillieren des Lösungsmittels das Enin. Ausbeute: 75 %. Entsprechend werden weitere erfindungsgemäße Enine mit abweichendem Substitutionsmuster hergestellt.

**c)**

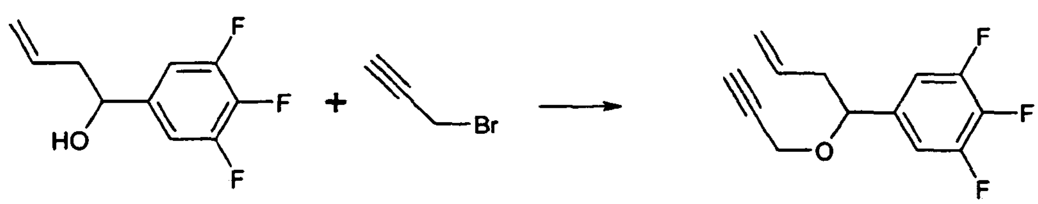

**Allgemeine Arbeitsvorschrift B6c (AAV-B6c)**

Festes Natriumhydroxid (8,1 mol) wird in Tetrahydrofuran suspendiert und mit wenig Wasser versetzt. Anschließend werden der Homoallylalkohol (4,03 mol) und Cetyltrimethylammoniumbromid (0,2 mol) zugegeben. Propargylbromid (4,1 mol) wird bei Raumtemperatur zugetropft. Dann wird auf ca. 40 ˚C erwärmt und 20 h bei dieser Temperatur gerührt.

Anschließend wird nochmals Propargylbromid (0,5 mol) zugetropft und weitere 12 h gerührt. Es wird abgekühlt und auf Eiswasser gegossen. Nach üblicher Aufarbeitung und Trocknen und Konzentrieren der organischen Phase wird an Kieselgel chromatographiert. Die Produktfraktion liefert nach Abdestillieren des Lösungsmittels das Enin. Ausbeute: 98 %. Entsprechend werden weitere erfindungsgemäße Enine mit abweichendem Substitutionsmuster hergestellt.

**Beispiel 7 - Physikalische Parameter erfindungsgemäßer Verbindungen**

In Tabelle B5 sind ausgewählte physikalische Parameter einiger erfindungsgemäßer Verbindungen, die gemäß den Vorschriften der angehenden Beispiele hergestellt worden sind, wiedergegeben. (Die Angaben in der Spalte "Verbindung" beziehen sich auf die in den Beispielen 1 bis 6 wiedergegebenen Bezeichnungen.)

**Tabelle B5**

| Verbindung | Δn | Δε | $\gamma_1$ [mPa.s] | Fp. [˚C] | Klp. [˚C] | Phasenübergänge |
|---|---|---|---|---|---|---|
| B1.2-3* | 0,0469 | 3,1 | 20 | 14 | | |
| B1.2-4* | 0,0240 | 7,7 | 23 | 28 | | K 28 1 |
| B1.2-6** | 0,0850 | 11,5 | 144 | 131 | 31*** | K 131 I |
| B1.2-7* | 0,1753 | 13,7 | 211 | 95 | 48*** | K 95 I |
| B1.2-11 * | 0,0870 | -3,4 | 92 | 53 | -25*** | K 53 1 |
| B1.2-22* | 0,1841 | 9,9 | | 117 | 124*** | K 117 I |
| B2.2-3* | 0,1020 | 11,7 | 398 | 70 | 72 | K 70 N 72 I |
| B2.2-5* | 0,0920 | 12,9 | | 59 | 75 | K 59 N 75 I |
| B2.2-11** | 0,1785 | 26,8 | | 125 | 159 | K 125 N 159 I |
| B3.1-5** | 0,1745 | 22,6 | 312 | 116 | 128 | K 116 N 128 I |
| B4.1-4* | 0,0475 | 14,0 | 140 | 72 | 16*** | K 72 I |
| B4.1-5* | 0,1159 | 14,5 | 130 | 52 | 20*** | K 52 I |
| B4.1-6* | 0,1493 | 27,3 | 287 | 78 | 92 | K 78 N 92 I |
| B4.1-7* | 0,0440 | -2,5 | 84 | 22 | -40*** | |
| B4.1-8* | 0.1422 | 10,1 | 116 | 54 | 107*** | K 54 Sm? 87 SmB 154 SmA 168 I |
| B4.1-9* | 0,0540 | 14,1 | 124 | 45 | 26*** | |
| B4.1-12* | 0,1070 | 29,7 | 213 | 89 | 45*** | K 89 I |
| B4.1-13* | 0,1310 | 29,9 | | 89 | 57*** | |
| B4.1-14* | 0,1157 | 12,3 | | 28 | 25*** | K 28 N (11) I |
| B4.1-15* | 0,1100 | 15,2 | | 37 | 39 | K 37 SmA(35) N 39 I |
| B4.1-16* | 0,0920 | 17,8 | | 68 | -12*** | K 68 I |
| B4.2-4* | 0,1243 | 14,8 | 178 | 41 | 44*** | K 41 I |
| B4.2-5* | 0,1256 | 18,0 | 239 | 58 | 61 | K 58 SmA (37) N 61 I |
| B4.2-6* | 0,0599 | 14,1 | 136 | 71 | 31*** | K 71 I |
| B4.2-7* | 0,0570 | 13,4 | 160 | 35 | 66 | K 35 N 66 I |
| B4.2-8* | 0,1430 | 9,6 | 189 | 50 | 99 | K 50 Sm? (32) SmA 64 N 99 I |
| B4.2-9* | 0,1380 | 12,7 | 236 | 45 | 89*** | K 45 Sm? 68 Sm? 118 I |
| B4.2-10* | 0,0688 | 9,7 | 187 | 41 | 96 | K 41 SmB 51 N 96 I |
| B4.2-1 1 * | 0,0780 | 9,1 | 178 | -41 | 129 | K-41 SmB 123 N 129 |
| B4.2-12* | 0,0701 | 11,7 | 199 | | 106 | SmB 74 N 106 I |
| B4.2-13* | 0,0623 | 11,5 | | | 106 | SmB 81 N 106 I |
| B4.2-14* | 0,0689 | 9,1 | | -54 | 124*** | K -54 SmB 129 I |
| B4.2-15* | 0,1220 | 19,1 | 625 | 61 | 192 | K 61 N 192 I |
| B4.2-16* | 0,1328 | 13,4 | 806 | 44 | 213 | K 44 Sm? 45 N 213 |
| B4.2-17* | 0,1390 | 9,4 | | 84 | 232 | K 84 N 232 I |
| B4.2-18* | 0,1370 | 10,7 | | 47 | 238 | K 47 SmB 91 N 238 I |
| B4.2-19* | 0,1291 | 15,7 | 725 | 60 | 207 | K 60 SmB 81 N 207 I |
| B4.2-20* | 0,1254 | 12,7 | | 43 | 44 | K 43 N 44 I |

(fortgesetzt)

| **Verbindung** | $\Delta n$ | $\Delta\varepsilon$ | $\gamma_1$ [mPa.s] | **Fp.** [˚C] | **Klp.** [˚C] | **Phasenübergänge** |
|---|---|---|---|---|---|---|
| B4.2-21* | 0,0568 | 11,8 | 216 | 39 | 75 | K 39 N 75 I |
| B4.2-22* | 0,1364 | 29,7 | | 70 | 102 | K 70 N 102 I |
| B4.2-23* | 0,1231 | 34,9 | 457 | 81 | 83 | K 81 N 83 I |
| B4.2-24* | 0,1060 | 40,0 | | 27 | | |
| B4.2-25* | 0,1071 | 17,3 | 176 | 64 | 13*** | K 64 I |
| B4.2-26* | 0,1450 | 32,2 | 300 | 75 | 118 | K 75 N 118 I |
| B4.2-27* | 0,1384 | 19,7 | 718 | 74 | 198 | K 74 N 198 I |
| 84.2-28* | 0,0800 | 28,2 | 128 | 32 | -50*** | K 32 I |
| B4.2-29** | 0,1190 | 40,8 | | 91 | 55*** | K 91 |
| B4.2-30* | 0.1561 | 66,5 | | 74 | 141 | K 74 SmC? (65) N 141 |
| B4.2-31* | 0,1330 | 31,5 | | 95 | 75*** | K 95 N (87) |
| B4.2-32* | 0,1158 | 35,6 | | 73 | 98 | K 73 N 98 |
| B4.2-33* | 0,1330 | 25, 3 | | 80 | 120 | K 80 N 120 |

* Die Bestimmung der Parameter $\Delta n$, $\Delta\varepsilon$ und $\gamma_1$ erfolgte durch Messung einer Mischung von 10 Gew.% der Verbindung in dem Host ZLI-4792 (Merck KGaA, Darmstadt) und nachfolgende Extrapolation.

** Die Bestimmung der Parameter $\Delta n$, $\Delta\varepsilon$ und $\gamma_1$ erfolgte mit 5 Gew.% der Verbindung in ZLI-4792.

*** Die Bestimmung des Klärpunkts erfolgte durch Messung einer Mischung von 10 beziehungsweise 5 Gew.% der Verbindung in dem Host ZLI-4792 und nachfolgende Extrapolation.

## Patentansprüche

**1.** Verbindung der allgemeinen Formel I

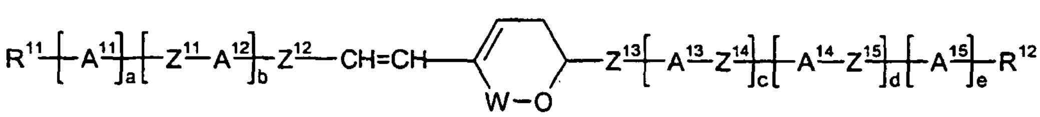

I

wobei

a, b, c, d und e jeweils unabhängig voneinander 0 oder 1 sind;
W $-CH_2-$ oder -C(=O)- bedeutet;
$R^{11}$ H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C=C-, -CH=CH-, -O-, -S-, -C(O)-O- und/oder -O-C(O)- so ersetzt sein können, dass Heteroatome (O, S) nicht direkt miteinander verknüpft sind;
$R^{12}$ H, Halogen, -CN, -NCS, Aralkyl, -O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C≡C-, -CH=CH-, -O-, -S-, -C(O)-O- und/oder -O-C(O)- so ersetzt sein können, dass Heteroatome (O, S) nicht direkt miteinander verknüpft sind;
$Z^{11}$ eine Einfachbindung, $-CH_2-$, $-CH_2CH_2-$, $-CF_2CH_2-$, $-CH_2CF_2-$, $-CF_2CF_2-$, -CH=CH- oder -C=C- bedeutet;
$Z^{12}$ eine Einfachbindung, $-CH_2-$, $-CH_2CH_2-$, $-CF_2CH_2-$, $-CH_2CF_2-$ oder $-CF_2CF_2-$ bedeutet;
$Z^{13}$, $Z^{14}$ und $Z^{15}$ jeweils unabhängig voneinander eine Einfachbindung, $-CH_2CH_2-$, $-CF_2CH_2-$, $-CH_2CF_2-$,

$-CF_2CF_2-$, -CH=CH-, -C≡C-, $-CH_2O-$, $-CF_2O-$, -C(O)- oder -C(O)-O- bedeuten;
$A^{11}$ und $A^{12}$ unabhängig voneinander für

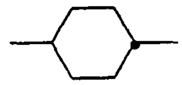

oder

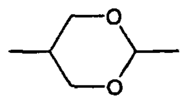

stehen;
$A^{13}$ und $A^{14}$ unabhängig voneinander für

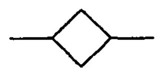
,

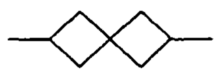
,
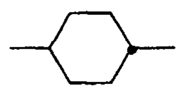
,
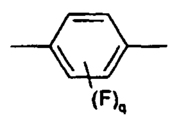
,

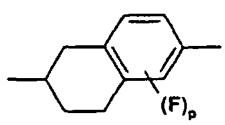
oder
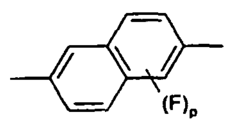

stehen;
$A^{15}$ für

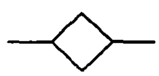
,
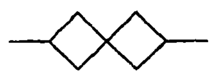
,
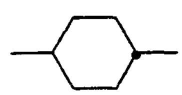
,

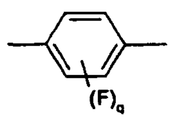
,
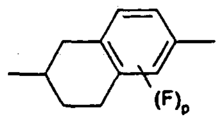
oder
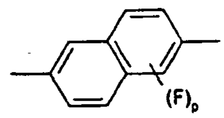

steht oder
$A^{15}$-$R^{12}$ zusammen für

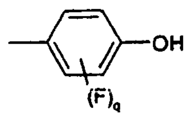 , 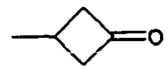 ,

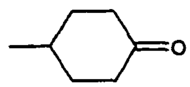 oder 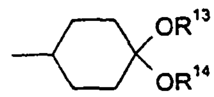

steht;
oder
$Z^{13}$-[-$A^{13}$-$Z^{14}$-]$_c$-[-$A^{14}$-$Z^{15}$-]$_d$-[-$A^{15}$-]$_e$-$R^{12}$ für

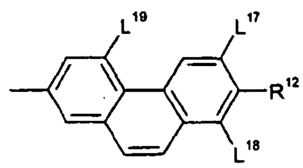

oder

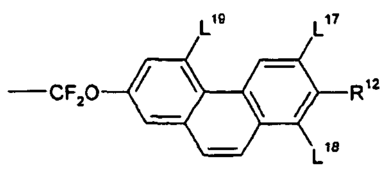

steht, wobei $R^{12}$ wie oben in diesem Anspruch definiert ist und $L^{17}$, $L^{18}$ und $L^{19}$ unabhängig voneinander H oder F bedeuten;
q 0, 1, 2, 3 oder 4 ist;
p 0, 1, 2 oder 3 ist;
$R^{13}$ und $R^{14}$ unabhängig voneinander einen Alkanylrest mit 1 bis 7 Kohlenstoffatomen bedeutet oder zusammen für eine Alkylenbrücke mit 2 bis 7 Kohlenstoffatomen stehen;

mit der Maßgabe,
dass für den Fall der direkten Verknüpfung von $Z^{13}$ und $R^{12}$ zu -$Z^{13}$-$R^{12}$ $R^{12}$ H, Aralkyl, Alkanyl oder Alkenyl bedeutet, wenn $Z^{13}$ -C(=O)-O- oder -C(=O)- bedeutet, und $Z^{13}$ nicht -$CH_2$O- oder -$CF_2$O-bedeutet;
dass für den Fall der direkten Verknüpfung von $Z^{14}$ und $R^{12}$ zu -$Z^{14}$-$R^{12}$ $R^{12}$ H, Aralkyl, Alkanyl oder Alkenyl bedeutet, wenn $Z^{14}$ -C(=O)-O- oder -C(=O)- bedeutet, und $Z^{14}$ nicht -$CH_2$O- oder -$CF_2$O-bedeutet;
dass für den Fall der direkten Verknüpfung von $Z^{15}$ und $R^{12}$ zu -$Z^{15}$-$R^{12}$ $R^{12}$ H, Aralkyl, Alkanyl oder Alkenyl bedeutet, wenn $Z^{15}$ -C(=O)-O- oder -C(=O)- bedeutet, und $Z^{15}$ nicht -$CH_2$O- oder -$CF_2$O-bedeutet.

**2.** Verbindung gemäß Anspruch 1, **dadurch gekennzeichnet, dass**

$$a+b+c+d+e \leq 3.$$

**3.** Verbindung gemäß einem der vorangehenden Ansprüche, **dadurch gekennzeichnet, dass** W -C(=O)- bedeutet.

4. Verbindung gemäß Anspruch 1 oder 2, **dadurch gekennzeichnet, dass** W $-CH_2-$ bedeutet.

5. Verbindung gemäß einem der vorangehenden Ansprüche, **dadurch gekennzeichnet, dass**

$Z^{12}$ eine Einfachbindung bedeutet.

6. Verbindung gemäß Anspruch 5, **dadurch gekennzeichnet, dass**

a und b null sind; und $R^{11}$ H ist.

7. Verbindung gemäß einem der vorangehenden Ansprüche, **dadurch gekennzeichnet, dass**

$Z^{13}$ eine Einfachbindung, -C(O)-O- oder $-CF_2O-$ bedeutet.

8. Verbindung gemäß einem der Ansprüche 1 bis 5 oder 7, **dadurch gekennzeichnet, dass**

a null ist;
b 1 ist;
$Z^{11}$ eine Einfachbindung bedeutet; und
$A^{12}$ für

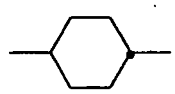

steht.

9. Verbindung gemäß einem der vorangehenden Ansprüche, **dadurch gekennzeichnet, dass**

c, d und e zugleich null sind;
$Z^{13}$ -C(O)-O- bedeutet; und
$R^{12}$ H, Aralkyl, Alkanyl oder Alkenyl bedeutet.

10. Verbindung gemäß einem der Ansprüche 1 bis 8, **dadurch gekennzeichnet, dass**

e 1 ist;
$A^{15}$ für

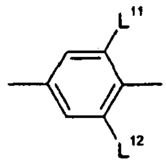

steht; und
$L^{11}$ und $L^{12}$ unabhängig voneinander H oder F bedeuten.

11. Verbindung gemäß einem der Ansprüche 1 bis 8 oder 10, **dadurch gekennzeichnet, dass** wenigstens eines von c und d 1 ist;

e 1 ist; und
$Z^{14}$ und $Z^{15}$ unabhängig voneinander eine Einfachbindung oder $-CF_2O-$ bedeuten.

12. Verbindung gemäß einem der Ansprüche 1 bis 8 oder 11, **dadurch gekennzeichnet, dass**

e 1 ist;
$A^{15}$-$R^{12}$ für

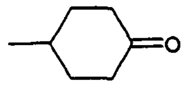 oder 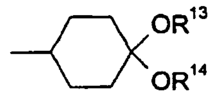

steht; und
$R^{13}$ und $R^{14}$ wie in Anspruch 1 definiert sind.

**13.** Verfahren zur Herstellung von Pyranderivaten umfassend einen Verfahrensschritt, der eine Enin-Metathese-Reaktion eines Enins der allgemeinen Formel II

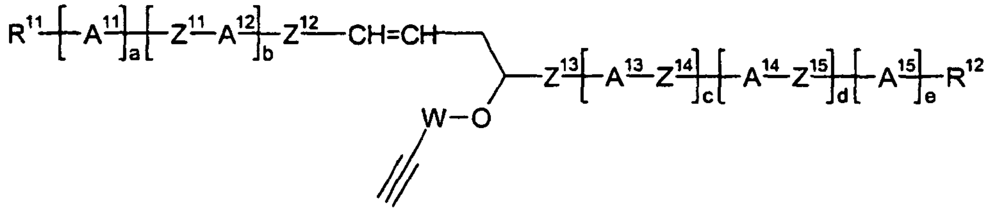

II

zu einem Pyranderivat der Formel I

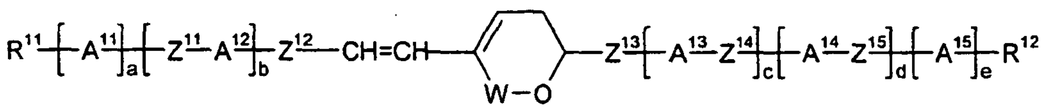

I

wobei a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ in den Formeln I und II die gleiche Bedeutung wie in Anspruch 1 für Formel I haben;
mit der Maßgabe,
dass für den Fall der direkten Verknüpfung von $Z^{13}$ und $R^{12}$ zu -$Z^{13}$-$R^{12}$ $R^{12}$ H, Aralkyl oder Alkanyl bedeutet, wenn $Z^{13}$ -C(=O)-bedeutet, $R^{12}$ Aralkyl oder Alkanyl bedeutet, wenn $Z^{13}$ -C(=O)-O-bedeutet, und $Z^{13}$ nicht -$CH_2O$- oder -$CF_2O$- bedeutet;
dass für den Fall der direkten Verknüpfung von $Z^{14}$ und $R^{12}$ zu -$Z^{14}$-$R^{12}$ $R^{12}$ H, Aralkyl oder Alkanyl bedeutet, wenn $Z^{14}$ -C(=O)-bedeutet, $R^{12}$ Aralkyl oder Alkanyl bedeutet, wenn $Z^{14}$ -C(=O)-O-bedeutet, und $Z^{14}$ nicht -$CH_2O$- oder -$CF_2O$- bedeutet;
dass für den Fall der direkten Verknüpfung von $Z^{15}$ und $R^{12}$ zu -$Z^{15}$-$R^{12}$ $R^{12}$ H, Aralkyl oder Alkanyl bedeutet, wenn $Z^{15}$ -C(=O)-bedeutet, $R^{12}$ Aralkyl oder Alkanyl bedeutet, wenn $Z^{15}$ -C(=O)-O-bedeutet, und $Z^{15}$ nicht -$CH_2O$- oder -$CF_2O$- bedeutet; in Gegenwart eines Metathese-Katalysators umfaßt.

**14.** Verfahren nach Anspruch 13, **dadurch gekennzeichnet, dass** der Metathese-Katalysator ein Ruthenium-Alkyliden-Komplex ist.

**15.** Verfahren zur Herstellung von Pyranderivaten umfassend einen Verfahrensschritt, der eine Kreuz-Metathese-Reaktion eines Pyranderivats der allgemeinen Formel I-C mit einem Alken der Formel IIIa oder der Formel IIIb

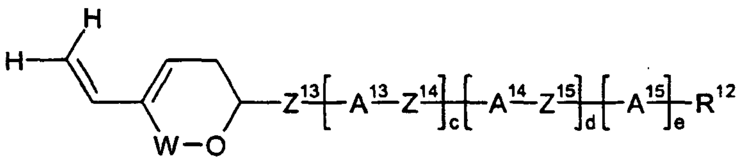

I-C

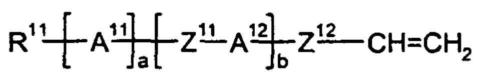

IIIa

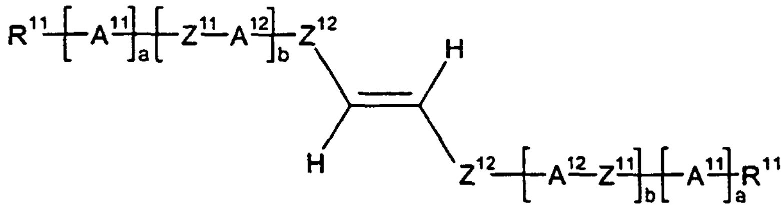

IIIb

zu einem Pyranderivat der Formel I

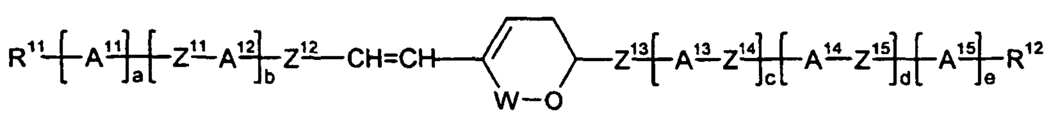

I

wobei a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ in den Formeln I, I-C, IIIa und IIIb die gleiche Bedeutung wie in Anspruch 1 für Formel I haben;
mit der Maßgabe,
dass für den Fall der direkten Verknüpfung von $Z^{13}$ und $R^{12}$ zu $-Z^{13}-R^{12}$ $R^{12}$ H, Aralkyl oder Alkanyl bedeutet, wenn $Z^{13}$ -C(=O)-bedeutet, $R^{12}$ Aralkyl oder Alkanyl bedeutet, wenn $Z^{13}$ -C(=O)-O-bedeutet, und $Z^{13}$ nicht $-CH_2O-$ oder $-CF_2O-$ bedeutet;
dass für den Fall der direkten Verknüpfung von $Z^{14}$ und $R^{12}$ zu $-Z^{14}-R^{12}$ $R^{12}$ H, Aralkyl oder Alkanyl bedeutet, wenn $Z^{14}$ -C(=O)-bedeutet, $R^{12}$ Aralkyl oder Alkanyl bedeutet, wenn $Z^{14}$ -C(=O)-O-bedeutet, und $Z^{14}$ nicht $-CH_2O-$ oder $-CF_2O-$ bedeutet;
dass für den Fall der direkten Verknüpfung von $Z^{15}$ und $R^{12}$ zu $-Z^{15}-R^{12}$ $R^{12}$ H, Aralkyl oder Alkanyl bedeutet, wenn $Z^{15}$ -C(=O)-bedeutet, $R^{12}$ Aralkyl oder Alkanyl bedeutet, wenn $Z^{15}$ -C(=O)-O-bedeutet, und $Z^{15}$ nicht $-CH_2O-$ oder $-CF_2O-$ bedeutet;
in Gegenwart eines Metathese-Katalysators umfaßt.

**16.** Verfahren nach Anspruch 15, **dadurch gekennzeichnet, dass** der Metathese-Katalysator ein Ruthenium-Alkyliden-Komplex ist.

**17.** Verfahren nach Anspruch 15 oder 16, **dadurch gekennzeichnet, dass** die Verbindung der allgemeinen Formel I-C nach einem Verfahren gemäß einem der Ansprüche 13 oder 14 hergestellt wird.

**18.** Verfahren gemäß einem der Ansprüche 13 bis 17 umfassend nach dem Metathese-Reaktionsschritt als einen

weiteren Reaktionsschritt eine Reduktionsreaktion zur Überführung des Pyranderivats der Formel I, worin W -C(=O)- bedeutet und $Z^{13}$, $Z^{14}$ und $Z^{15}$ nicht -C(O)-bedeuten, in ein Pyranderivat der Formel 1, worin

W $-CH_2-$ bedeutet;
$R^{11}$ H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C≡C-, -CH=CH-, -O- und/oder -S- so ersetzt sein können, dass Heteroatome (O, S) nicht direkt miteinander verknüpft sind;
$R^{12}$ H, Halogen, -CN, -NCS, Aralkyl, -O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere $CH_2$-Gruppen durch -C≡C-, CH=CH-, -O- und/oder -S- so ersetzt sein können, dass Heteroatome (O, S) nicht direkt miteinander verknüpft sind;
$Z^{13}$, $Z^{14}$ und $Z^{15}$ jeweils unabhängig voneinander eine Einfachbindung, $-CH_2CH_2-$, $-CF_2CH_2-$, $-CH_2CF_2-$, $-CF_2CF_2-$, -CH=CH-, -C≡C-, $-CH_2O-$ oder $-CF_2O-$ bedeuten;

und a, b, c, d, e, $Z^{11}$, $Z^{12}$, $A^{11}$, $A^{12}$, $A^{13}$, $A^{14}$ und $A^{15}$ wie in Anspruch 1 definiert sind.

**19.** Verfahren gemäß einem der Ansprüche 13 bis 18 umfassend nach dem Metathese-Reaktionsschritt und nach dem gegebenenfalls durchzuführenden Reduktionsreaktionsschritt als einen weiteren Reaktionsschritt eine katalytische Hydrierung zu einem Pyran der allgemeinen Formel IV:

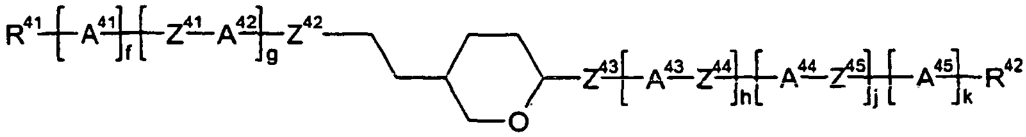

IV

worin

f, g, h, j und k jeweils unabhängig voneinander 0 oder 1 sind;
$R^{41}$ H, einen unsubstituierten oder einfach oder mehrfach gleich oder verschieden mit Halogen oder -CN substituierten gesättigten Alkylrest mit 1 bis 15 Kohlenstoffatomen bedeutet, wobei in diesem Rest auch eine oder mehrere $CH_2$-Gruppen durch -O-, -S-, -C(O)-O- und/oder -O-C(O)- so ersetzt sein können, dass Heteroatome (O, S) nicht direkt miteinander verknüpft sind;
$R^{42}$ H, Halogen, -CN, -NCS, Aralkyl, -O-Aralkyl oder einen unsubstituierten oder einfach oder mehrfach gleich oder verschieden mit Halogen oder -CN substituierten gesättigten Alkylrest mit 1 bis 15 C-Atomen bedeutet, wobei in diesem Rest auch eine oder mehrere $CH_2$-Gruppen durch -O-, -S-, - C(O)-O- und/oder -O-C(O)- so ersetzt sein können, dass Heteroatome (O, S) nicht direkt miteinander verknüpft sind;
$Z^{41}$ und $Z^{42}$ unabhängig voneinander eine Einfachbindung, $-CH_2-$, $-CH_2CH_2-$, $-CF_2CH_2-$, $-CH_2CF_2-$ oder $-CF_2CF_2-$ bedeuten;
$Z^{43}$, $Z^{44}$ und $Z4^5$ jeweils unabhängig voneinander eine Einfachbindung, $-CH_2CH_2-$, $-CF_2CH_2-$, $-CH_2CF_2-$, $-CF_2CF_2-$, $-CH_2O-$, $-CF_2O-$, -C(O)- oder - C(O)-O- bedeuten;
$A^{41}$ und $A^{42}$ unabhängig voneinander für

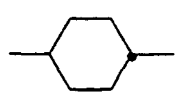

oder

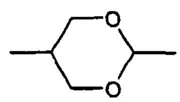

stehen;
$A^{43}$ und $A^{44}$ unabhängig voneinander für

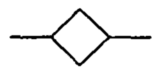
,

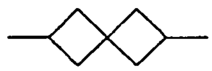 , 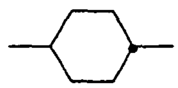 , 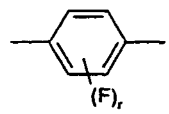 ,

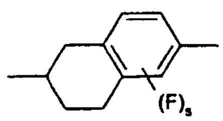 oder 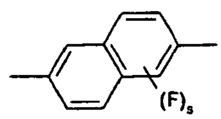

stehen;
$A^{45}$ für

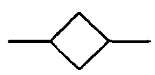 , 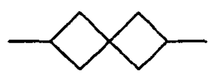 , 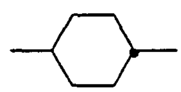 ,

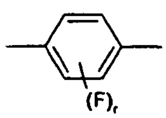 , 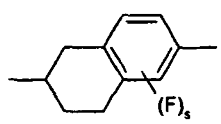 oder 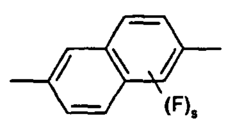

steht oder
$A^{45}$-$R^{42}$ zusammen für

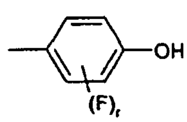 , 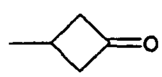 ,

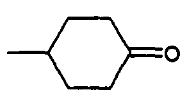 oder 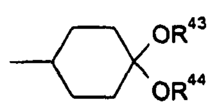

steht;
oder

$Z^{43}$-[-$A^{43}$-$Z^{44}$-]$_h$-[-$A^{44}$-$Z^{45}$-]$_j$-[-$A^{45}$-]$_k$-$R^{42}$ für

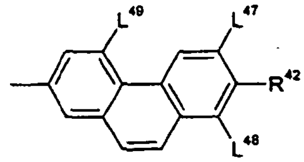

oder

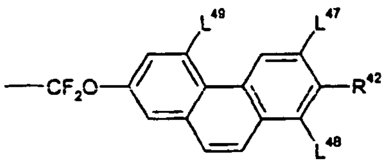

steht, wobei $R^{42}$ wie oben in diesem Anspruch definiert ist und $L^{47}$, $L^{48}$ und $L^{49}$ unabhängig voneinander H oder F bedeuten;
r 0, 1, 2, 3 oder 4 ist;
s 0, 1, 2 oder 3 ist;
$R^{43}$ und $R^{44}$ unabhängig voneinander einen Alkanylrest mit 1 bis 7 Kohlenstoffatomen bedeutet oder zusammen für eine Alkylenbrücke mit 2 bis 7 Kohlenstoff atomen stehen;

mit der Maßgabe,
dass für den Fall der direkten Verknüpfung von $Z^{43}$ und $R^{42}$ zu -$Z^{43}$-$R^{42}$ $R^{42}$ H, Aralkyl oder Alkanyl bedeutet, wenn $Z^{43}$ -C(=O)-bedeutet, $R^{42}$ Aralkyl oder Alkanyl bedeutet, wenn $Z^{43}$ -C(=O)-O-bedeutet, und $Z^{43}$ nicht -$CH_2O$- oder -$CF_2O$- bedeutet;
dass für den Fall der direkten Verknüpfung von $Z^{44}$ und $R^{42}$ zu - $Z^{44}$-$R^{42}$ $R^{42}$ H, Aralkyl oder Alkanyl bedeutet, wenn $Z^{44}$ -C(=O)-bedeutet, $R^{42}$ Aralkyl oder Alkanyl bedeutet, wenn $Z^{44}$ -C(=O)-O-bedeutet, und $Z^{44}$ nicht -$CH_2O$- oder -$CF_2O$- bedeutet;
dass für den Fall der direkten Verknüpfung von $Z^{45}$ und $R^{42}$ zu -$Z^{45}$-$R^{42}$ $R^{42}$ H, Aralkyl oder Alkanyl bedeutet, wenn $Z^{45}$ -C(=O)-bedeutet, $R^{42}$ Aralkyl oder Alkanyl bedeutet, wenn $Z^{45}$ -C(=O)-O-bedeutet, und $Z^{45}$ nicht -$CH_2O$- oder -$CF_2O$- bedeutet.

**20.** Verbindung der Formel II

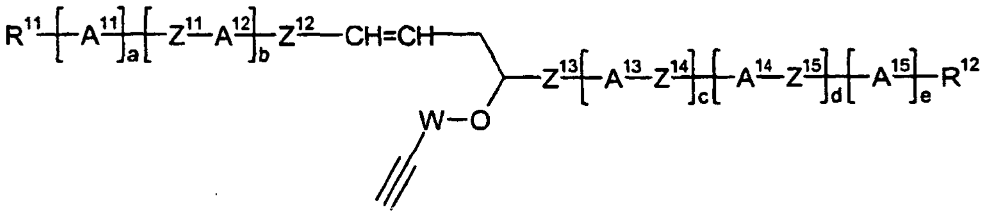

II

wobei a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ die gleiche Bedeutung wie in Anspruch 1 für Formel I haben.

**21.** Verbindung der Formel V-II

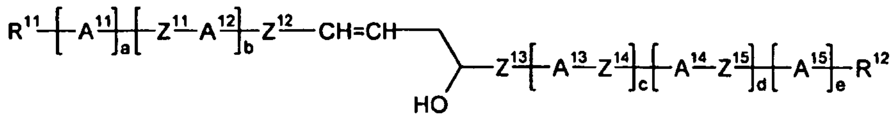 V-II

wobei a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ und $A^{15}$ die gleiche Bedeutung wie in Anspruch 1 für Formel I haben.

**22.** Verwendung einer Verbindung der Formel I gemäß Anspruch 1 als Bestandteil eines flüssigkristallinen Mediums in einer elektrooptischen Anzeigevorrichtung.

## Claims

**1.** Compound of the general formula I

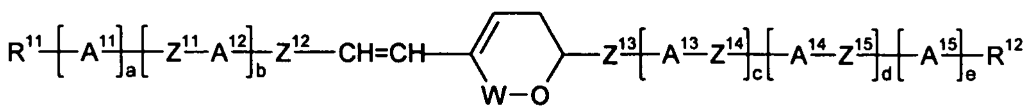

I

where

a, b, c, d and e are each, independently of one another, 0 or 1;
W denotes $-CH_2-$ or -C(=O)-;
$R^{11}$ denotes H, an alkyl radical having 1 to 15 carbon atoms which is unsubstituted or mono- or polysubstituted, identically or differently, by halogen or -CN, where, in addition, one or more $CH_2$ groups in this radical may be replaced by -C≡C-, -CH=CH-, -O-, -S-, -C(O)-O- and/or -O-C(O)- in such a way that heteroatoms (O, S) are not linked directly to one another;
$R^{12}$ denotes H, halogen, -CN, -NCS, aralkyl, -O-aralkyl or an alkyl radical having 1 to 15 carbon atoms which is unsubstituted or mono- or polysubstituted, identically or differently, by halogen or -CN, where, in addition, one or more $CH_2$ groups in this radical may be replaced by -C≡C-, -CH=CH-, -O-, -S-, -C(O)-O- and/or -O-C (O)- in such a way that heteroatoms (O, S) are not linked directly to one another;
$Z^{11}$ denotes a single bond, $-CH_2-$, $-CH_2CH_2-$, $-CF_2CH_2-$, $-CH_2CF_2-$, $-CF_2CF_2-$, -CH=CH- or -C≡C-;
$Z^{12}$ denotes a single bond, $-CH_2-$, $-CH_2CH_2-$, $-CF_2CH_2-$, $-CH_2CF_2-$ or $-CF_2CF_2-$;
$Z^{13}$, $Z^{14}$ and $Z^{15}$ each, independently of one another, denote a single bond, $-CH_2CH_2-$, $-CF_2CH_2-$, $-CH_2CF_2-$, $-CF_2CF_2-$, -CH=CH-, -C≡C-, $-CH_2O-$, $-CF_2O-$, -C(O)- or -C(O)-O-;
$A^{11}$ and $A^{12}$, independently of one another, stand for

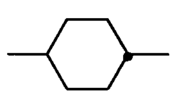 or 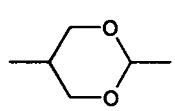 ;

$A^{13}$ and $A^{14}$, independently of one another, stand for

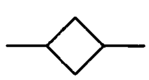 , 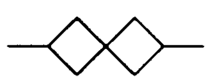 , 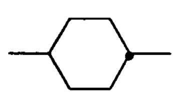 ,

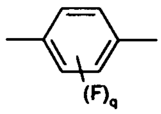 , 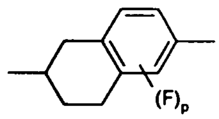 or 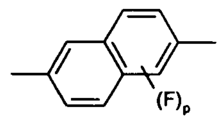 ;

$A^{15}$ stands for

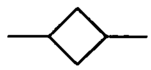 , 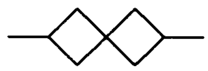 ,

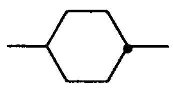 , 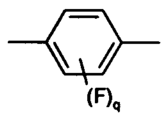 , 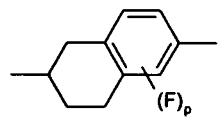

or

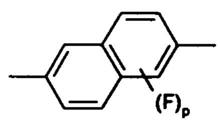

or
$A^{15}$-$R^{12}$ together stand for

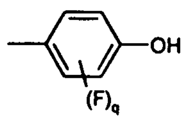 , 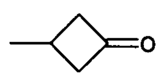 ,

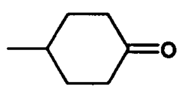 or 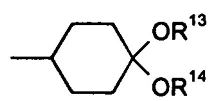 ;

or
$Z^{13}$-[-$A^{13}$-$Z^{14}$-]$_c$-[-$A^{14}$-$Z^{15}$-]$_d$-[-$A^{15}$-]$_e$-$R^{12}$ stands for

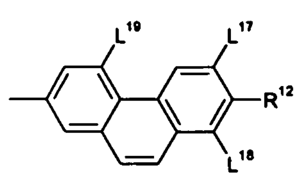

or

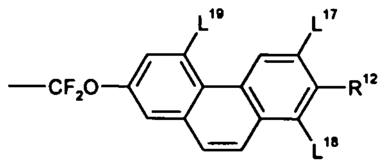
,

where $R^{12}$ is as defined above in this claim and $L^{17}$, $L^{18}$ and $L^{19}$, independently of one another, denote H or F;
q is 0, 1, 2, 3 or 4;
p is 0, 1, 2 or 3;
$R^{13}$ and $R^{14}$, independently of one another, denote an alkanyl radical having 1 to 7 carbon atoms or together stand for an alkylene bridge having 2 to 7 carbon atoms;

with the proviso
that, in the case of direct linking of $Z^{13}$ and $R^{12}$ to give $-Z^{13}-R^{12}$, $R^{12}$ denotes H, aralkyl, alkanyl or alkenyl if $Z^{13}$ denotes -C(=O)-O- or -C(=O)-, and $Z^{13}$ does not denote $-CH_2O-$ or $-CF_2O-$;
that, in the case of direct linking of $Z^{14}$ and $R^{12}$ to give $-Z^{14}-R^{12}$, $R^{12}$ denotes H, aralkyl, alkanyl or alkenyl if $Z^{14}$ denotes -C(=O)-O- or -C(=O)-, and $Z^{14}$ does not denote $-CH_2O-$ or $-CF_2O-$;
that, in the case of direct linking of $Z^{15}$ and $R^{12}$ to give $-Z^{15}-R^{12}$, $R^{12}$ denotes H, aralkyl, alkanyl or alkenyl if $Z^{15}$ denotes -C(=O)-O- or -C(=O)-, and $Z^{15}$ does not denote $-CH_2O-$ or $-CF_2O-$.

2. Compound according to Claim 1, **characterised in that**

$$a+b+c+d+e \leq 3.$$

3. Compound according to one of the preceding claims, **characterised in that**

W denotes -C(=O)-.

4. Compound according to Claim 1 or 2, **characterised in that**

W denotes $-CH_2-$.

5. Compound according to one of the preceding claims, **characterised in that**

$Z^{12}$ denotes a single bond.

6. Compound according to Claim 5, **characterised in that**

a and b are zero; and
$R^{11}$ is H.

7. Compound according to one of the preceding claims, **characterised in that**

$Z^{13}$ denotes a single bond, -C(O)-O- or $-CF_2O-$.

8. Compound according to one of Claims 1 to 5 or 7, **characterised in that**

a is zero;
b is 1;
$Z^{11}$ denotes a single bond; and
$A^{12}$ stands for

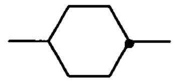

.

**9.** Compound according to one of the preceding claims, **characterised in that**

c, d and e are simultaneously zero;
$Z^{13}$ denotes -C(O)-O-; and
$R^{12}$ denotes H, aralkyl, alkanyl or alkenyl.

**10.** Compound according to one of Claims 1 to 8, **characterised in that**

e is 1;
$A^{15}$ stands for

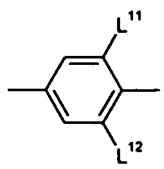

;

and
$L^{11}$ and $L^{12}$, independently of one another, denote H or F.

**11.** Compound according to one of Claims 1 to 8 or 10, **characterised in that** at least one of c and d is 1;

e is 1; and
$Z^{14}$ and $Z^{15}$, independently of one another, denote a single bond or $-CF_2O-$.

**12.** Compound according to one of Claims 1 to 8 or 11, **characterised in that**

e is 1;
$A^{15}$-$R^{12}$ stands for

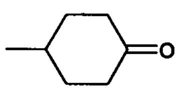

or

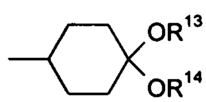

;

and
$R^{13}$ and $R^{14}$ are as defined in Claim 1.

**13.** Process for the preparation of pyran derivatives which includes a process step which includes an enyne metathesis reaction of an enyne of the general formula II

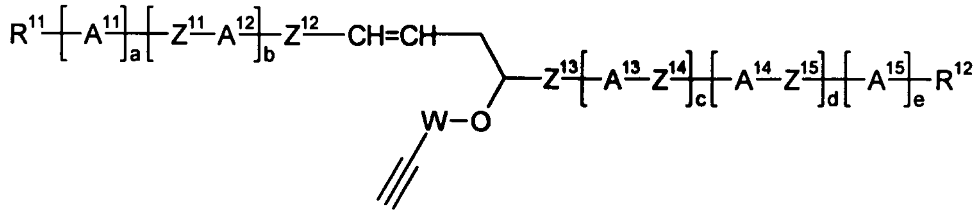

II

to give a pyran derivative of the formula I

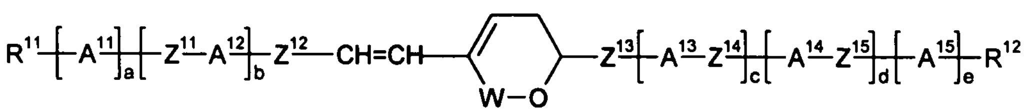

I

where a, b, c, d, e, W, $R^{11}$, $R^{12}$, $Z^{11}$, $Z^{12}$, $Z^{13}$, $Z^{14}$, $Z^{15}$ , $A^{11}$, $A^{12}$, $A^{13}$, $A^{14}$ and $A^{15}$ in the formulae I and II have the same meaning as in Claim 1 for formula I;
with the proviso
that, in the case of direct linking of $Z^{13}$ and $R^{12}$ to give $-Z^{13}-R^{12}$, $R^{12}$ denotes H, aralkyl or alkanyl if $Z^{13}$ denotes -C(=O)-, $R^{12}$ denotes aralkyl or alkanyl if $Z^{13}$ denotes -C(=O)-O-, and $Z^{13}$ does not denote $-CH_2O-$ or $-CF_2O-$;
that, in the case of direct linking of $Z^{14}$ and $R^{12}$ to give $-Z^{14}-R^{12}$, $R^{12}$ denotes H, aralkyl or alkanyl if $Z^{14}$ denotes -C(=O)-, $R^{12}$ denotes aralkyl or alkanyl if $Z^{14}$ denotes -C(=O)-O-, and $Z^{14}$ does not denote $-CH_2O-$ or $-CF_2O-$;
that, in the case of direct linking of $Z^{15}$ and $R^{12}$ to give $-Z^{15}-R^{12}$, $R^{12}$ denotes H, aralkyl or alkanyl if $Z^{15}$ denotes -C(=O)-, $R^{12}$ denotes aralkyl or alkanyl if $Z^{15}$ denotes -C(=O)-O-, and $Z^{15}$ does not denote $-CH_2O-$ or $-CF_2O-$;
in the presence of a metathesis catalyst.

14. Process according to Claim 13, **characterised in that** the metathesis catalyst is a ruthenium-alkylidene complex.

15. Process for the preparation of pyran derivatives which includes a process step which includes a cross metathesis reaction of a pyran derivative of the general formula I-C with an alkene of the formula IIIa or of the formula IIIb

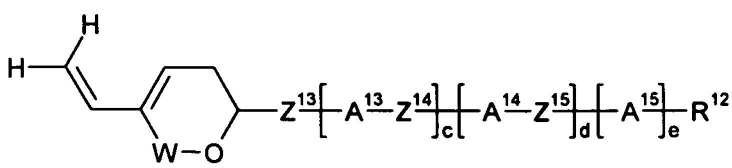

I-C

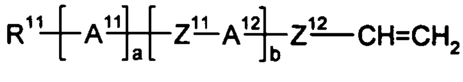

IIIa

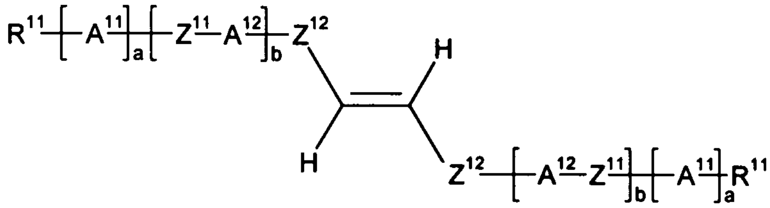

IIIb

to give a pyran derivative of the formula I

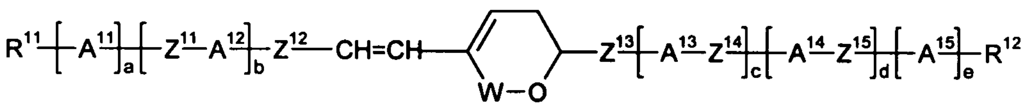

I

where a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ and $A^{15}$ in the formulae I, I-C, IIIa and IIIb have the same meaning as in Claim 1 for formula I;
with the proviso
that, in the case of direct linking of $Z^{13}$ and $R^{12}$ to give $-Z^{13}-R^{12}$, $R^{12}$ denotes H, aralkyl or alkanyl if $Z^{13}$ denotes -C(=O)-, $R^{12}$ denotes aralkyl or alkanyl if $Z^{13}$ denotes -C(=O)-O-, and $Z^{13}$ does not denote $-CH_2O-$ or $-CF_2O-$;
that, in the case of direct linking of $Z^{14}$ and $R^{12}$ to give $-Z^{14}-R^{12}$, $R^{12}$ denotes H, aralkyl or alkanyl if $Z^{14}$ denotes -C(=O)-, $R^{12}$ denotes aralkyl or alkanyl if $Z^{14}$ denotes -C(=O)-O-, and $Z^{14}$ does not denote $-CH_2O-$ or $-CF_2O-$;
that, in the case of direct linking of $Z^{15}$ and $R^{12}$ to give $-Z^{15}-R^{12}$, $R^{12}$ denotes H, aralkyl or alkanyl if $Z^{15}$ denotes -C(=O)-, $R^{12}$ denotes aralkyl or alkanyl if $Z^{15}$ denotes -C(=O)-O-, and $Z^{15}$ does not denote $-CH_2O-$ or $-CF_2O-$;
in the presence of a metathesis catalyst.

**16.** Process according to Claim 15, **characterised in that** the metathesis catalyst is a ruthenium-alkylidene complex.

**17.** Process according to Claim 15 or 16, **characterised in that** the compound of the general formula I-C is prepared by a process according to one of Claims 13 and 14.

**18.** Process according to one of Claims 13 to 17 which includes, as a further reaction step after the metathesis reaction step, a reduction reaction for conversion of the pyran derivative of the formula I in which W denotes -C(=O)- and $Z^{13}$, $Z^{14}$ and $Z^{15}$ do not denote -C(O)- into a pyran derivative of the formula I in which

W denotes $-CH_2-$;
$R^{11}$ denotes H, an alkyl radical having 1 to 15 carbon atoms which is unsubstituted or mono- or polysubstituted, identically or differently, by halogen or -CN, where, in addition, one or more $CH_2$ groups in this radical may be replaced by -C≡C-, -CH=CH-, -O- and/or -S- in such a way that heteroatoms (O, S) are not linked directly to one another;
$R^{12}$ denotes H, halogen, -CN, -NCS, aralkyl, -O-aralkyl or an alkyl radical having 1 to 15 carbon atoms which is unsubstituted or mono- or polysubstituted, identically or differently, by halogen or -CN, where, in addition, one or more $CH_2$ groups in this radical may be replaced by -C≡C-, -CH=CH-, -O- and/or -S- in such a way that heteroatoms (O, S) are not linked directly to one another;
$Z^{13}$, $Z^{14}$ and $Z^{15}$ each, independently of one another, denote a single bond, $-CH_2CH_2-$, $-CF_2CH_2-$, $-CH_2CF_2-$, $-CF_2CF_2-$, -CH=CH-, -C≡C-, $-CH_2O-$ or $-CF_2O-$;

and a, b, c, d, e, $Z^{11}$, $Z^{12}$, $A^{11}$, $A^{12}$, $A^{13}$, $A^{14}$ and $A^{15}$ are as defined in Claim 1.

**19.** Process according to one of Claims 13 to 18 which includes, as a further reaction step after the metathesis reaction step and after the optional reduction reaction step, a catalytic hydrogenation to give a pyran of the general formula IV:

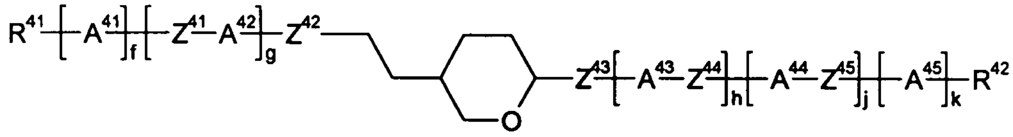

IV

in which

f, g, h, j and k are each, independently of one another, 0 or 1;
$R^{41}$ denotes H, a saturated alkyl radical having 1 to 15 carbon atoms which is unsubstituted or mono- or polysubstituted, identically or differently, by halogen or -CN, where, in addition, one or more $CH_2$ groups in this radical may be replaced by -O-, -S-, -C(O)-O- and/or -O-C(O)- in such a way that heteroatoms (O, S) are not linked directly to one another;
$R^{42}$ denotes H, halogen, -CN, -NCS, aralkyl, -O-aralkyl or a saturated alkyl radical having 1 to 15 C atoms which is unsubstituted or mono- or polysubstituted, identically or differently, by halogen or -CN, where, in addition, one or more $CH_2$ groups in this radical may be replaced by -O-, -S-, -C(O)-O- and/or -O-C(O)- in such a way that heteroatoms (O, S) are not linked directly to one another;
$Z^{41}$ and $Z^{42}$, independently of one another, denote a single bond, $-CH_2-$, $-CH_2CH_2-$, $-CF_2CH_2-$, $-CH_2CF_2-$ or $-CF_2CF_2-$;
$Z^{43}$, $Z^{44}$ and $Z^{45}$ each, independently of one another, denote a single bond, $-CH_2CH_2-$, $-CF_2CH_2-$, $-CH_2CF_2-$, $-CF_2CF_2-$, $-CH_2O-$, $-CF_2O-$, -C(O)- or -C(O)-O-;
$A^{41}$ and $A^{42}$, independently of one another, stand for

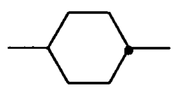 or 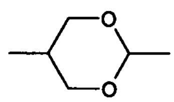 ;

$A^{43}$ and $A^{44}$, independently of one another, stand for

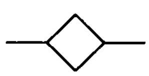 , 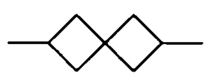 , 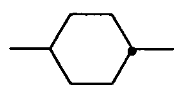 ,

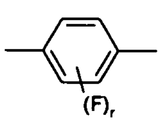 , 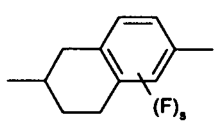 or 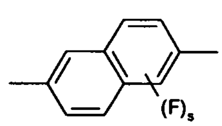 ;

$A^{45}$ stands for

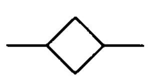 , 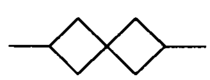 ,

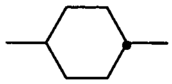 , 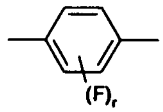 , 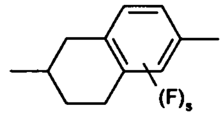

or

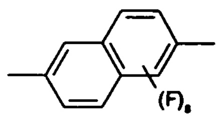

or

$A^{45}$-$R^{42}$ together stand for

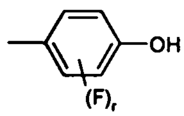 , 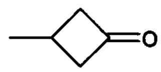 ,

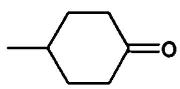 or 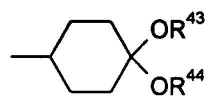 ;

or

$Z^{43}$-[-$A^{43}$-$Z^{44}$-]$_h$-[-$A^{44}$-$Z^{45}$-]$_j$-[-$A^{45}$-]$_k$-$R^{42}$ stands for

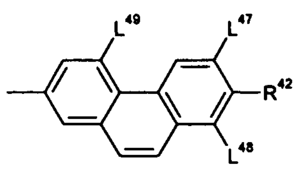 or 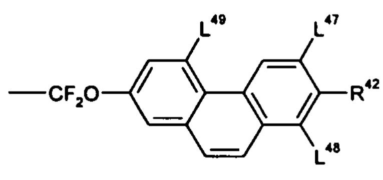 ,

where $R^{42}$ is as defined above in this claim and $L^{47}$, $L^{48}$ and $L^{49}$, independently of one another, denote H or F;
r is 0, 1, 2, 3 or 4;
s is 0, 1, 2 or 3;
$R^{43}$ and $R^{44}$, independently of one another, denote an alkanyl radical having 1 to 7 carbon atoms or together stand for an alkylene bridge having 2 to 7 carbon atoms;

with the proviso
that, in the case of direct linking of $Z^{43}$ and $R^{42}$ to give -$Z^{43}$-$R^{42}$, $R^{42}$ denotes H, aralkyl or alkanyl if $Z^{43}$ denotes -C(=O)-, $R^{42}$ denotes aralkyl or alkanyl if $Z^{43}$ denotes -C(=O)-O-, and $Z^{43}$ does not denote -$CH_2$O- or -$CF_2$O-;
that, in the case of direct linking of $Z^{44}$ and $R^{42}$ to give -$Z^{44}$-$R^{42}$, $R^{42}$ denotes H, aralkyl or alkanyl if $Z^{44}$ denotes -C(=O)-, $R^{42}$ denotes aralkyl or alkanyl if $Z^{44}$ denotes -C(=O)-O-, and $Z^{44}$ does not denote -$CH_2$O- or -$CF_2$O-;
that, in the case of direct linking of $Z^{45}$ and $R^{42}$ to give -$Z^{45}$-$R^{42}$, $R^{42}$ denotes H, aralkyl or alkanyl if $Z^{45}$ denotes -C(=O)-, $R^{42}$ denotes aralkyl or alkanyl if $Z^{45}$ denotes -C(=O)-O-, and $Z^{45}$ does not denote -$CH_2$O- or -$CF_2$O-.

**20.** Compound of the formula II

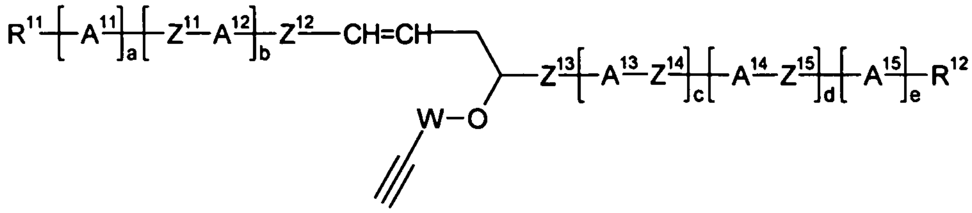

II

where a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ and $A^{15}$ have the same meaning as in Claim 1 for formula I.

**21.** Compound of the formula V-II

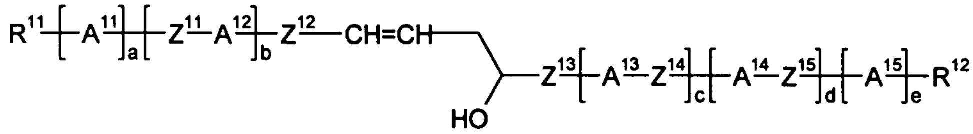

V-II

where a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ and $A^{15}$ have the same meaning as in Claim 1 for formula I.

**22.** Use of a compound of the formula I according to Claim 1 as constituent of a liquid-crystalline medium in an electro-optical display device.

**Revendications**

**1.** Composé de la formule générale I

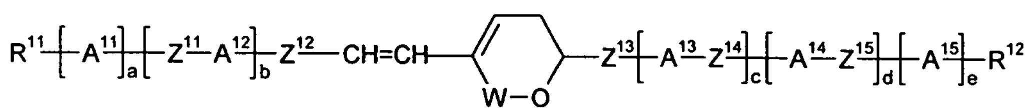

I

dans laquelle

a, b, c, d et e sont, chacun indépendamment les uns des autres, 0 ou 1 ;
W représente $-CH_2-$ ou -C(=O)- ;
$R^{11}$ représente H, un radical alkyle comportant de 1 à 15 atomes de carbone, lequel est non substitué ou mono- ou polysubstitué, de façon identique ou différente, par halogène ou -CN, où, en plus, un ou plusieurs groupes $CH_2$ dans ce radical peuvent être remplacés par -C≡C-, -CH=CH-, -O-, -S-, -C(O)-O- et/ou -O-C(O)- de telle sorte que des hétéroatomes (O, S) ne soient pas liés directement les uns aux autres ;
$R^{12}$ représente H, halogène, -CN, -NCS, aralkyle, -O-aralkyle ou un radical alkyle comportant de 1 à 15 atomes de carbone, lequel est non substitué ou mono- ou polysubstitué, de façon identique ou différente, par halogène ou -CN, où, en plus, un ou plusieurs groupes $CH_2$ dans ce radical peuvent être remplacés par -C≡C-, -CH=CH-,

-O-, -S-, -C(O)-O- et/ou -O-C(O)- de telle sorte que des hétéroatomes (O, S) ne soient pas liés directement les uns aux autres ;

$Z^{11}$ représente une liaison simple, $-CH_2-$, $-CH_2CH_2-$, $-CF_2CH_2-$, $-CH_2CF_2-$, $-CF_2CF_2-$, -CH=CH- ou -C≡C- ;

$Z^{12}$ représente une liaison simple, $-CH_2-$, $-CH_2CH_2-$, $-CF_2CH_2-$, $-CH_2CF_2-$ ou $-CF_2CF_2-$ ;

$Z^{13}$, $Z^{14}$ et $Z^{15}$ chacun indépendamment les uns des autres, représentent une liaison simple, $-CH_2CH_2-$, $-CF_2CH_2-$, $-CH_2CF_2-$, $-CF_2CF_2-$, -CH=CH-, -C≡C-, $-CH_2O-$, $-CF_2O-$, -C(O)- ou -C(O)-O- ;

$A^{11}$ et $A^{12}$ indépendamment l'un de l'autre, représentent

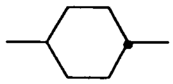 ou 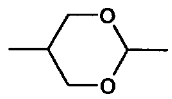 ;

$A^{13}$ et $A^{14}$, indépendamment l'un de l'autre, représentent

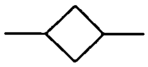, 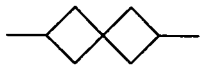, 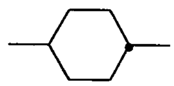,

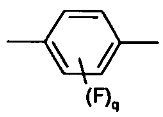, 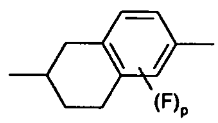

ou

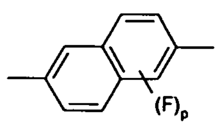 ;

$A^{15}$ représente

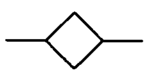, 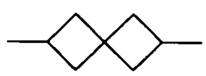,

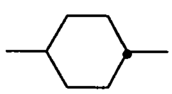, 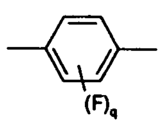, 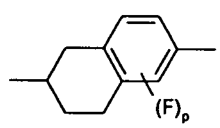

ou

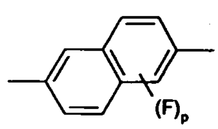

ou
$A^{15}$-$R^{12}$ représentent ensemble

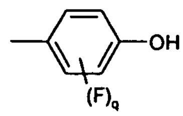 , 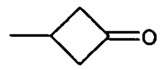 ,

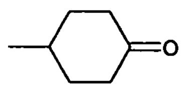 ou 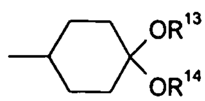 ;

ou
$Z^{13}$-[-$A^{13}$-$Z^{14}$-]$_c$-[-$A^{14}$-$Z^{15}$-]$_d$-[-$A^{15}$-]$_e$-$R^{12}$ représente

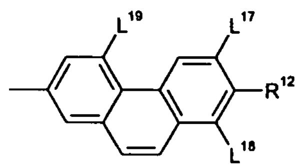

ou

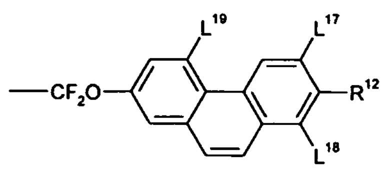 ,

où $R^{12}$ est tel que défini ci-avant dans cette revendication et $L^{17}$, $L^{18}$ et $L^{19}$, indépendamment les uns des autres, représentent H ou F ;
q est 0, 1, 2, 3 ou 4 ;
p est 0, 1,2 ou 3;
$R^{13}$ et $R^{14}$ indépendamment l'un de l'autre, représentent un radical alcanyle comportant de 1 à 7 atomes de carbone ou représentent ensemble un pont alkylène comportant de 2 à 7 atomes de carbone ;

étant entendu
que dans le cas d'une liaison directe de $Z^{13}$ et $R^{12}$ pour donner -$Z^{13}$-$R^{12}$, $R^{12}$ représente H, aralkyle, alcanyle ou alkényle si $Z^{13}$ représente -C(=O)-O- ou -C(=O)-, et $Z^{13}$ ne représente pas -$CH_2O$- ou -$CF_2O$- ;
que, dans le cas d'une liaison directe de $Z^{14}$ et $R^{12}$ pour donner -$Z^{14}$-$R^{12}$, $R^{12}$ représente H, aralkyle, alcanyle ou alkényle si $Z^{14}$ représente -C(=O)-O- ou -C(=O)-, et $Z^{14}$ ne représente pas -$CH_2O$- ou -$CF_2O$- ;
que dans le cas d'une liaison directe de $Z^{15}$ et $R^{12}$ pour donner -$Z^{15}$-$R^{12}$, $R^{12}$ représente H, aralkyle, alcanyle ou alkényle si $Z^{15}$ représente -C(=O)-O- ou -C(=O)-, et $Z^{15}$ ne représente pas -$CH_2O$- ou -$CF_2O$-.

**2.** Composé selon la revendication 1, **caractérisé en ce que**

$$a+b+c+d+e \leq 3.$$

**3.** Composé selon l'une des revendications précédentes, **caractérisé en ce que**

W représente -C(=O)-.

**4.** Composé selon la revendication 1 ou 2, **caractérisé en ce que**

W représente $-CH_2-$.

**5.** Composé selon l'une des revendications précédentes, **caractérisé en ce que**

$Z^{12}$ représente une liaison simple.

**6.** Composé selon la revendication 5, **caractérisé en ce que**

a et b sont zéro ; et
$R^{11}$ est H.

**7.** Composé selon l'une des revendications précédentes, **caractérisé en ce que**

$Z^{13}$ représente une liaison simple, -C(O)-O- ou $-CF_2O-$.

**8.** Composé selon l'une des revendications 1 à 5 ou 7, **caractérisé en ce que**

a est zéro ;
b est 1 ;
$Z^{11}$ représente une liaison simple ; et
$A^{12}$ représente

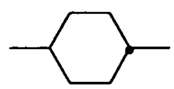

.

**9.** Composé selon l'une des revendications précédentes, **caractérisé en ce que**

c, d et e sont simultanément zéro ;
$Z^{13}$ représente -C(O)-O- ; et
$R^{12}$ représente H, aralkyle, alcanyle ou alkényle.

**10.** Composé selon l'une des revendications 1 à 8, **caractérisé en ce que**

e est 1 ;
$A^{15}$ représente

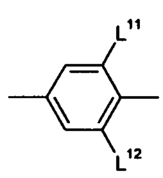

;

et
$L^{11}$ et $L^{12}$, indépendamment l'un de l'autre, représentent H ou F.

**11.** Composé selon l'une des revendications 1 à 8 ou 10, **caractérisé en ce que** au moins l'un de c et d est 1 ;

e est 1 ; et
$Z^{14}$ et $Z^{15}$, indépendamment l'un de l'autre, représentent une liaison simple ou $-CF_2O-$.

**12.** Composé selon l'une des revendications 1 à 8 ou 11, **caractérisé en ce que**

e est 1 ;
$A^{15}$-$R^{12}$ représentent

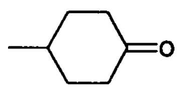 ou 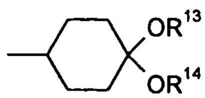 ; et

$R^{13}$ et $R^{14}$ sont tels que définis dans la revendication 1.

**13.** Procédé pour la préparation de dérivés de pyranne, lequel inclut une étape de processus qui inclut un réaction de métathèse d'enyne d'un enyne de la formule générale II

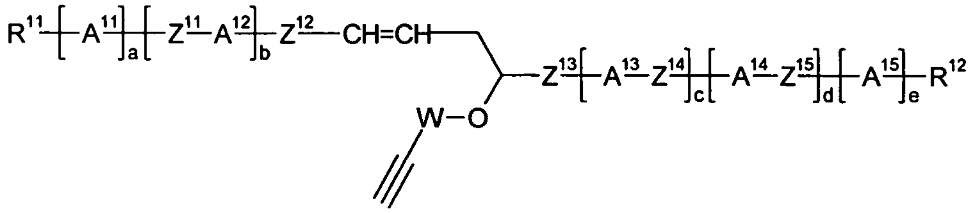

II

pour donner un dérivé de pyranne de la formule I

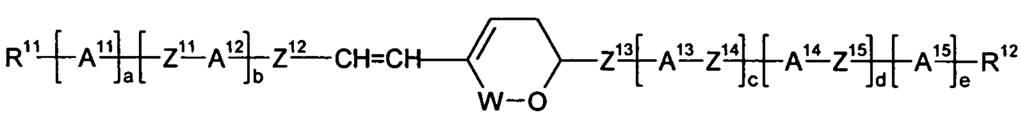

I

dans laquelle a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ et $A^{15}$ dans les formules I et II présentent les mêmes significations que dans la revendication 1 pour la formule I ;
étant entendu
que, dans le cas d'une liaison directe de $Z^{13}$ et $R^{12}$ pour donner $-Z^{13}-R^{12}$, $R^{12}$ représente H, aralkyle ou alcanyle si $Z^{13}$ représente -C(=O)-, $R^{12}$ représente aralkyle ou alcanyle si $Z^{13}$ représente -C(=O)-O-, et $Z^{13}$ ne représente pas $-CH_2O-$ ou $-CF_2O-$ ;
que, dans le cas d'une liaison directe de $Z^{14}$ et $R^{12}$ pour donner $-Z^{14}-R^{12}$, $R^{12}$ représente H, aralkyle ou alcanyle si $Z^{14}$ représente -C(=O)-, $R^{12}$ représente aralkyle ou alcanyle si $Z^{14}$ représente -C(=O)-O-, et $Z^{14}$ ne représente pas $-CH_2O-$ ou $-CF_2O-$ ;
que, dans le cas d'une liaison directe de $Z^{15}$ et $R^{12}$ pour donner $-Z^{15}-R^{12}$, $R^{12}$ représente H, aralkyle ou alcanyle si $Z^{15}$ représente -C(=O)-, $R^{12}$ représente aralkyle ou alcanyle si $Z^{15}$ représente -C(=O)-O-, et $Z^{15}$ ne représente pas $-CH_2O-$ ou $-CF_2O-$ ;
en présence d'un catalyseur de métathèse.

**14.** Procédé selon la revendication 13, **caractérisé en ce que** le catalyseur de métathèse est un complexe ruthénium-alkylidène.

**15.** Procédé pour la préparation de dérivés de pyranne, lequel inclut une étape de processus qui inclut une réaction de métathèse croisée d'un dérivé de pyranne de la formule générale I-C avec un alcène de la formule IIIa ou de la formule IIIb

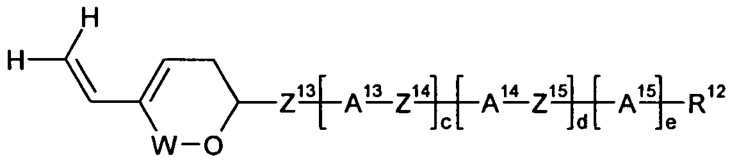

I-C

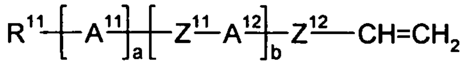

IIIa

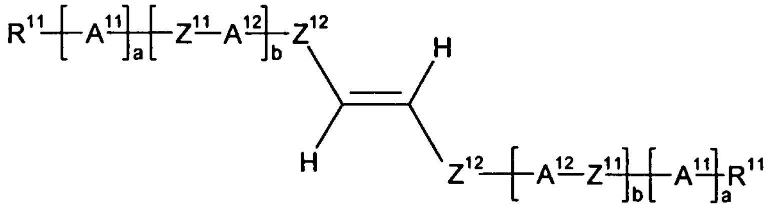

IIIb

pour donner un dérivé de pyranne de la formule I

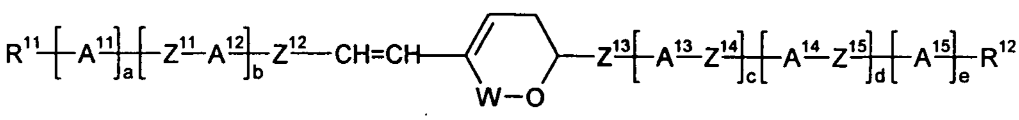

I

dans laquelle a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ et $A^{15}$ dans les formules I, I-C, IIIa et IIIb présentent les mêmes significations que dans la revendication 1 pour la formule I ; étant entendu
que, dans le cas d'une liaison directe de $Z^{13}$ et $R^{12}$ pour donner $-Z^{13}-R^{12}$, $R^{12}$ représente H, aralkyle ou alcanyle si $Z^{13}$ représente -C(=O)-, $R^{12}$ représente aralkyle ou alcanyle si $Z^{13}$ représente -C(=O)-O-, et $Z^{13}$ ne représente pas $-CH_2O-$ ou $-CF_2O-$ ;
que, dans le cas d'une liaison directe de $Z^{14}$ et $R^{12}$ pour donner $-Z^{14}-R^{12}$, $R^{12}$ représente H, aralkyle ou alcanyle si $Z^{14}$ représente -C(=O)-, $R^{12}$ représente aralkyle ou alcanyle si $Z^{14}$ représente -C(=O)-O-, et $Z^{14}$ ne représente pas $-CH_2O-$ ou $-CF_2O-$ ;
que, dans le cas d'une liaison directe de $Z^{15}$ et $R^{12}$ pour donner $-Z^{15}-R^{12}$, $R^{12}$ représente H, aralkyle ou alcanyle si $Z^{15}$ représente -C(=O)-, $R^{12}$ représente aralkyle ou alcanyle si $Z^{15}$ représente -C(=O)-O-, et $Z^{15}$ ne représente pas $-CH_2O-$ ou $-CF_2O-$ ;
en présence d'un catalyseur de métathèse.

**16.** Procédé selon la revendication 15, **caractérisé en ce que** le catalyseur de métathèse est un complexe ruthénium-alkylidène.

**17.** Procédé selon la revendication 15 ou 16, **caractérisé en ce que** le composé de la formule générale I-C est préparé au moyen d'un procédé selon l'une des revendications 13 et 14.

**18.** Procédé selon l'une des revendications 13 à 17, lequel inclut, en tant qu'autre étape de réaction après l'étape de réaction de métathèse, une réaction de réduction pour la conversion du dérivé de pyranne de la formule I dans laquelle W représente -C(=O)- et $Z^{13}$, $Z^{14}$ et $Z^{15}$ ne représente -C(O)- selon un dérivé de pyranne de la formule I dans laquelle

W représente $-CH_2-$ ;
$R^{11}$ représente H, un radical alkyle comportant de 1 à 15 atomes de carbone, lequel est non substitué ou mono- ou polysubstitué, de façon identique ou différente, par halogène ou -CN, où, en plus, un ou plusieurs groupes $CH_2$ dans ce radical peuvent être remplacés par -C≡C-, -CH=CH-, -O- et/ou -S- de telle sorte que des hétéroatomes (O, S) ne soient pas liés directement les uns aux autres ;
$R^{12}$ représente H, halogène, -CN, -NCS, aralkyle, -O-aralkyle ou un radical alkyle comportant de 1 à 15 atomes de carbone, lequel est non substitué ou mono- ou polysubstitué, de façon identique ou différente, par halogène ou -CN, où, en plus, un ou plusieurs groupes $CH_2$ dans ce radical peuvent être remplacés par -C≡C-, -CH=CH-, -O- et/ou -S- de telle sorte que des hétéroatomes (O, S) ne soient pas liés directement les uns aux autres ;
$Z^{13}$, $Z^{14}$ et $Z^{15}$ chacun indépendamment les uns des autres, représentent une liaison simple, $-CH_2CH_2-$, $-CF_2CH_2-$, $-CH_2CF_2-$, $-CF_2CF_2-$, -CH=CH-, -C≡C-, $-CH_2O-$ ou $-CF_2O-$ ;

et a, b, c, d, e, $Z^{11}$, $Z^{12}$, $A^{11}$, $A^{12}$, $A^{13}$, $A^{14}$ et $A^{15}$ sont tels que définis dans la revendication 1.

**19.** Procédé selon l'une des revendications 13 à 18, lequel inclut, en tant qu'autre étape de réaction après l'étape de réaction de métathèse et après l'étape de réaction de réduction optionnelle, une hydrogénation catalytique pour donner un pyranne de la formule générale IV:

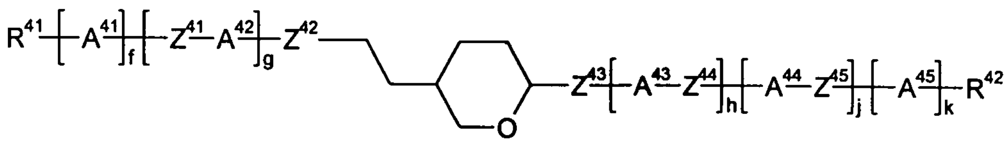

IV

dans laquelle

f, g, h, j et k sont, chacun indépendamment les uns des autres, 0 ou 1 ;
$R^{41}$ représente H, un radical alkyle saturé comportant de 1 à 15 atomes de carbone, lequel est non substitué ou mono- ou polysubstitué, de façon identique ou différente, par halogène ou -CN, où, en plus, un ou plusieurs groupes $CH_2$ dans ce radical peuvent être remplacés par -O-, -S-, -C(O)-O- et/ou -O-C(O)- de telle sorte que des hétéroatomes (O, S) ne soient pas liés directement les uns aux autres ;
$R^{42}$ représente H, halogène, -CN, -NCS, aralkyle, -O-aralkyle ou un radical alkyle saturé comportant de à 15 atomes de C, lequel est non substitué ou mono- ou polysubstitué, de façon identique ou différente, par halogène ou -CN, où, en plus, un ou plusieurs groupes $CH_2$ dans ce radical peuvent être remplacés par -O-, -S-, -C(O)-O- et/ou -O-C(O)- de telle sorte que des hétéroatomes (O, S) ne soient pas liés directement les uns aux autres ;
$Z^{41}$ et $Z^{42}$ indépendamment l'un de l'autre, représentent une liaison simple, $-CH_2-$, $-CH_2CH_2-$, $-CF_2CH_2-$, $-CH_2CF_2-$ ou $-CF_2CF_2-$ ;
$Z^{43}$, $Z^{44}$ et $Z^{45}$ chacun indépendamment les uns des autres, représentent une liaison simple, $-CH_2CH_2-$, $-CF_2CH_2-$, $-CH_2CF_2-$, $-CF_2CF_2-$, $-CH_2O-$, $-CF_2O-$, -C(O)- ou -C(O)-O- ;
$A^{41}$ et $A^{42}$ indépendamment l'un de l'autre, représentent

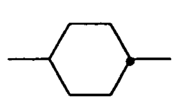 ou 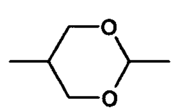 ;

$A^{43}$ et $A^{44}$ indépendamment l'un de l'autre, représentent

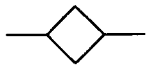 , 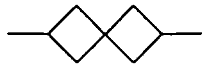 , 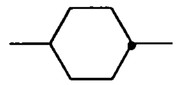 ,

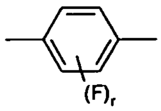 , 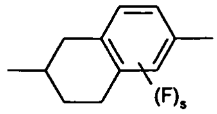 ou 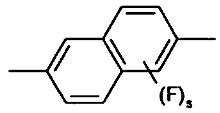 ;

$A^{45}$ représente

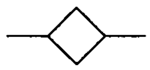 , 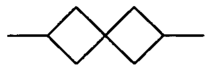 ,

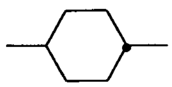 , 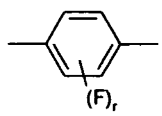 , 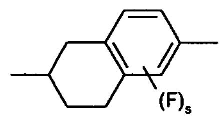

ou

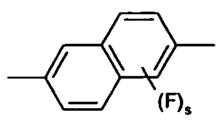

ou
$A^{45}$-$R^{42}$ représentent ensemble

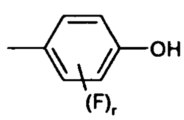 , 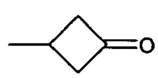 ,

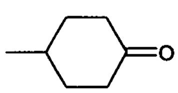 ou 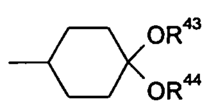 ;

ou
$Z^{43}$-[-$A^{43}$-$Z^{44}$-]$_h$-[-$A^{44}$-$Z^{45}$-]$_j$-[-$A^{45}$-]$_k$-$R^{42}$ représente

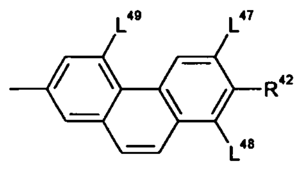

ou

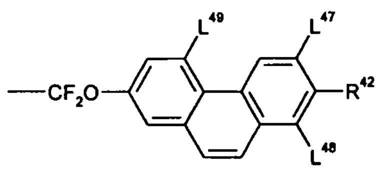

,

où $R^{42}$ est tel que défini avant dans cette revendication et $L^{47}$, $L^{48}$ et $L^{49}$, indépendamment les uns des autres, représentent H ou F;

r est 0, 1, 2, 3 ou 4 ;

s est 0, 1, 2 ou 3 ;

$R^{43}$ et $R^{44}$ indépendamment l'un de l'autre, représentent un radical alcanyle comportant de 1 à 7 atomes de carbone ou représentent ensemble un pont alkylène comportant de 2 à 7 atomes de carbone ;

étant entendu

que, dans le cas d'une liaison directe de $Z^{43}$ et $R^{42}$ pour donner $-Z^{43}-R^{42}$, $R^{42}$ représente H, aralkyle ou alcanyle si $Z^{43}$ représente -C(=O)-, $R^{42}$ représente aralkyle ou alcanyle si $Z^{43}$ représente -C(=O)-O-, et $Z^{43}$ ne représente pas $-CH_2O-$ ou $-CF_2O-$ ;

que, dans le cas d'une liaison directe de $Z^{44}$ et $R^{42}$ pour donner $-Z^{44}-R^{42}$, $R^{42}$ représente H, aralkyle ou alcanyle si $Z^{44}$ représente -C(=O)-, $R^{42}$ représente aralkyle ou alcanyle si $Z^{44}$ représente -C(=O)-O-, et $Z^{44}$ ne représente pas $-CH_2O-$ ou $-CF_2O-$ ;

que, dans le cas d'une liaison directe de $Z^{45}$ et $R^{42}$ pour donner $-Z^{45}-R^{42}$, $R^{42}$ représente H, aralkyle ou alcanyle si $Z^{45}$ représente -C(=O)-, $R^{42}$ représente aralkyle ou alcanyle si $Z^{45}$ représente -C(=O)-O-, et $Z^{45}$ ne représente pas $-CH_2O-$ ou $-CF_2O-$.

**20.** Composé de la formule II

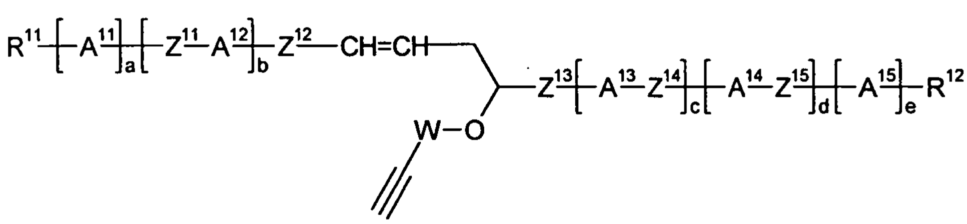

II

dans laquelle a, b, c, d, e, W, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ et $A^{15}$ présentent les mêmes significations que dans la revendication 1 pour la formule I.

**21.** Composé de la formule V-II

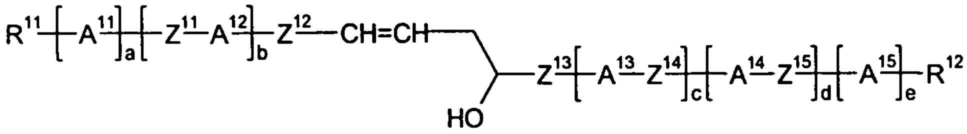

V-II

dans laquelle a, b, c, d, e, $R^{11}$, $R^{12}$, $Z^{11}$, $Z^{12}$, $Z^{13}$, $Z^{14}$, $Z^{15}$, $A^{11}$, $A^{12}$, $A^{13}$, $A^{14}$ et $A^{15}$ présentent les mêmes significations que dans la revendication 1 pour la formule I.

**22.** Utilisation d'un composé de la formule I selon la revendication 1 en tant que constituant d'un milieu de cristal liquide dans un dispositif d'affichage électro-optique.